# EUROPEAN PATENT APPLICATION

(11) **EP 4 628 494 A1**
(43) Date of publication of application: **08.10.2025**
(21) Application number: 23900029.2
(22) Date of filing: 06.12.2023
(51) Int. Cl.: C07F 9/24, A61K 47/18, A61P 35/00, A61P 1/16, A61P 19/02

(54) **PHOSPHATIDYLAMINE COMPOUND COMPRISING MULTIPLE TERTIARY AMINO GROUP STRUCTURES, AND COMPOSITION AND USE THEREOF**

(30) Priority: 06.12.2022 CN 202211559019
(71) Applicant: Peking University, Beijing 100871 (CN); Proxybio Therapeutics (Shenzhen) Co., Ltd., Shenzhen, Guangdong 518000 (CN)
(72) Inventor: MIAO, Lei, Beijing 100083 (CN); MA, Bin, Beijing 100083 (CN); FANG, Yian, Beijing 100083 (CN); ZHANG, Huijuan, Beijing 100083 (CN); XUE, Yizhe, Beijing 100083 (CN)
(74) Representative: Bayramoglu et al.
(86) International application number: PCT/CN2023/136906
(87) International publication number: WO 2024/120455

(57) **Abstract**

A phosphatidylamine compound including a plurality of tertiary amino group structures and the composition and use thereof are provided. The phosphatidylamine compound is a phospholipid compound including two or more tertiary amino group structures, the structure of which is represented by the following formula (I), where, the definition of each substituent is detailed in the instructions. The compound works together with other lipid components such as cholesterol, DSPC/DOPE, DMG-PEG2000, and other helper lipids to form lipid nanoparticles (LNPs), which may be used for efficient delivery of drug molecules such as nucleic acids (siRNA, mRNA, pDNA), thereby realizing diagnosis and treatment of diseases such as cancer, fibrosis (e.g., liver, lung, kidney).

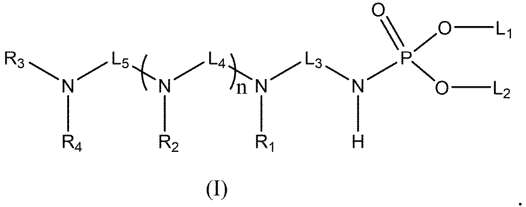

## Description

This application is required to be submitted to the Chinese Patent Office on December 06,2022, with the application number of 202211559019.0, and the name of the invention is " phosphatidylamine compounds including a plurality of tertiary amino group structures and compositions thereof " The priority of the Chinese patent application should be understood as being incorporated into this application by reference.

### TECHNICAL FIELD

The present disclosure relates to the pharmaceutical technology, and in particular to a phosphatidylamine compound including a plurality of tertiary amino group structures and a composition thereof.

### BACKGROUND TECHNOLOGY

Nucleic acid therapeutics are evolving into precision medicine technologies that can deal with specific genes, which simultaneously imposes greater demands on safe and effective delivery systems for target cells. Lipid nanoparticles (LNPs), mainly including cationic lipids, neutral lipids, steroidal lipids, and polymer-conjugated lipids, have enabled safe and effective delivery to target cells. However, there is still a demand for highly effective, safe, and stabilized lipid compounds due to differences in the action type of therapeutic drugs, formulation design, and biodistribution.

### CONTENT OF THE INVENTION

In a first aspect, this application provides a phosphatidylamine compound including a plurality of tertiary amino group structures, the phosphatidylamine compound being a compound represented by formula (I) or a stereoisomer, a prodrug, a pharmaceutically acceptable salt thereof: 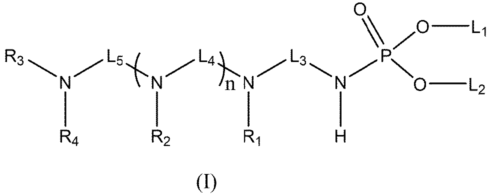
wherein n is an integer from 0-5 in formula (I) ;
L₁ and L₂ are each independently C₅-C₃₀ alkyl, C₅-C₄₀ alkenyl, or -R'-M-R";
R₁ and R₂ are each independently C₄-C₃₀ alkyl, C₂-C₄₀ alkenyl, or -R'-M-R"; or R₁ and R₂ together with the respective attached N and L₄ form a 5 to 8-membered diazacycloalkylene;
R₃ and R₄ are each independently C₁-C₃₀ alkyl, C₂-C₄₀ alkenyl, or -R'-M-R"; or R₃ is H, C₁-C₃₀ alkyl, C₂-C₄₀ alkenyl, or -R'-M-R", and R₄ is C₁-C₃₀ alkyl, C₂-C₄₀ alkenyl, or -R'-M-R";
R' is C₁-C₂₄ alkylene or C₂-C₂₄ alkenylene;
M is selected from -C(O)-, -C(O)O-, -OC(O)-, -OC(O)O-, -C(S)-, -C(O)S-, -SC(O)-, -C(S)S-, -SC(S)-, -S(O)₂-, -S-S-, -C(O)N(R^{a})-, -N(R^{a})C(O)-, -OC(O)N(R^{a})-, -N(R^{a})C(O)O-, -N(R^{a})C(O)N(R^{a})-, -C(O-C(O)-R^{a})(H)-C₁₋₆ alkylene-O-C(O)-, -C(O)O-C₁₋₆ alkylene-C(O)-O-, -CH(OH)-, -P(O)(OR^{b})O-, C₆-C₁₀ arylene, and 5 to 10-membered heteroarylene;
R", R^{a}, and R^{b} are each independently selected from H, C₁-C₃₀ alkyl, and C₂-C₄₀ alkenyl; and
L₃, L₄, and L₅ are each independently C₁-C₁₂ alkylene, C₂-C₁₂ alkenylene, C₃-C₈ cycloalkylene, -A-Y-B-, -A-Y-, or -Y-B-, wherein A and B are each independently C₁-C₁₂ alkylene or C₂-C₁₂ alkenylene, and Y is -C(OH)-, -C(O)-, -C(O)O-, -OC(O)-, -C(O)N(H)-, -N(H)C(O)-, arylene, or heteroarylene.

In a second aspect, this application provides a preparation method of the phosphatidylamine compound described above.

In a third aspect, this application provides a lipid composition comprising a phosphatidylamine compound described above, and a therapeutic agent or a prophylactic agent.

In a fourth aspect, this application provides a lipid nanoparticle, the lipid nanoparticle including a phosphatidylamine compound described above or a lipid composition described above.

In a fifth aspect, this application provides a pharmaceutical composition comprising a phosphatidylamine compound described above, a lipid composition described above, or a lipid nanoparticle described above, and pharmaceutically acceptable diluents or excipients.

In a sixth aspect, this application provides a use of a lipid composition described above, a lipid nanoparticle described above, or a pharmaceutical composition described above for treating or preventing a disease in an individual in corresponding need; or a use of the lipid composition described above, the lipid nanoparticle described above, or the pharmaceutical composition described above in the preparation of a drug for treating or preventing a disease in a corresponding need.

In a seventh aspect, this application provides a use of a lipid composition described above, a lipid nanoparticle described above, or a pharmaceutical composition described above in a drug for vaccination against viral pathogens.

In an eighth aspect, this application provides a method for treating or preventing a disease in an individual in a corresponding need, the method including administering a lipid composition, a lipid nanoparticle, or a pharmaceutical composition described above to the individual.

In a ninth aspect, this application provides a method for vaccinating an individual in a corresponding need against antiviral pathogens, the method including administering a lipid composition, a lipid nanoparticle, or a pharmaceutical composition described above to the individual.

Other features and advantages of the present disclosure may be set forth in the subsequent description, and some of them may become apparent from the description or may be understood by the implementation of the present disclosure. Other advantages of the present disclosure may be realized and obtained by the solutions described in the description and the accompanying drawings.

### BRIEF DESCRIPTION OF THE DRAWINGS

The accompanying drawings are used to provide an understanding of the technical solutions of the present disclosure and form a part of the present disclosure, and in conjunction with the application, are used to explain the technical solutions of the present disclosure, which does not constitute a limitation of the technical solutions of the present disclosure.

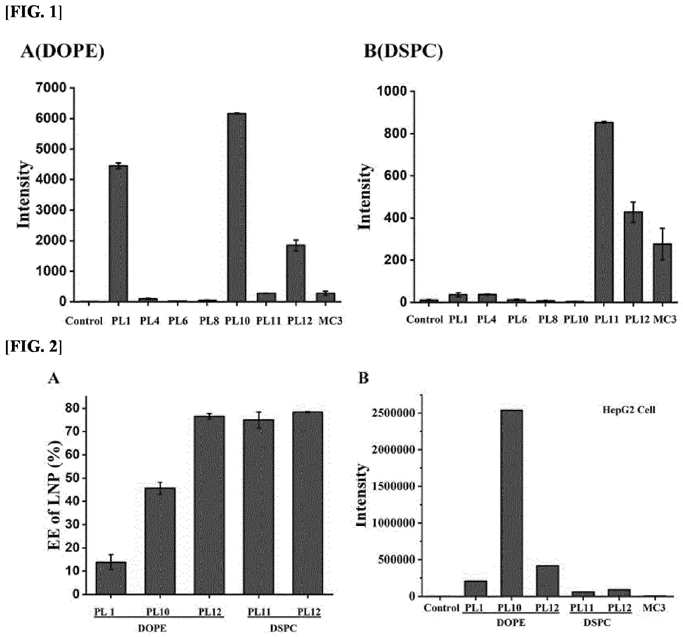

FIG. 1 illustrates the transfection of lipid nanoparticles in a DOPE group and a DSPC group in NIH-3T3 cells in this application.
FIG. 2 illustrates the encapsulation efficiency of lipid nanoparticles and cell transfection efficiency in this application, A is the encapsulation efficiency of lipid nanoparticles in the DOPE/DSPC group, and B is the transfection efficiency of lipid nanoparticles in the DOPE/DSPC group in HepG2 cells.

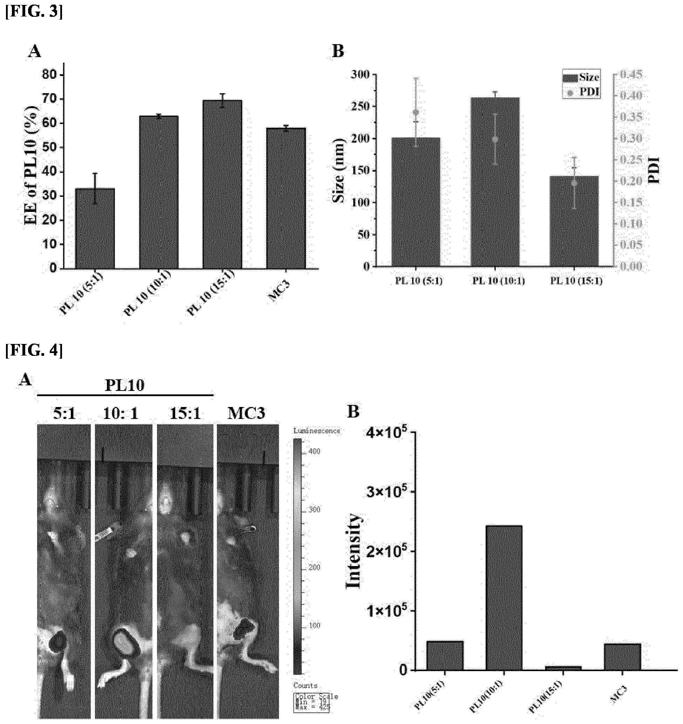

FIG. 3 illustrates the encapsulation efficiency and particle size of PL10 lipid nanoparticles with different N/P ratios in this application.
FIG. 4 illustrates in vivo transfection of PL10 lipid nanoparticles with different N/P ratios in this application (administered dose 3 µg Fluc-mRNA).

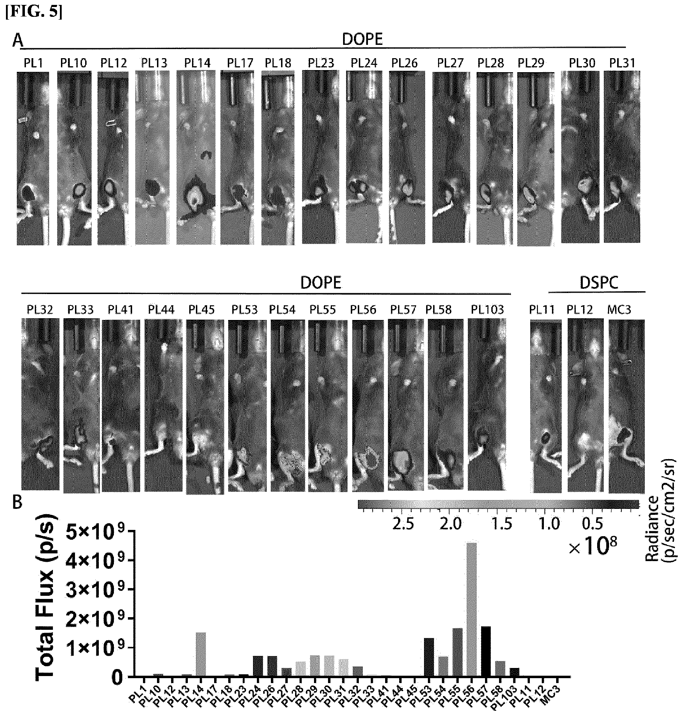

FIG. 5 illustrates in vivo transfection of different lipid nanoparticles in this application (administration dose: 3 µg Fluc-mRNA/mouse).

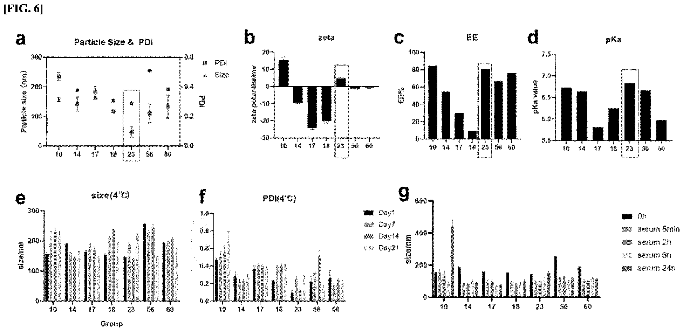

FIG. 6 illustrates physical properties of lipid nanoparticles PL LNPs constructed from representative biodegradable lipids in this application, introducing different hydrogen bond donors and biodegradable lipid tail structures. InFIG. 6, a)-f) show particle size, polydispersity (PDI), ζ potential, encapsulation efficiency, pKas, and plasma stability of biodegradable PL LNPs (i.e., PL10, PL14, PL17, PL18, PL23, PL56, PL60), respectively. In plasma stability assay, the LNPs are subjected to plasma for 24 h and the particle size is measured at different time points to assess the stability of the LNPs. Data are expressed as mean ± standard deviation.

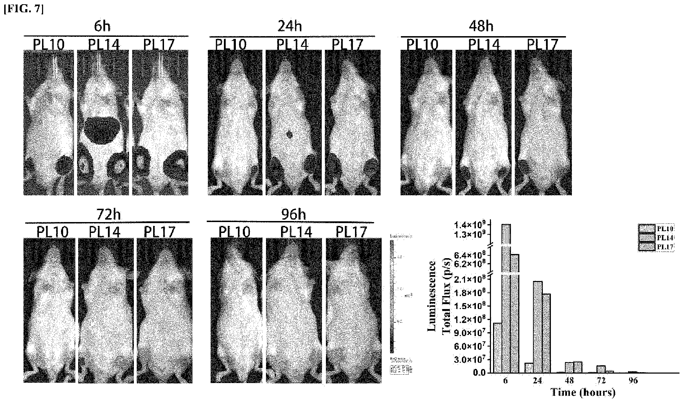

FIG. 7 illustrates delivery of Fluc-mRNA by intra-articular injection of exemplary lipid nanoparticles PL LNPs in this application. Images of luciferase expression in the joint cavity are detected with an IVIS imaging system at 6 h, 12 h, 24 h, 48 h, 72 h, and 96 h after intra-articular injection of 2 µg of Fluc-mRNA LNPs (PL10, PL14, and PL17, n=3).

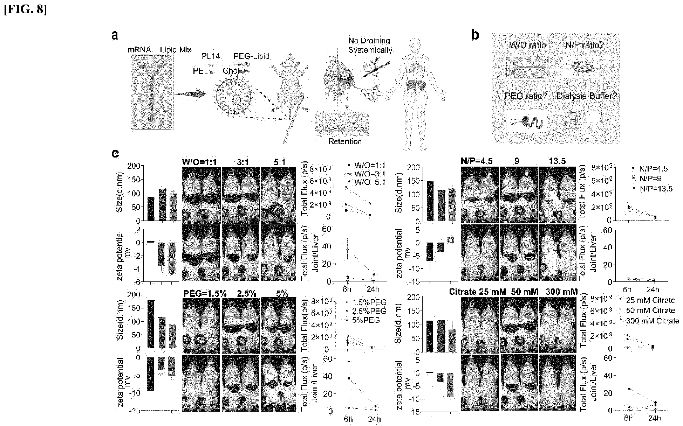

FIG. 8 illustrates a formulation for optimizing exemplary lipid nanoparticle PL14 LNPs in this application, to increase the specific accumulation of LNPs and mRNAs in the joint cavity while decreasing the expression in the liver. In the FIG. 8, a) shows a schematic diagram of a preparation method of nanoparticles; b) shows a graphical representation of the enhancement of Fluc-mRNA LNP accumulation in the joint cavity by adjusting a ratio of water to ethanol (W/O ratio), a N/P ratio, a percentage of PEG, and a concentration of sodium citrate buffer; and c) shows particle sizes and ζ potential of different formulations of LNP-Luc mRNA (2 µg of mRNA), and in vivo expression and distribution of different formulations of LNP-Luc mRNA (2 µg of mRNA) after administration in the joint cavities of ICR mice.

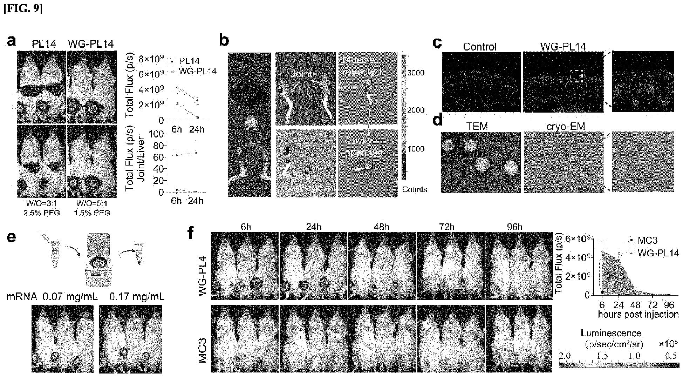

FIG. 9 illustrates a comparison of exemplary lipid nanoparticles PL14 LNPs and MC3 LNPs for intra-articular injection in this application. In the FIG. 9, a) shows comparison of in vivo expression and distribution of LNP-Luc mRNA between unoptimized and optimized formulations; b) shows luciferase expression and distribution in the joint cavity detected using an IVIS imaging system 24 h after intra-articular injection of 2 µg Fluc-mRNA LNPs; c) shows immunofluorescence co-staining of Luc in rat chondrocytes after intra-articular injection of LNP-Luc mRNA (2 µg mRNA); d) shows transmission electron microscopy (TEM) and cryo-electron microscopy (cryo-EM) images of LNPs; e) shows luciferase expression and distribution in the joint cavity after intra-articular injection of concentrated and unconcentrated LNP-Luc mRNA (2 µg mRNA); and f) shows comparison of the kinetics between optimized PL14 LNPs and MC3 LNPs.

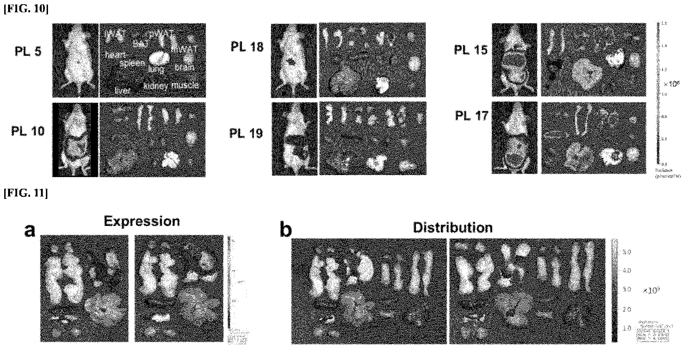

FIG. 10 illustrates the effect of Fluc-mRNA LNPs composed of different structural cationic lipids on the mRNA delivery efficiency after intraperitoneal administration in this application. a) PL lipid structures with a plurality of tertiary amino group structures can improve selective targeting of visceral adipocytes.
FIG. 11 illustrates expression and distribution of lipid nanoparticles LNPs with a plurality of tertiary amino groups in high-fat diet mice in this application. a) LNP mainly delivers FLuc mRNA to white adipose tissue (WAT). b) Collagenase I is used to separate adipocytes and non-adipocytes in water and passes through the cell filter. Luciferase assay is used to measure the expression of luciferase in each group. Strength is normalized by protein content.

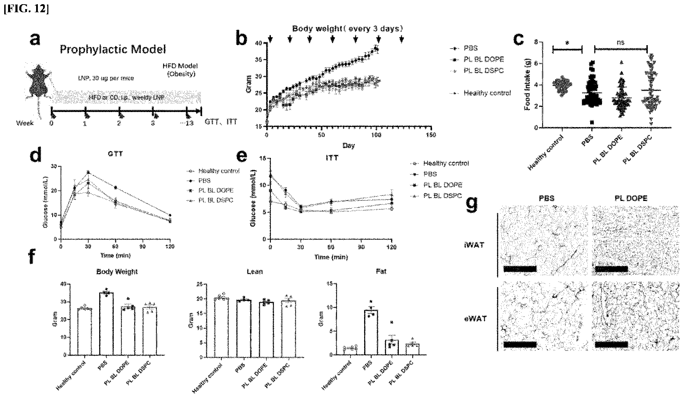

FIG. 12 illustrates lipid nanoparticles with a plurality of tertiary amino groups preventing obesity and improving metabolism in this application. In the FIG. 12, a) shows a schematic diagram of an experimental design, b) shows a weight curve before metabolic measurements interference, c) shows food intake in mice fed with high fat diet (HFD) or chow diet (CD), d) shows glucose tolerance test (GTT) results, e) shows insulin tolerance test (ITT) results, f) shows body weight, lean mass, and fat mass determined by QMR06-090H, and g) shows hematoxylin-eosin (HE) staining of inguinal white adipose tissue (iWAT) and epididymal white adipose tissue (eWAT).

### DETAILED DESCRIPTION

In the first aspect, this application provides a phosphatidylamine compound including a plurality of tertiary amino group structures, the phosphatidylamine compound being a compound represented by formula (I) or a stereoisomer, a prodrug, a pharmaceutically acceptable salt thereof: 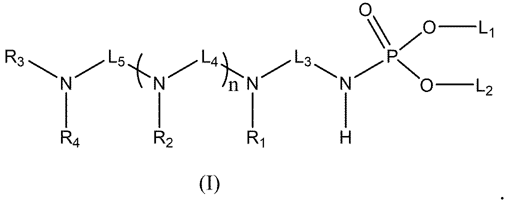
n in formula (I) is an integer from 0-5.
L₁ and L₂ are each independently C₅-C₃₀ alkyl, C₅-C₄₀ alkenyl, or -R'-M-R";
R₁ and R₂ are each independently C₄-C₃₀ alkyl, C₂-C₄₀ alkenyl, or -R'-M-R"; or R₁ and R₂ together with the respective attached N and L₄ form a 5 to 8-membered diazacycloalkylene;
R₃ and R₄ are each independently C₁-C₃₀ alkyl, C₂-C₄₀ alkenyl, or -R'-M-R"; or R₃ is H, C₁-C₃₀ alkyl, C₂-C₄₀ alkenyl, or -R'-M-R", and R₄ is C₁-C₃₀ alkyl, C₂-C₄₀ alkenyl, or -R'-M-R";
R' is C₁-C₂₄ alkylene or C₂-C₂₄ alkenylene;
M is selected from -C(O)-, -C(O)O-, -OC(O)-, -OC(O)O-, -C(S)-, -C(O)S-, -SC(O)-, -C(S)S-, -SC(S)-, -S(O)₂-, -S-S-, -C(O)N(R^{a})-, -N(R^{a})C(O)-, -OC(O)N(R^{a})-, -N(R^{a})C(O)O-, -N(R^{a})C(O)N(R^{a})-, -C(O-C(O)-R^{a})(H)-C₁₋₆ alkylene-O-C(O)-, -C(O)O-C₁₋₆ alkylene-C(O)-O-, -CH(OH)-, -P(O)(OR^{b})O-, C₆-C₁₀ arylene, and 5 to 10-membered heteroarylene;
R", R^{a}, and R^{b} are each independently selected from H, C₁-C₃₀ alkyl, and C₂-C₄₀ alkenyl; and
L₃, L₄, and L₅ are each independently C₁-C₁₂ alkylene, C₂-C₁₂ alkenylene, C₃-C₈ cycloalkylene, -A-Y-B-, -A-Y-, or -Y-B-, wherein A and B are each independently C₁-C₁₂ alkylene or C₂-C₁₂ alkenylene, and Y is -C(OH)-, -C(O)-, -C(O)O-, -OC(O)-, -C(O)N(H)-, -N(H)C(O)-, arylene, or heteroarylene.

In some embodiments of the first aspect, n is 0 and the compound represented by formula (I) is a compound represented by formula (I-1): 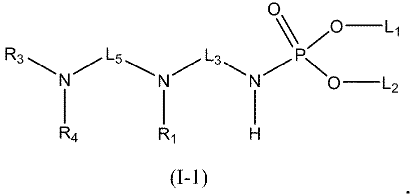
L₁ and L₂ in formula (I-1) are each independently C₅-C₃₀ alkyl, C₅-C₄₀ alkenyl, or -R'-M-R";
R₁ is C₄-C₃₀ alkyl, C₂-C₄₀ alkenyl, or -R'-M-R";
R₃ and R₄ are each independently C₁-C₃₀ alkyl, C₂-C₄₀ alkenyl, or -R'-M-R", or R₃ is H, C₁-C₃₀ alkyl, C₂-C₄₀ alkenyl, or -R '-M-R", and R₄ is C₁-C₃₀ alkyl, C₂-C₄₀ alkenyl, or -R'-M-R";
R' is C₁-C₂₄ alkylene or C₂-C₂₄ alkenylene;
M is selected from -C(O)-, -C(O)O-, -OC(O)-, -OC(O)O-, -C(S)-, -C(O)S-, -SC(O)-, -C(S)S-, -SC(S)-, -S(O)₂-, -S-S-, -C(O)N(R^{a})-, -N(R^{a})C(O)-, -OC(O)N(R^{a})-, -N(R^{a})C(O)O-, -N(R^{a})C(O)N(R^{a})-, -C(OC(O)R^{a})-C₁₋₆ alkylene-O-C(O)-, -C(O)-C₁₋₆ alkylene-C(O)-O-, -CH(OH)-, -P(O)(OR^{b})O-, C₆-C₁₉ arylene, and 5 to 10-membered heteroarylene;
R", R^{a}, and R^{b} are each independently selected from H, C₁-C₃₀ alkyl, and C₂-C₄₀ alkenyl.
L₃ and L₅ are each independently C₁-C₁₂ alkylene, C₂-C₁₂ alkenylene, C₃-C₈ cycloalkylene, -A-Y-B-, -A-Y-, or -Y-B-. A and B are each independently C₁-C₁₂ alkylene or C₂-C₁₂ alkenylene, and Y is -C(OH)-, -C(O)-, -C(O) O-, -OC(O)-, -C(O)N(H)-, -N(H)C(O)-, arylene, or heteroarylene.

In some embodiments of the first aspect, n is 1 and the compound represented by formula (I) is a compound represented by formula (I-2): 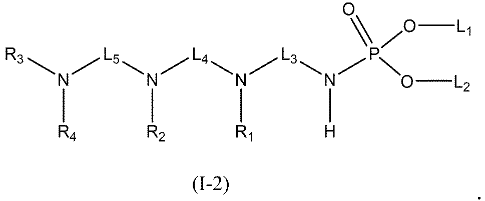
L₁ and L₂ are each independently C₅-C₃₀ alkyl, C₅-C₄₀ alkenyl, or -R'-M-R";
R₁ and R₂, together with their respective attached N and L₄, form a 5-membered to 8-membered diazacycloalkylene; or R₁ and R₂ are each independently C₄-C₃₀ alkyl, C₂-C₄₀ alkenyl, or -R'-M-R", L₄ is C₁-C₁₂ alkylene, C₂-C₁₂ alkenylene, C₃-C₈ cycloalkylene, -A-Y-B-, -A-Y-, or -Y-B-; wherein A and B are each independently C₁-C₁₂ alkylene or C₂-C₁₂ alkenylene, and Y is -C(OH)-, -C(O)-, -C(O)O-, -OC(O)-, -C(O)N(H)-, -N(H)C(O)-, arylene, or heteroarylene.
R₃ and R₄ are each independently C₁-C₃₀ alkyl, C₂-C₄₀ alkenyl, or -R'-M-R"; or R₃ is H, C₁-C₃₀ alkyl, C₂-C₄₀ alkenyl, or -R '-M-R", and R₄ is C₁-C₃₀ alkyl, C₂-C₄₀ alkenyl, or -R'-M-R".
R' is C₁-C₂₄ alkylene or C₂-C₂₄ alkenylene.
M is selected from -C(O)-, -C(O)O-, -OC(O)-, -OC(O)O-, -C(S)-, -C(O)S-, -SC(O)-, -C(S)S-, -SC(S)-, -S(O)₂-, -S-S-, -C(O)N(R^{a})-, -N(R^{a})C(O)-, -OC(O)N(R^{a})-, -N(R^{a})C(O)O-, -N(R^{a})C(O)N(R^{a})-, -C(OC(O)R^{a})-C₁₋₆ alkylene-O-C(O)-, -C(O)-C₁₋₆ alkylene-C(O)-O-, -CH(OH)-, -P(O)(OR^{b})O-, C₆-C₁₀ arylene, and 5 to 10-membered heteroarylene;
R", R^{a}, and R^{b} are each independently selected from H, C₁-C₃₀ alkyl, and C₂-C₄₀ alkenyl.
L₃ and L₅ are each independently C₁-C₁₂ alkylene, C₂-C₁₂ alkenylene, C₃-C₈ cycloalkylene, -A-Y-B-, -A-Y-, or -Y-B-; wherein A and B are each independently C₁-C₁₂ alkylene or C₂-C₁₂ alkenylene, and Y is -C(OH)-, -C(O)-, -C(O)O -, -OC(O)-, -C(O)N(H)-, -N(H)C(O)-, arylene, or heteroarylene.

In some embodiments of the first aspect, L₁ and L₂ are each independently C₆-C₃₀ alkyl, C₆-C₄₀ alkenyl, or -R'-M-R"; merely by way of example; wherein R' is C₁-C₂₄ alkylene or C₂-C₂₄ alkenylene, M is selected from -C(O)-, -C(O)O-, -OC(O)-, -OC(O)O-, -C(S)-, -C(O)S-, -SC(O)-, -C(S)S-, -SC(S)-, -S(O)₂-, -S-S-, -C(O)N(R^{a})-, -N(R^{a})C(O)-, -OC(O)N(R^{a})-, -N(R^{a})C(O)O-, -N(R^{a})C(O)N(R^{a})-, -C(OC(O)R^{a})-CH₂-O-C(O)-, -C(O)O-CH(CH₃)CH₂-C(O)-O-, -CH(OH)-, -P(O)(OR^{b})O-, C₆-C₁₀ arylene, and 5 to 10-membered heteroarylene, R" is C₁-C₃₀ alkyl or C₂-C₄₀ alkenyl, and R^{a} and R^{b} are each independently selected from H, C₁-C₃₀ alkyl and C₂-C₄₀ alkenyl.

In some embodiments of the first aspect, L₁ and L₂ are each independently C₆-C₂₄ alkyl, C₆-C₂₄ alkenyl, or -R'-M-R", R' is C₁-C₂₄ alkylene or C₂-C₁₀ alkenyl, M is -C(O)-, -C(O)O-, -OC(O)-, -C(OC(O)R^{a})-CH₂-O-C(O)-, -C(O)O-CH(CH₃)CH₂-C(O)-O-, or -CH(OH)-, and R^{a} or R" is C₁-C₃₀ alkyl or C₂-C₄₀ alkenyl.

In some embodiments of the first aspect, L₁ and L₂ are each independently one of the following structures:

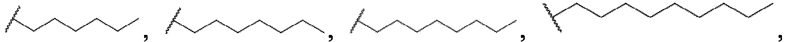

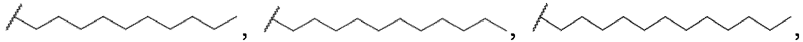

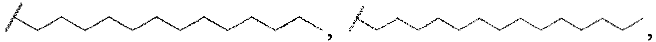

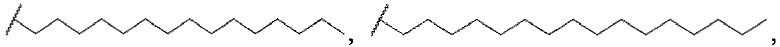

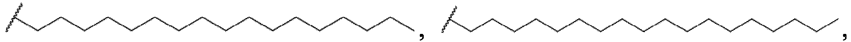

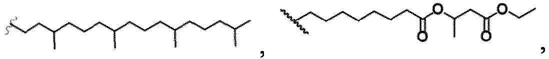

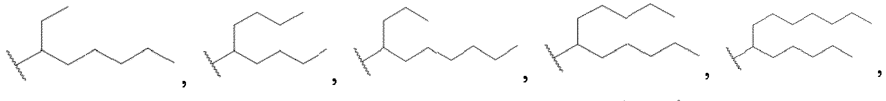

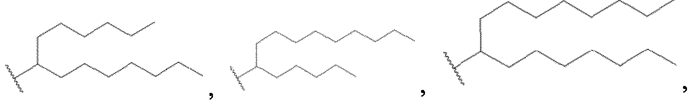

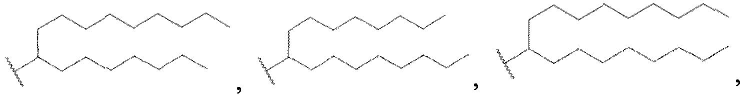

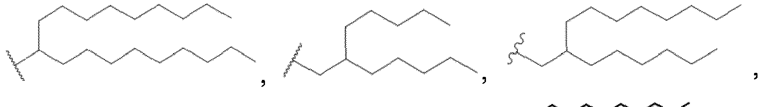

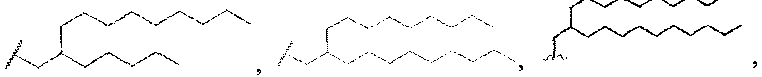

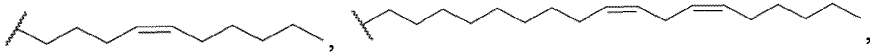

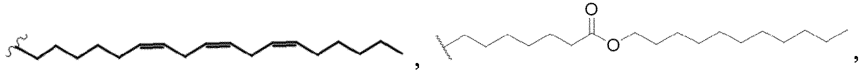

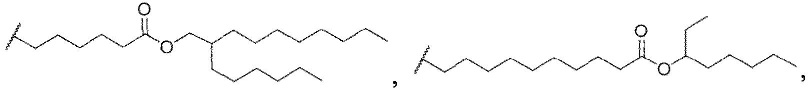

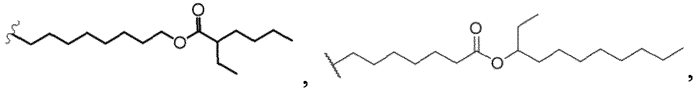

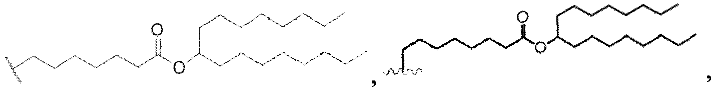

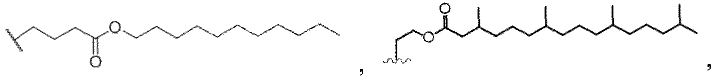

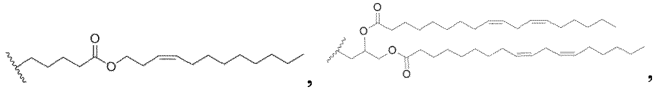

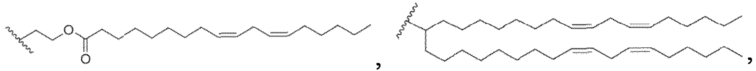

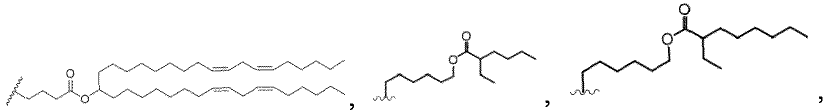

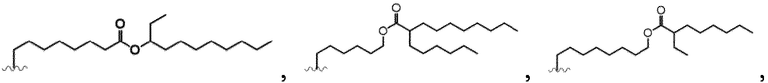

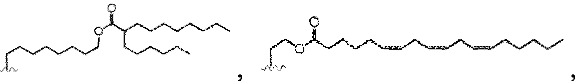

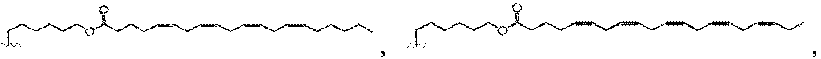

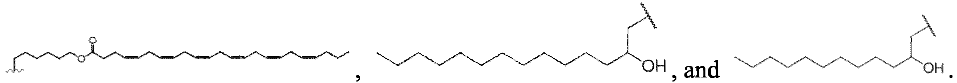

In some embodiments of the first aspect, L₁ and L₂ are each independently one of the following structures: 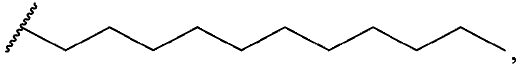 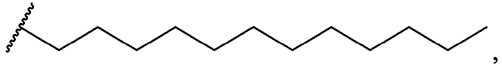 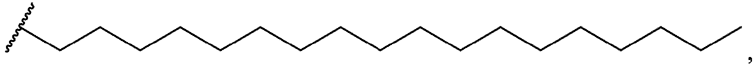 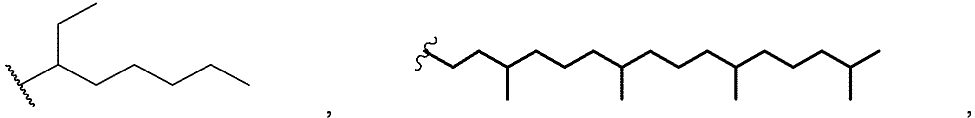 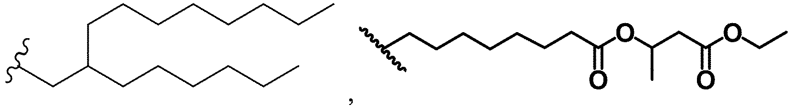 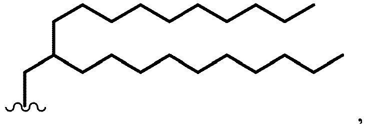 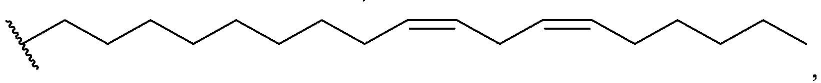 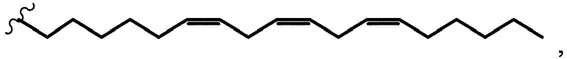 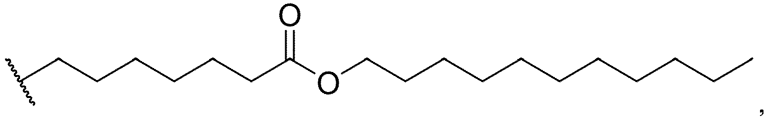 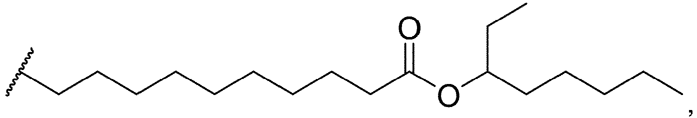 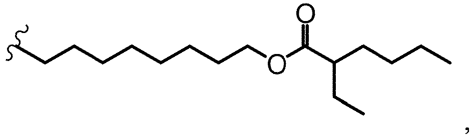 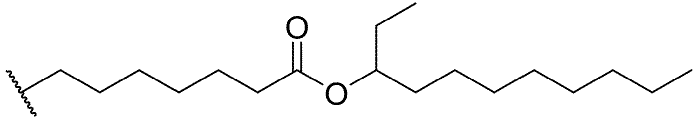 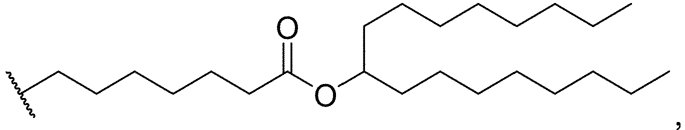 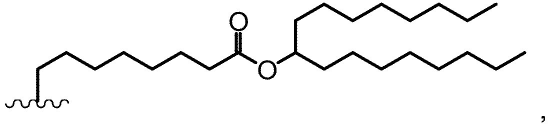 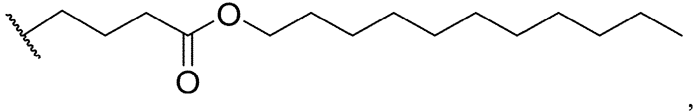 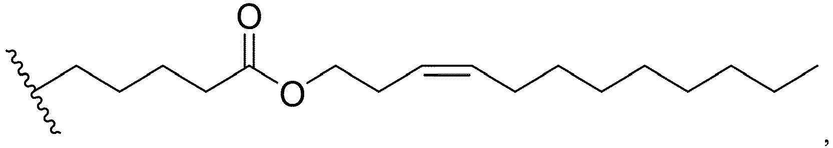 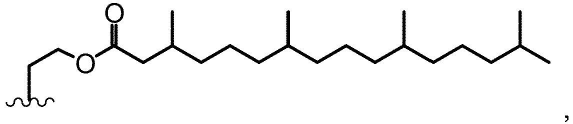 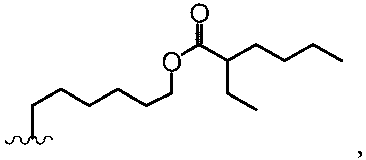 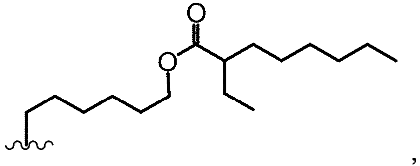 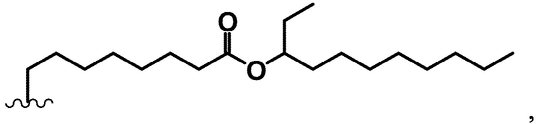 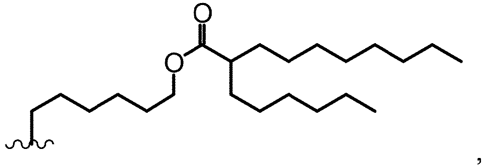 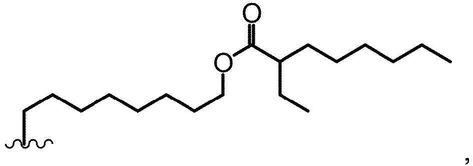 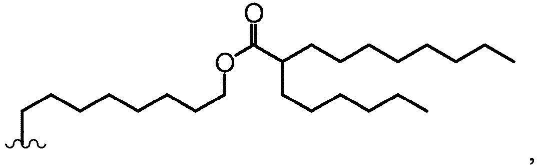 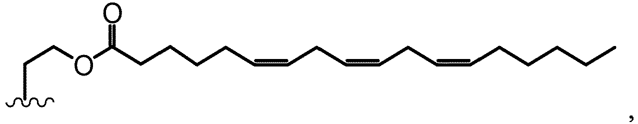 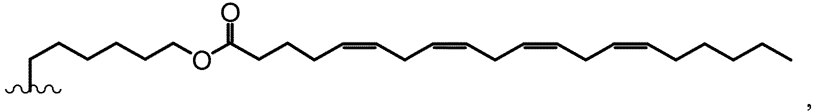 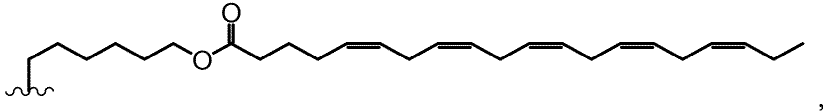 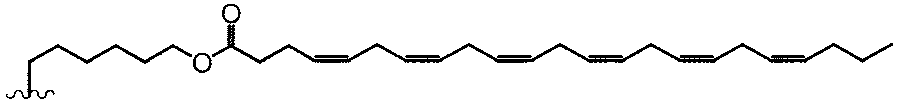 and

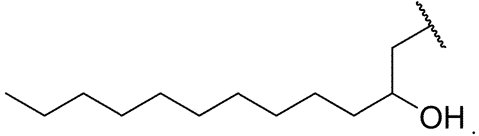

In some embodiments of the first aspect, R₁ and R₂ are each independently one of the following structures: 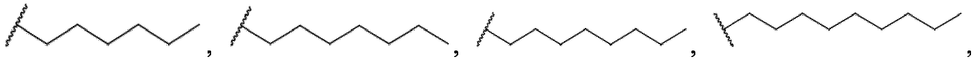 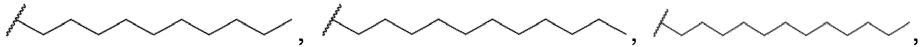 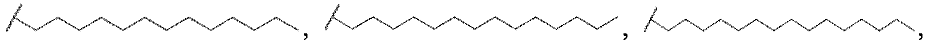 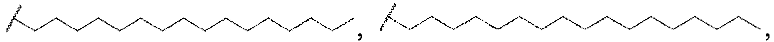 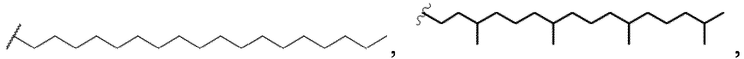 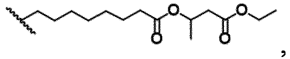 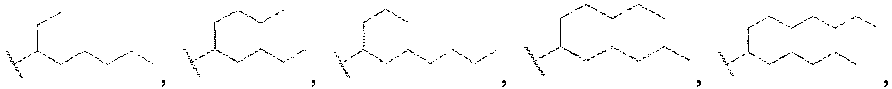 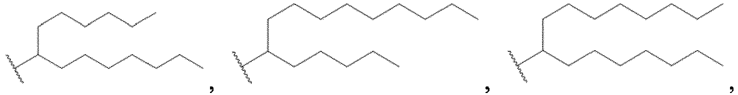 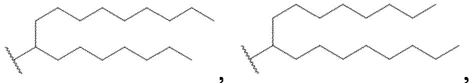 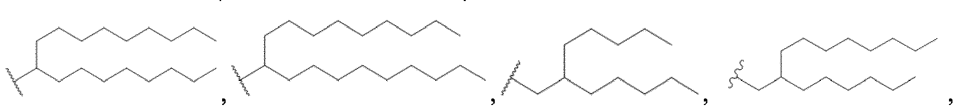 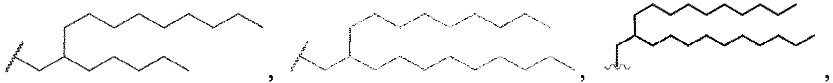 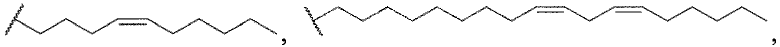 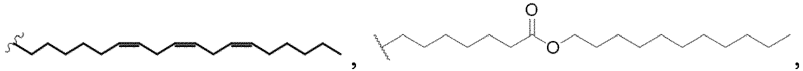 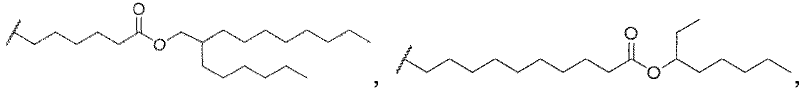 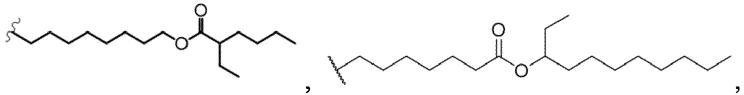 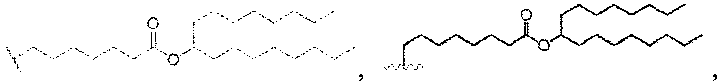 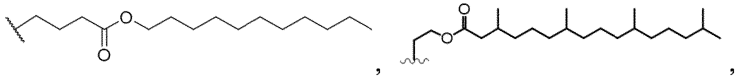 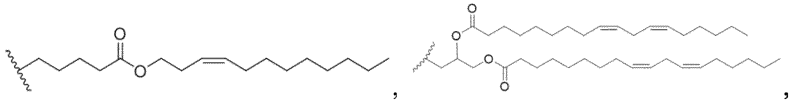 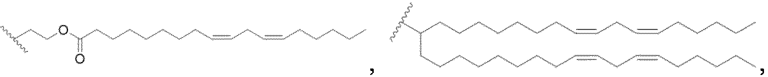 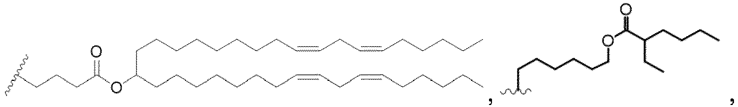 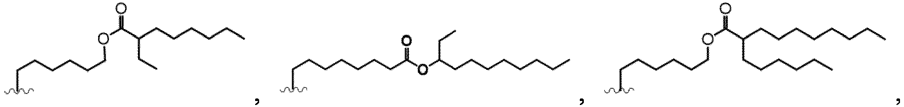 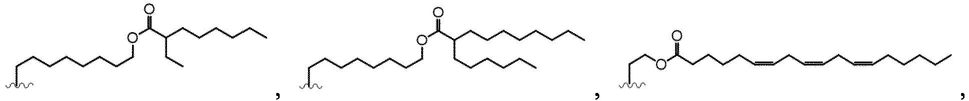 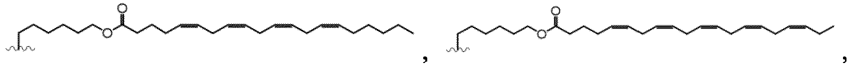 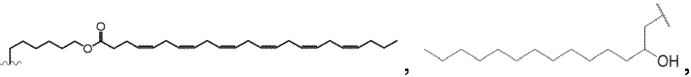 and

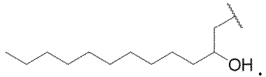

In some embodiments of the first aspect, R₁ and R₂ are each independently one of the following structures:

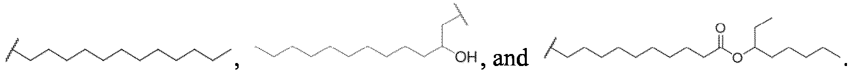

In some embodiments of the first aspect, R₁ and R₂, together with their respective attached N and L₄, form one of the following groups that is optionally substituted:

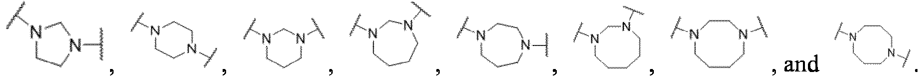

In some embodiments of the first aspect, R₁ and R₂, together with their respective attached N and L₄, form the following groups that is optionally substituted:

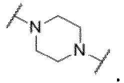

In some embodiments of the first aspect, L₃ and L₅, or L₃, L₄, and L₅ (when L₄ is present) are each independently methylene, ethylene, propylene, butylene, pentylene, hexylene, vinylidene, propenylene, butenylene, pentenylene, hexenylene, cyclopropylene, cyclobutylene, cyclobutylene, cyclohexylene, cycloheptylene, -A-Y-B-, -A-Y-, or -Y-B-. A and B are each independently methylene, ethylene, propylene, butylene, pentylene, hexylene, vinylidene, propenylene, butenylene, pentenylene, or hexenylene, and Y is -C(OH)-, -C(O)-, -C(O)O-, -OC(O)-, -C(O)N(H)-, -N(H)C(O)-, phenylene, pyrroloylene, imidazolylene, thiazolylene, thienylene, furylene, pyridylene, or pyrimidylene.

In some embodiments of the first aspect, L₃ and L₅, or L₃, L₄ and L₅ (when L₄ is present) are each independently -CH₂-, -CH₂CH₂-, -CH(CH₃)CH₂-, -CH₂CH(CH₃)-, -CH₂CH₂CH₂-, -CH(CH₃)CH₂CH₂-, -CH₂CH(CH₃)CH₂-, -CH₂CH₂CH(CH₃)-, -CH₂CH₂CH₂CH₂-, -CH(CH₃)CH₂CH₂CH₂-, -CH₂CH(CH₃)CH₂CH₂-, -CH₂CH₂CH₂CH(CH₃)-, -A-Y-B-, -A-Y-, or -Y-B-. A and B are each independently -CH₂-, -CH₂CH₂-, -CH(CH₃)CH₂-, -CH₂CH(CH₃)-, -CH₂CH₂CH₂-, -CH(CH₃)CH₂CH₂-, -CH₂CH(CH₃)CH₂-, or -CH₂CH₂CH(CH₃)-, and Y is -C(OH)-, -C(O)-, -C(O)O-, -OC(O)-, -C(O)N(H)-, -N(H)C(O)-, or phenylene.

In some embodiments of the first aspect, L₃ is -CH₂-, -CH₂CH₂-, -CH(CH₃)CH₂-, -CH₂CH(CH₃)-, -CH₂CH₂CH₂-, -CH(CH₃)CH₂CH₂-, -CH₂CH(CH₃)CH₂-, -CH₂CH₂CH(CH₃)-, -CH₂CH₂CH₂CH₂-, -CH(CH₃)CH₂CH₂CH₂-, -CH₂CH(CH₃)CH₂CH₂-, -CH₂CH₂CH₂CH(CH₃)-, -A-Y-B-, -A-Y-, or -Y-B-. A and B are each independently -CH₂-, -CH₂CH₂-, -CH(CH₃)CH₂-, -CH₂CH(CH₃)-, -CH₂CH₂CH₂-, -CH(CH₃)CH₂CH₂-, -CH₂CH(CH₃)CH₂-, or -CH₂CH₂CH(CH₃)-, and Y is -C(OH)-, -C(O)-, -C(O)O-, -OC(O)-, -C(O)N(H)-, or -N(H)C(O)-.

In some embodiments of the first aspect, L₃ is -CH₂CH₂- or -CH₂CH₂CH₂-.

In some embodiments of the first aspect, L₄ is -CH₂-, -CH₂CH₂-, -CH(CH₃)CH₂-, -CH₂CH(CH₃)-, -CH₂CH₂CH₂-, -CH(CH₃)CH₂CH₂-, -CH₂CH(CH₃)CH₂-, -CH₂CH₂CH(CH₃)-, -CH₂CH₂CH₂CH₂-, -CH(CH₃)CH₂CH₂CH₂-, -CH₂CH(CH₃)CH₂CH₂-, -CH₂CH₂CH₂CH(CH₃)-, -A-Y-B-, -A-Y-, or -Y-B-. A and B are each independently -CH₂-, -CH₂CH₂-, -CH(CH₃)CH₂-, -CH₂CH(CH₃)-, -CH₂CH₂CH₂-, -CH(CH₃)CH₂CH₂-, -CH₂CH(CH₃)CH₂-, or -CH₂CH₂CH(CH₃)-, and Y is -C(OH)-, -C(O)-, -C(O)O-, -OC(O)-, -C(O)N(H)-, or -N(H)C(O)-.

In some embodiments of the first aspect, L₄ is -CH₂CH₂- or -CH₂CH₂CH₂-.

In some embodiments of the first aspect, L₅ is -CH₂-, -CH₂CH₂-, -CH(CH₃)CH₂-, -CH₂CH(CH₃)-, -CH₂CH₂CH₂-, -CH(CH₃)CH₂CH₂-, -CH₂CH(CH₃)CH₂-, -CH₂CH₂CH(CH₃)-, -CH₂CH₂CH₂CH₂-, -CH (CH₃)CH₂CH₂CH₂-, -CH₂CH(CH₃)CH₂CH₂-, -CH₂CH₂CH₂CH(CH₃)-, -A-Y-B-, -A-Y-, or -Y-B-. A and B are each independently -CH₂-, -CH₂CH₂-, -CH(CH₃)CH₂-, -CH₂CH(CH₃)-, - CH₂CH₂CH₂-, -CH(CH₃)CH₂CH₂-, -CH₂CH(CH₃)CH₂-, or -CH₂CH₂CH(CH₃)-, and Y is -C(OH)-, -C(O)-, -C(O)O-, -OC(O)-, -C(O)N(H)-, or -N(H)C(O)-, or phenylene.

In some embodiments of the first aspect, L₅ is -CH₂CH₂-, -CH₂CH₂CH₂-, -CH₂CH(OH)CH₂-, -CH₂CH₂-C(O)O-CH₂CH₂CH₂-, -CH₂CH₂-C(O)N(H)-CH₂CH₂CH₂-, or

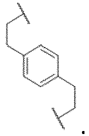

In some embodiments of the first aspect, R₃ and R₄ are each independently C₁-C₂₄ alkyl, C₂-C₂₀ alkenyl, or -R'-M-R"; or R₃ is H, C₁-C₃₀ alkyl, C₂-C₄₀ alkenyl, or -R '-M-R", and R₄ is C₁-C₃₀ alkyl, C₂-C₄₀ alkenyl, or -R'-M-R".

R' is C₁-C₂₄ alkylene or C₂-C₂₄ alkenylene.

M is selected from -C(O)-, -C(O)O-, -OC(O)-, -OC(O)O-, -C(S)-, -C(O)S-, -SC(O)-, -C(S)S-, -SC(S)-, -S(O)₂-, -S-S-, -C(O)N(R^{a})-, -N(R^{a})C(O)-, -OC(O)N(R^{a})-, -N(R^{a})C(O)O-, -N(R^{a})C(O)N(R^{a})-, -C(OC(O)R^{a})-C₁₋₆ alkylene-O-C(O)-, -C(O)-C₁₋₆ alkylene-C(O)-O-, -CH(OH)-, -P(O)(OR^{b})O-, C₆-C₁₀ arylene, and 5 to 10-membered heteroarylene.

R", R^{a}, and R^{b} are each independently selected from H, C₁-C₃₀ alkyl, and C₂-C₄₀ alkenyl.

In some embodiments of the first aspect, R₃ and R₄ are each independently methyl, ethyl, propyl, butyl, pentyl, or hexyl. In some embodiments of the first aspect, R₃ and R₄ are both methyl or ethyl.

In some embodiments of the first aspect, R₃ and R₄ are each independently C₆-C₃₀ alkyl, C₆-C₄₀ alkenyl, or -R'-M-R", R' is C₁-C₂₄ alkylene or C₂-C₂₄ alkenylene; M is -C(O)-, -C(O)O-, -OC(O)-, -C(OC(O)R^{a})-CH₂-O-C(O)-, -C(O)-CH(CH₃)CH₂-C(O)-O-, or -CH(OH)-; and R^{a} or R" is C₁-C₃₀ alkyl or C₂-C₄₀ alkenyl.

In some embodiments of the first aspect, R₃, R₄ are each independently one of the following structures: 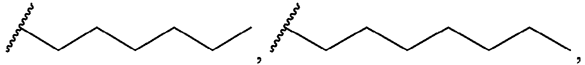 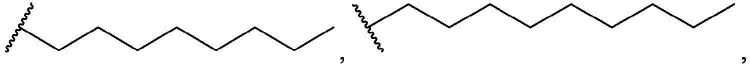 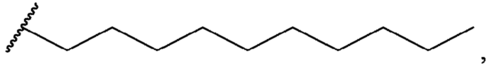 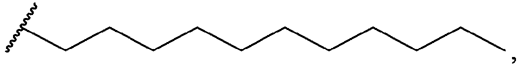 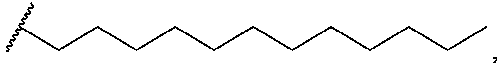 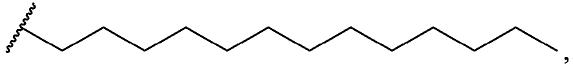 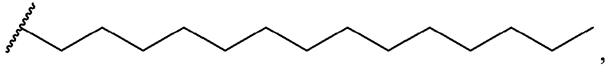 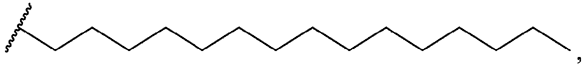 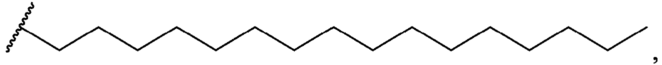 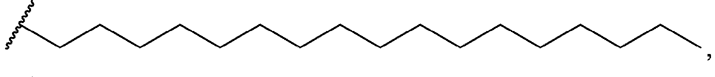 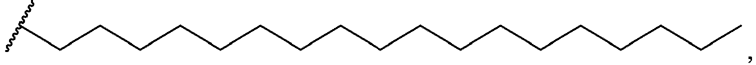 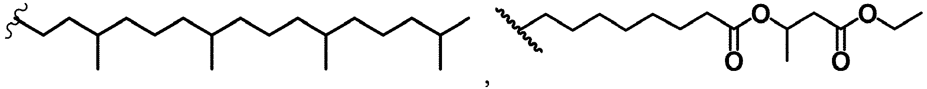 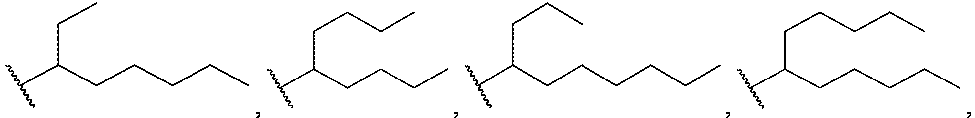 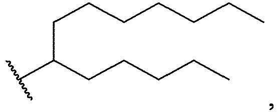 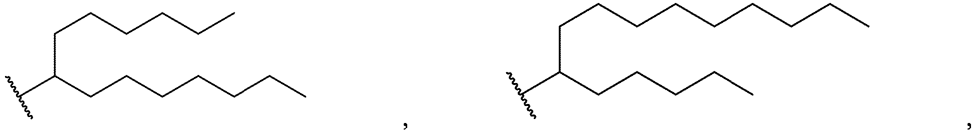 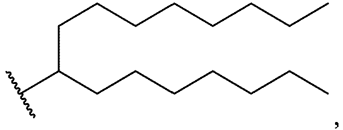 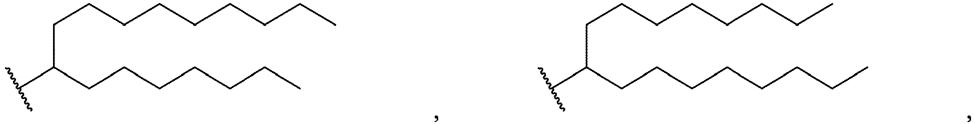 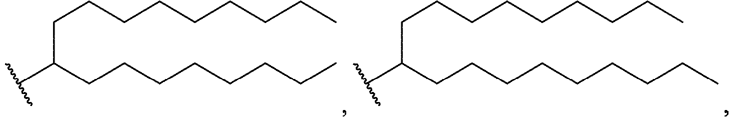 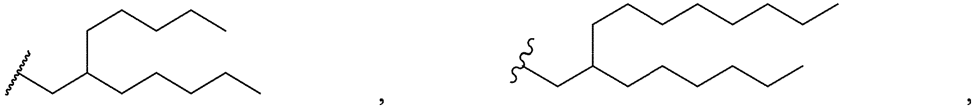 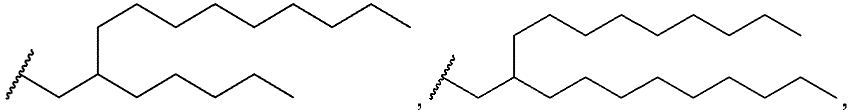 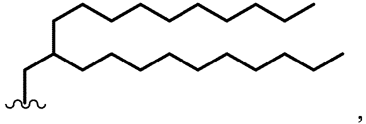 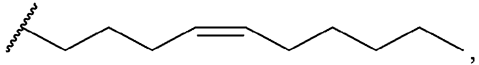 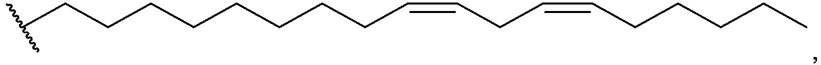 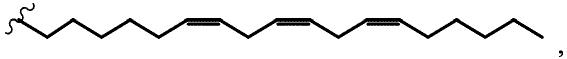 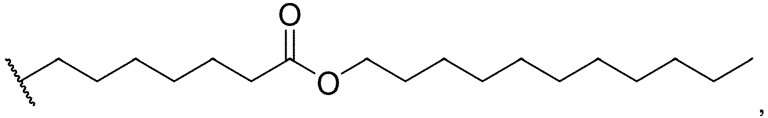 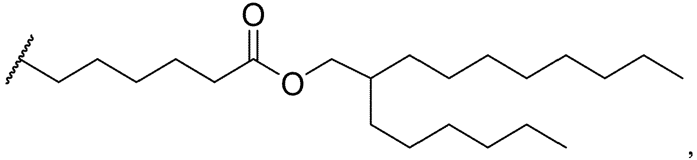 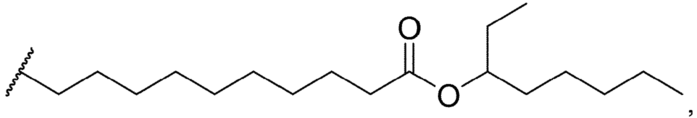 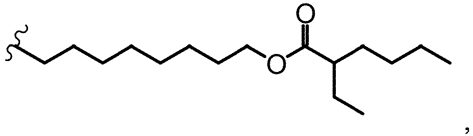 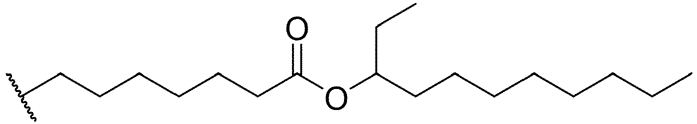 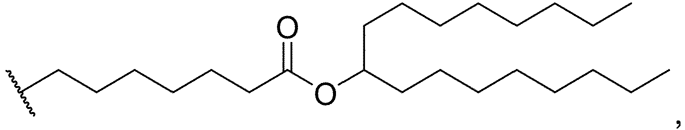 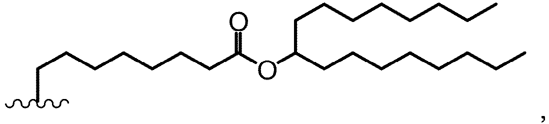 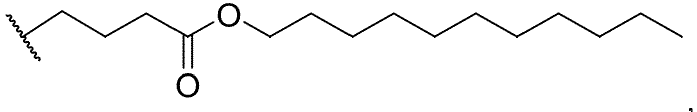 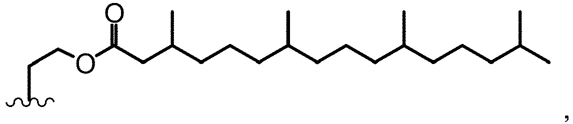 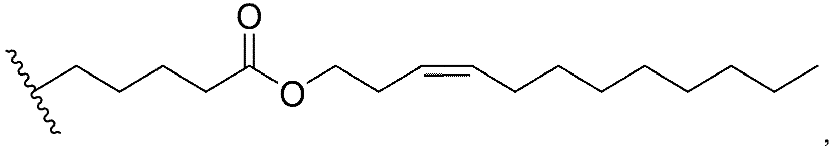 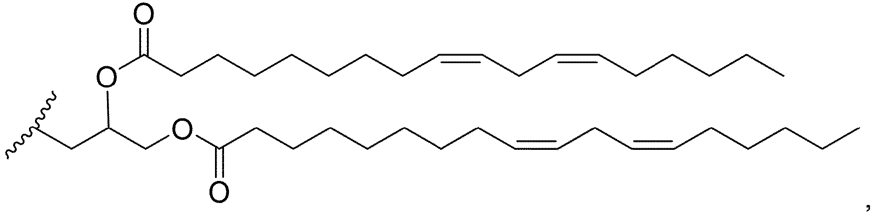 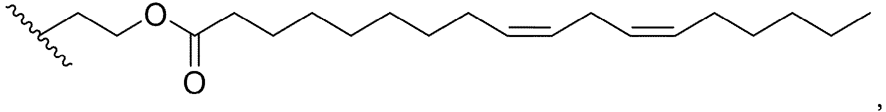 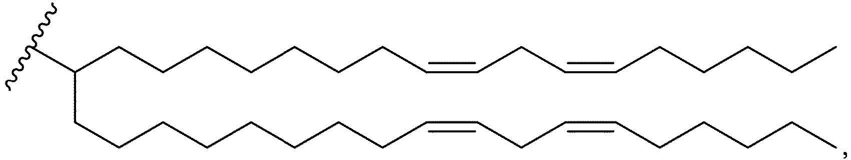 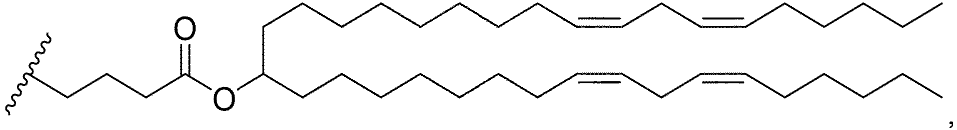 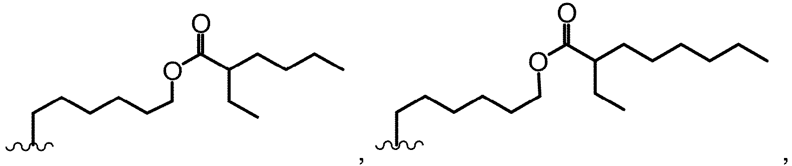 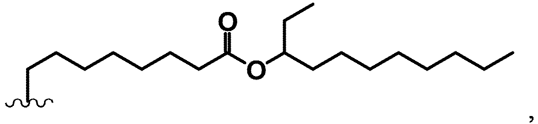 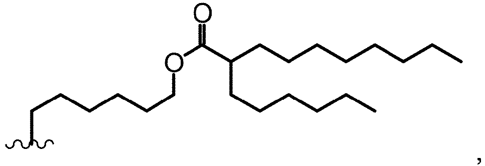 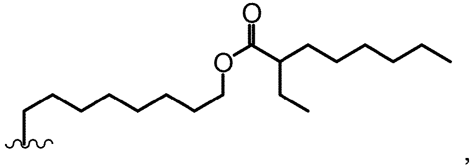 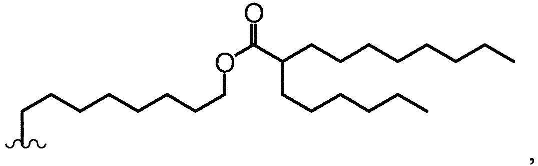 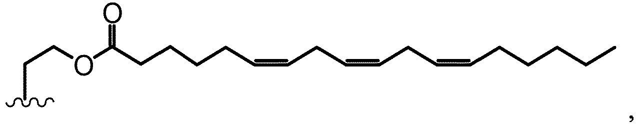 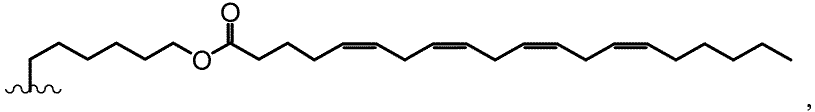 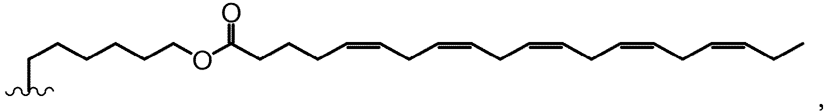 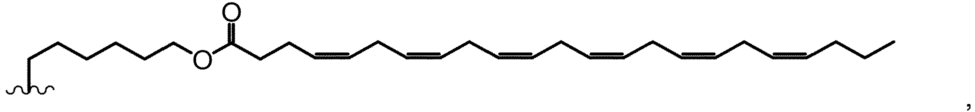 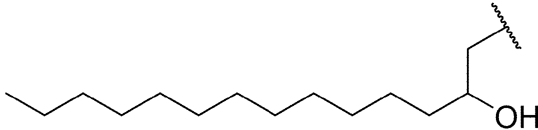 and

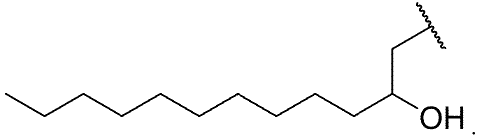

In some embodiments of the first aspect, R₃ and R₄ are each independently

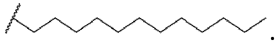

In some embodiments of the first aspect, n is 0, R₁, R₃, R₄, L₁, and L₂ are each independently one of the above structures. 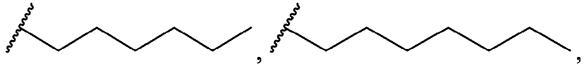 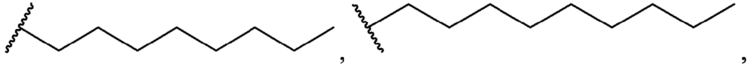 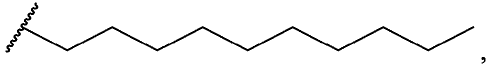 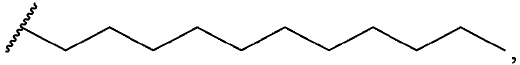 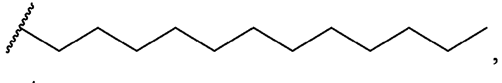 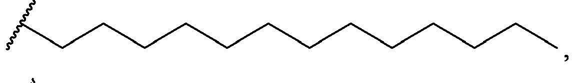 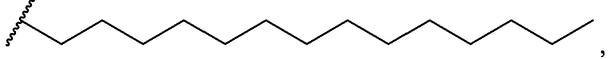 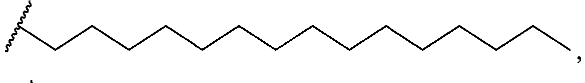 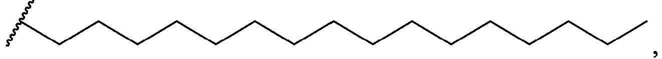 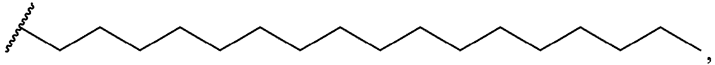 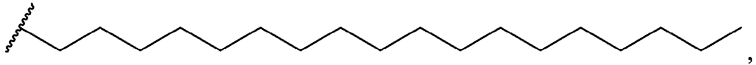 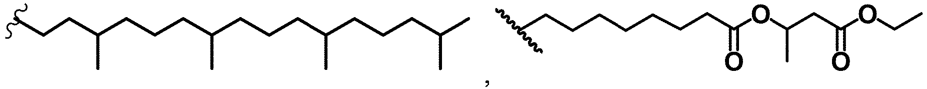 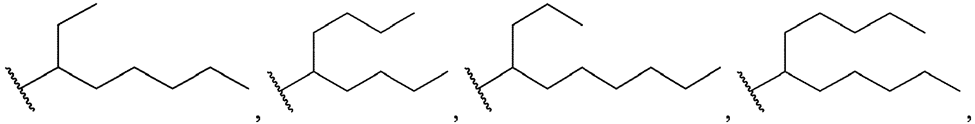 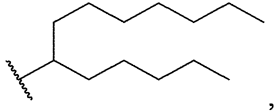 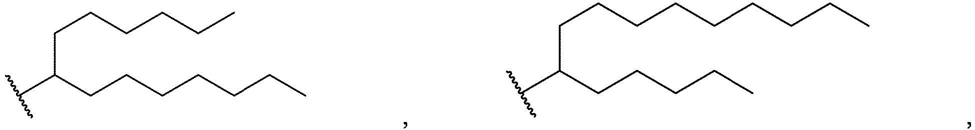 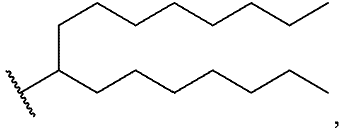 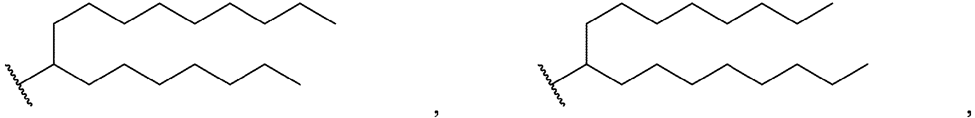 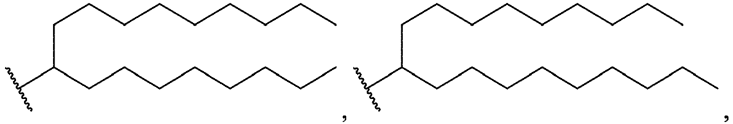 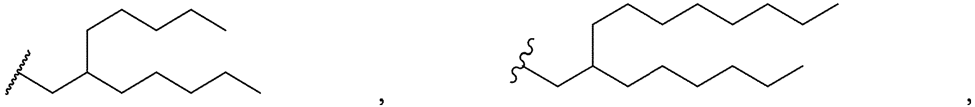 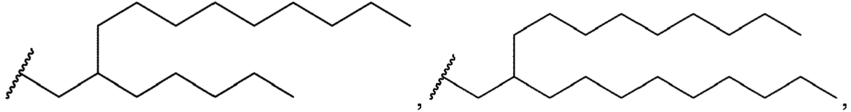 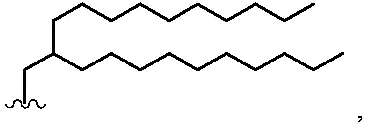 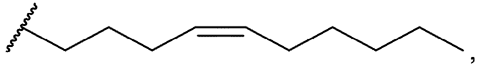 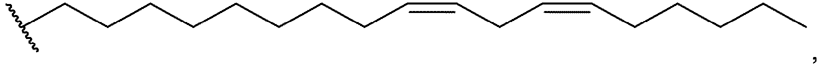 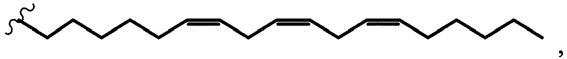 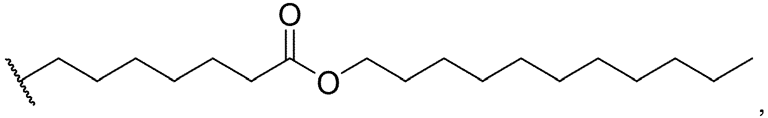 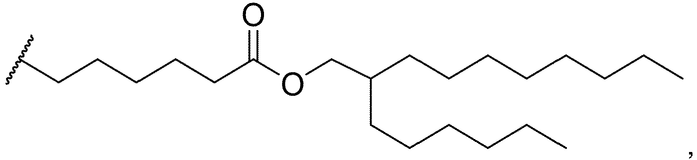 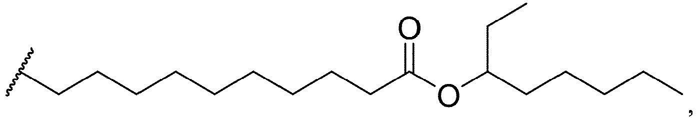 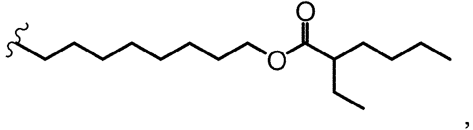 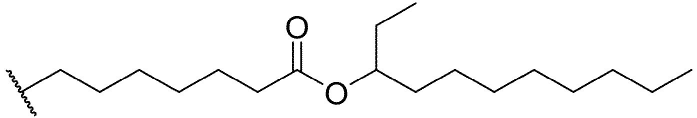 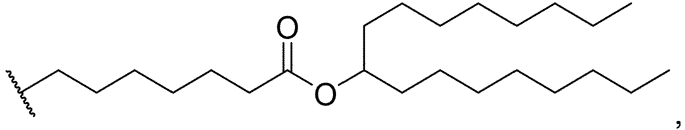 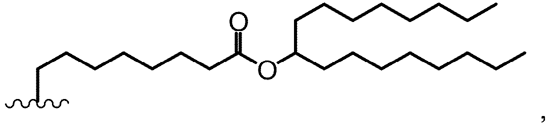 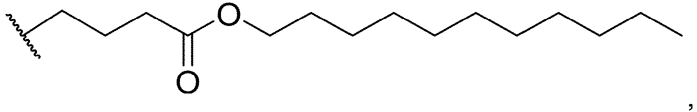 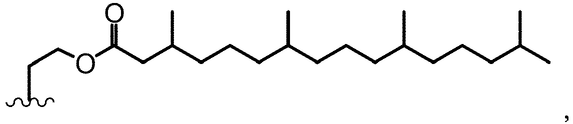 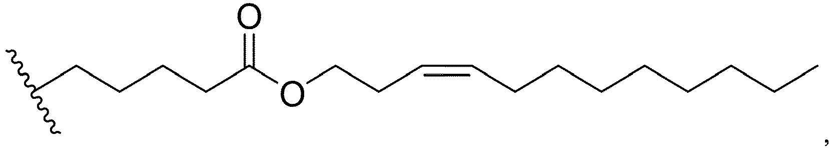 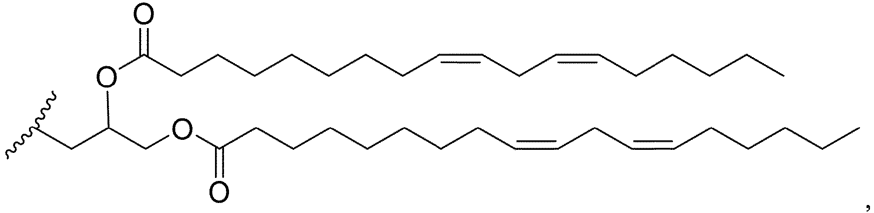 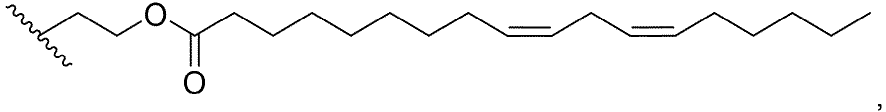 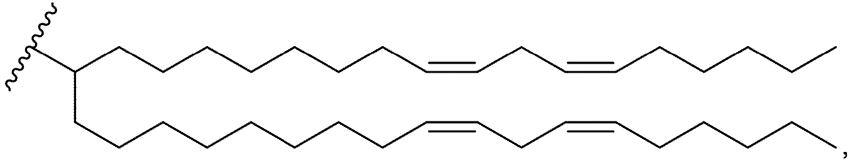 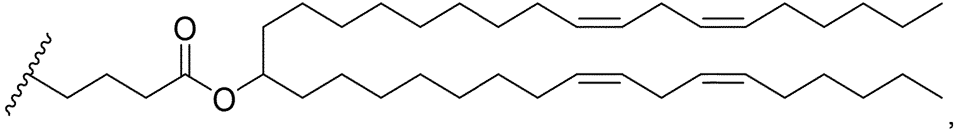 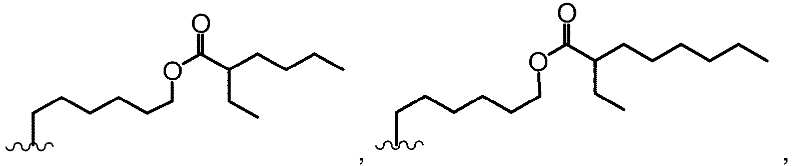 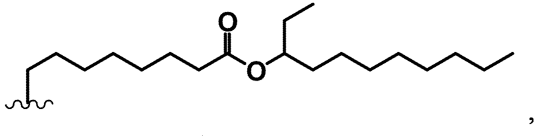 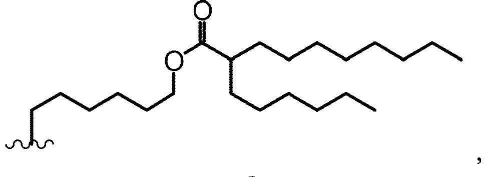 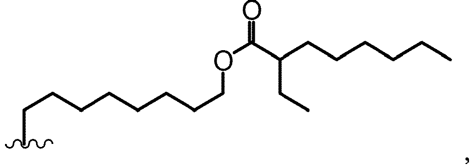 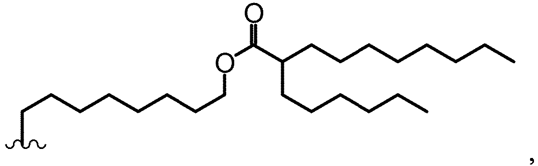 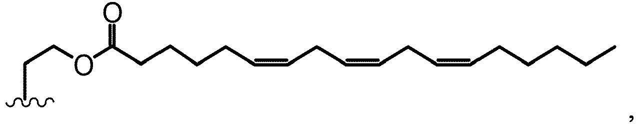 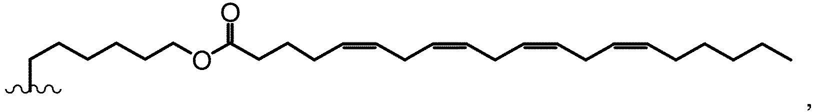 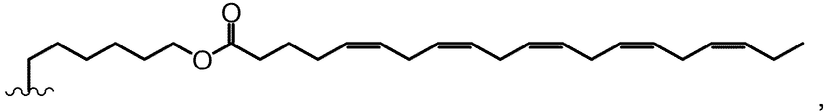 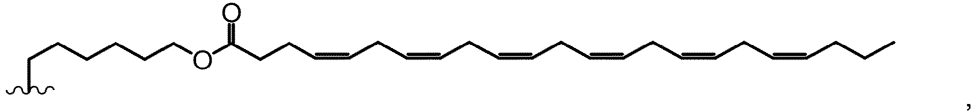 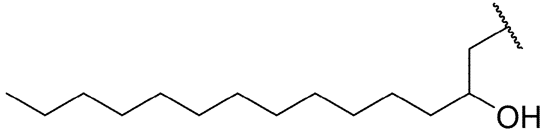 and 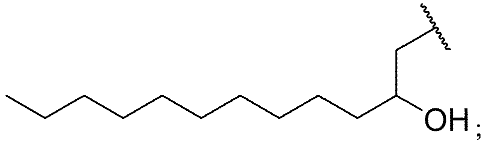
Or, R₃ and R₄ are both methyl, and R₁, L₁ and L₂ are defined by the above structures;
L₃ is -CH₂CH₂- or -CH₂CH₂CH₂-;
L₅ is -CH₂CH₂-, -CH₂CH₂CH₂-, -CH₂CH₂-C(O)O-CH₂CH₂CH₂-, -CH₂CH₂-C(O)N(H)-CH₂CH₂CH₂-, -CH₂CH(OH)CH₂-, or

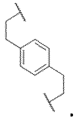

In some embodiments of the first aspect, n is 1; R₁ and R₂, together with their respective attached N and L₄, form the following groups that is optionally substituted: 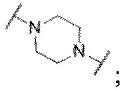
L₃ is -CH₂CH₂- or -CH₂CH₂CH₂-;
L₅ is -CH₂CH₂-, -CH₂CH₂CH₂-, -CH₂CH₂-C(O)O-CH₂CH₂CH₂-, -CH₂CH₂-C(O)N(H)-CH₂CH₂CH₂-, or -CH₂CH(OH)CH₂-.
R₃, R₄, L₁ and L₂ are each independently one of the following structures: 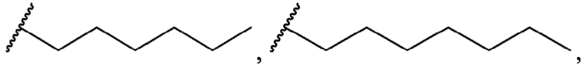 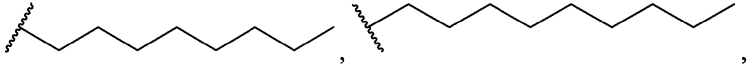 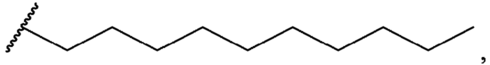 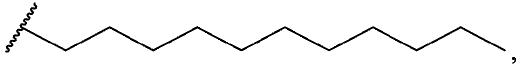 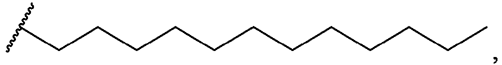 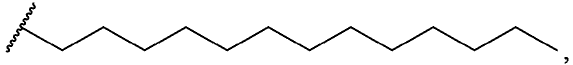 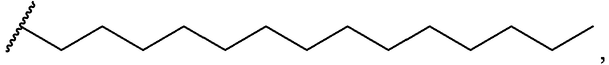 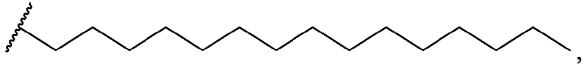 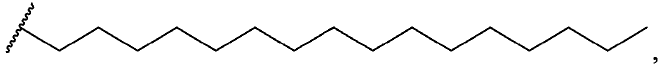 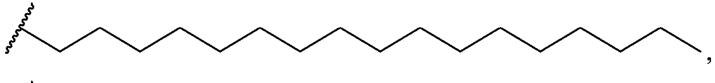 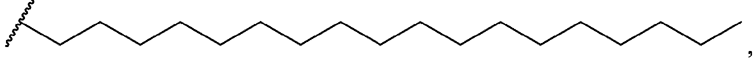 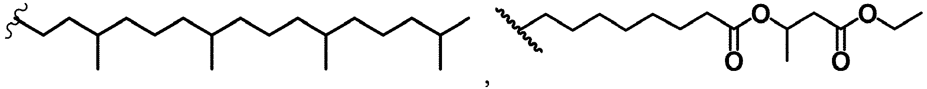 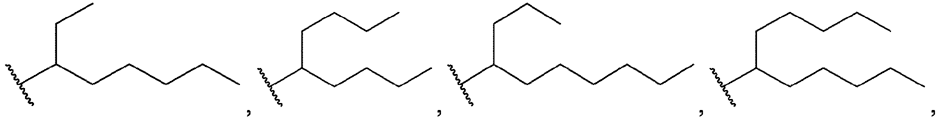 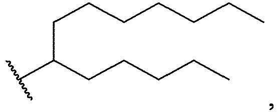 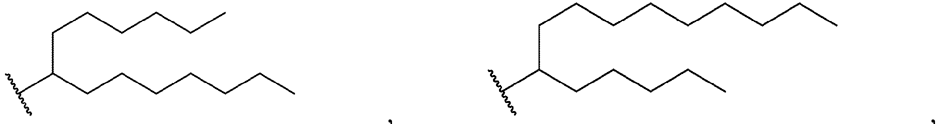 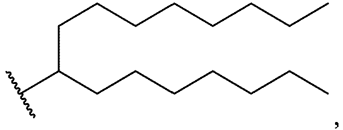 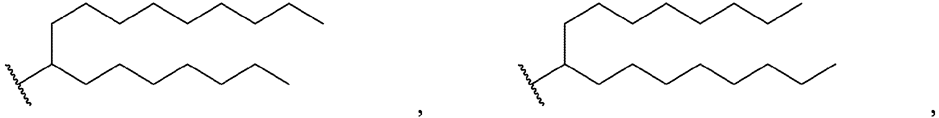 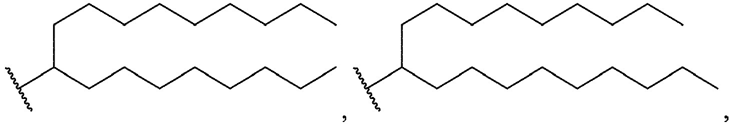 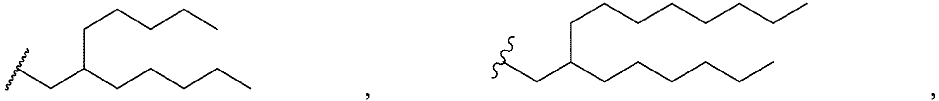 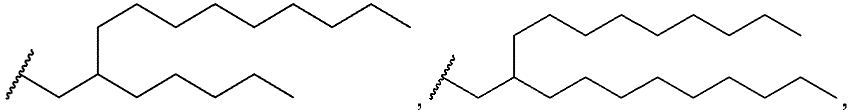 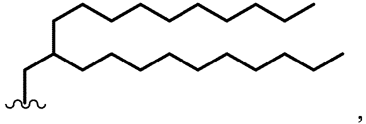 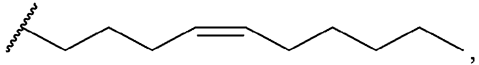 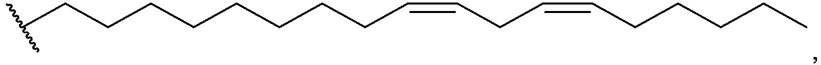 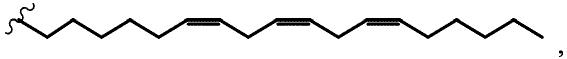 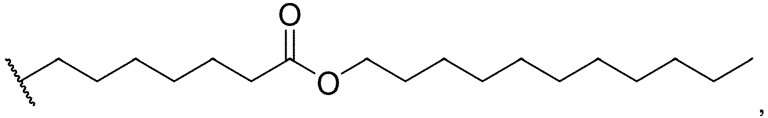 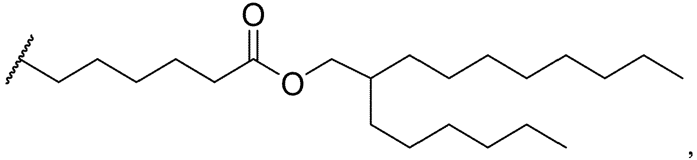 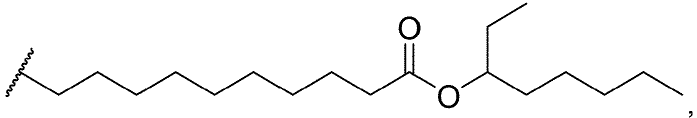 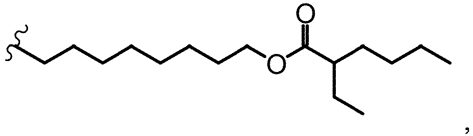 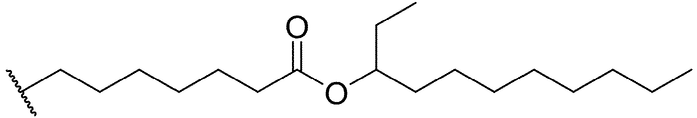 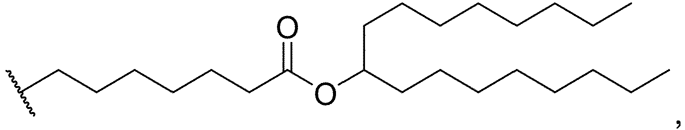 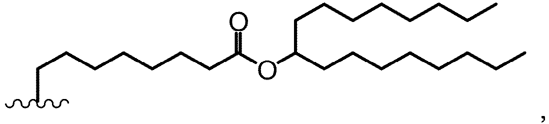 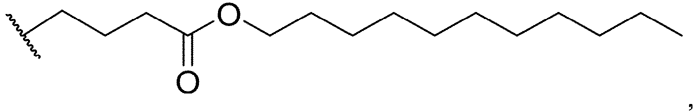 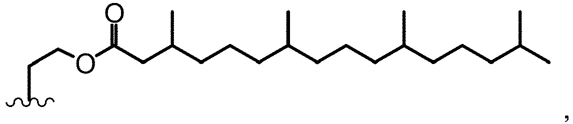 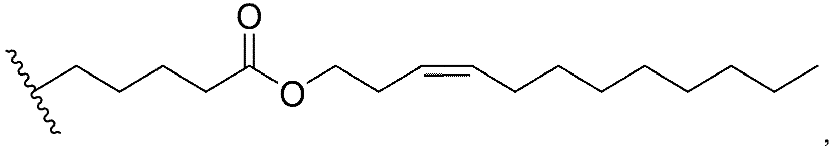 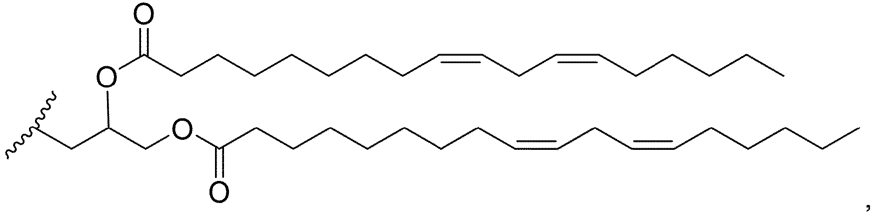 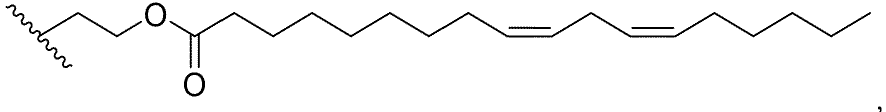 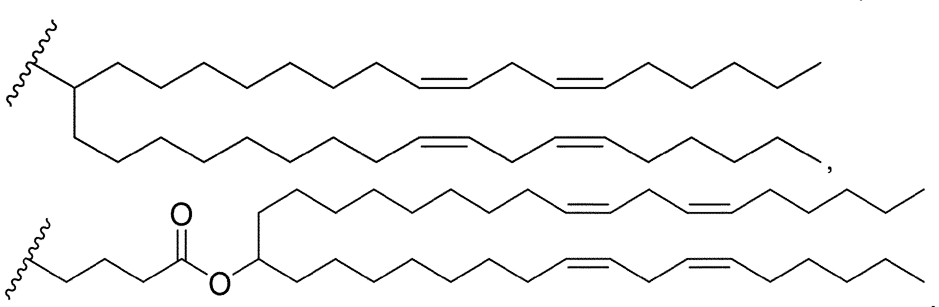 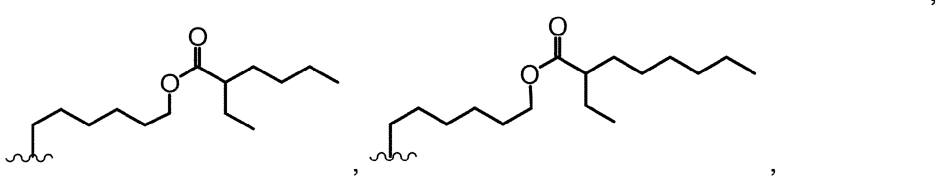 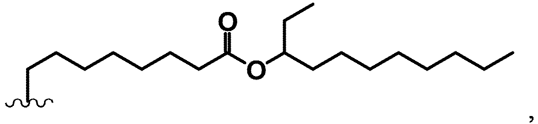 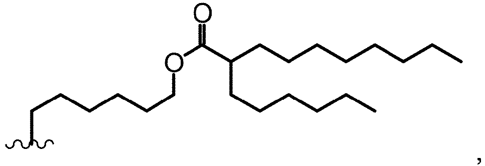 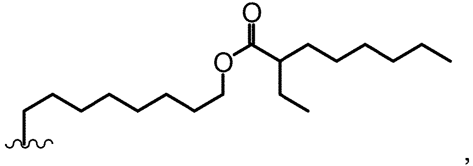 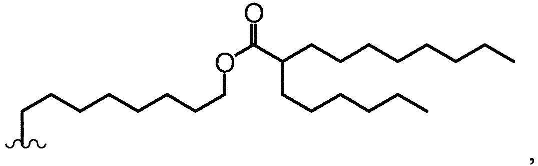 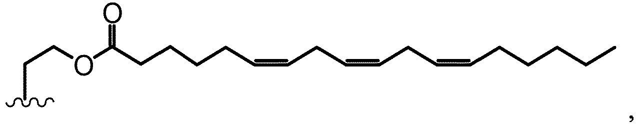 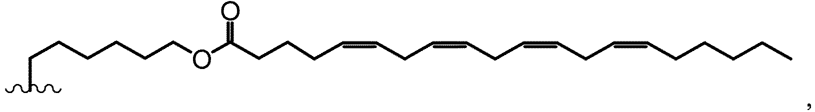 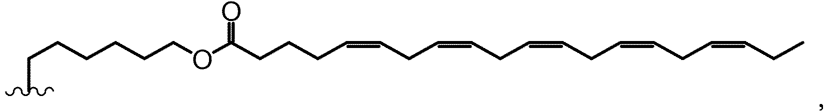 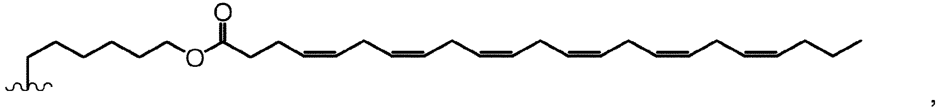 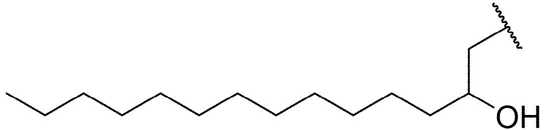 and

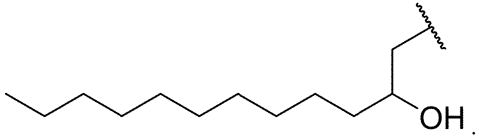

In some embodiments of the first aspect, n is 1;
L₃ and L₄ are independently -CH₂CH₂- or -CH₂CH₂CH₂-;
L₅ is -CH₂CH₂-, -CH₂CH₂CH₂-, -CH₂CH₂-C(O)O-CH₂CH₂CH₂-, -CH₂CH₂-C(O)N(H)-CH₂CH₂CH₂-, or -CH₂CH(OH)CH₂-, and
R₁, R₂, R₃, R₄, L₁, and L₂ are each independently one of following structures: 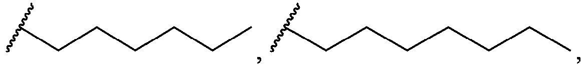 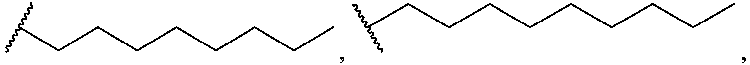 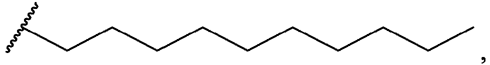 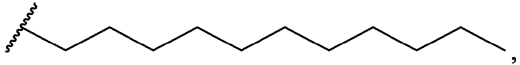 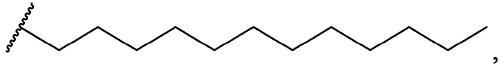 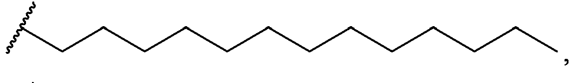 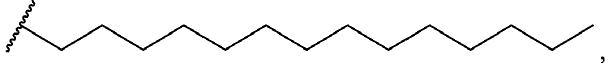 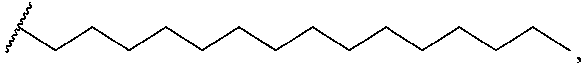 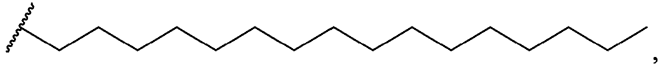 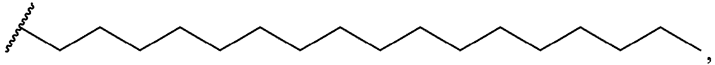 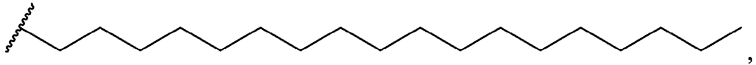 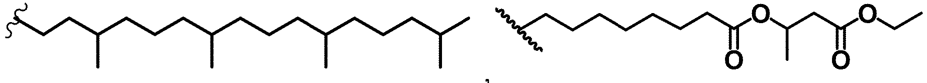 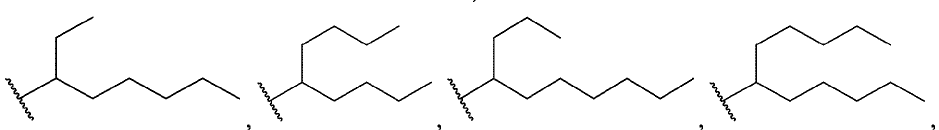 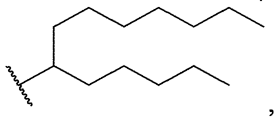 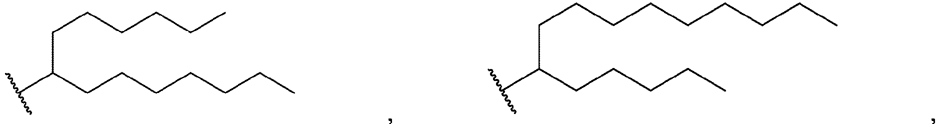 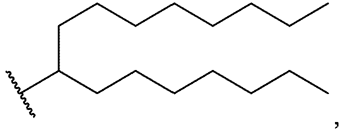 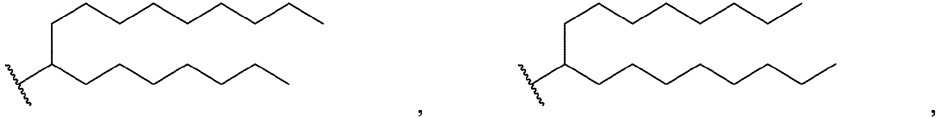 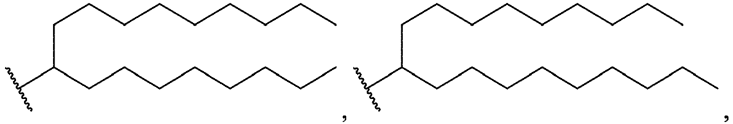 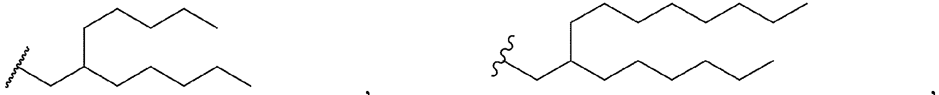 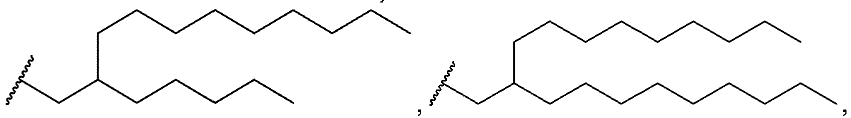 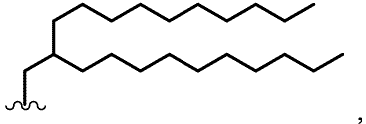 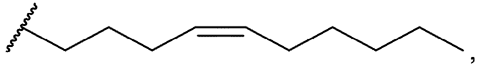 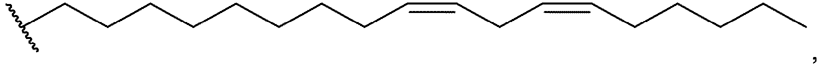 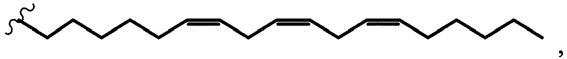 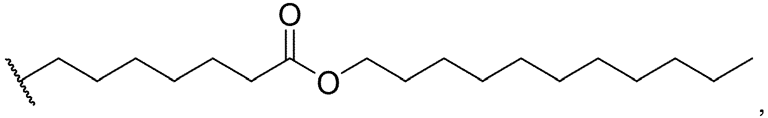 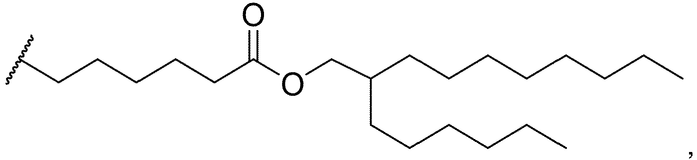 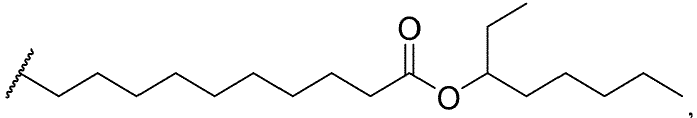 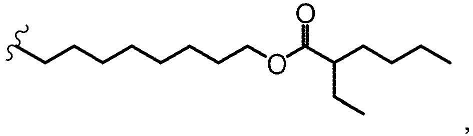 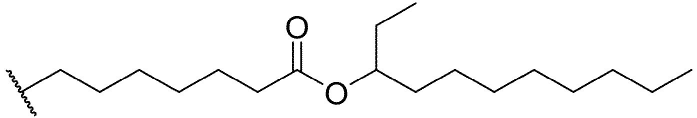 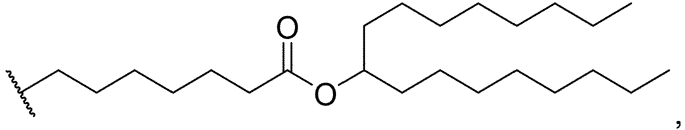 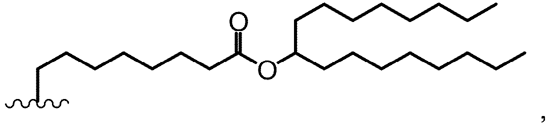 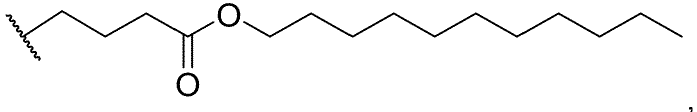 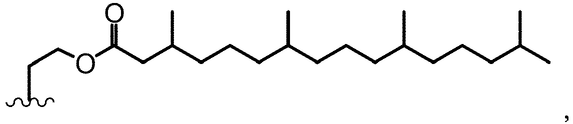 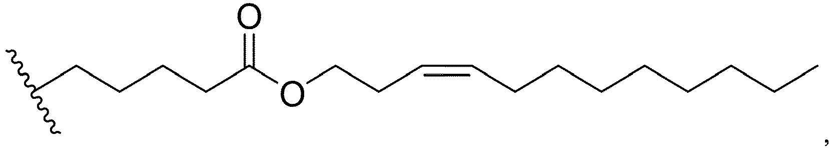 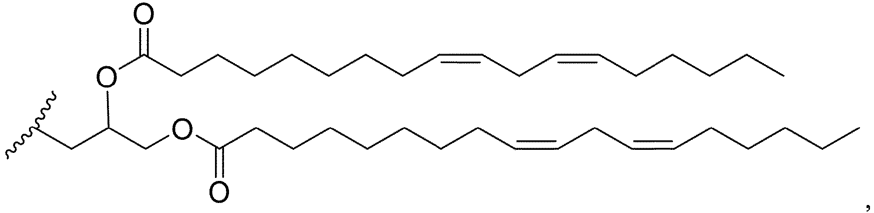 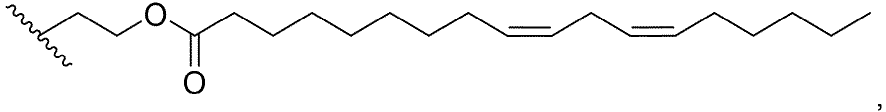 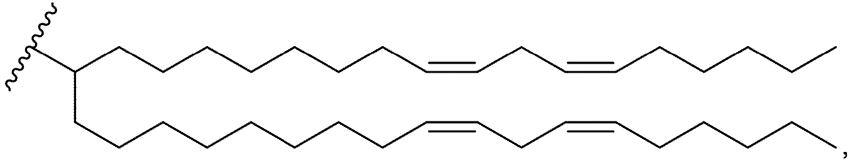 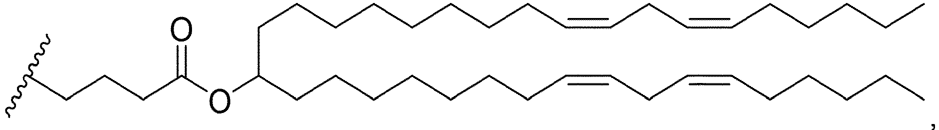 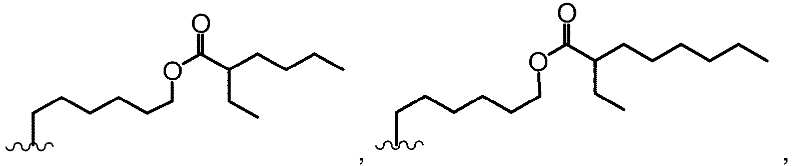 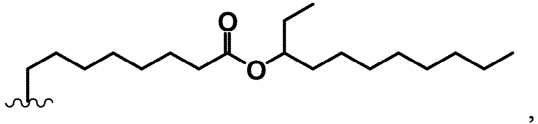 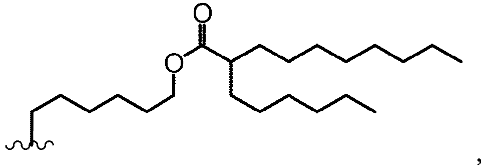 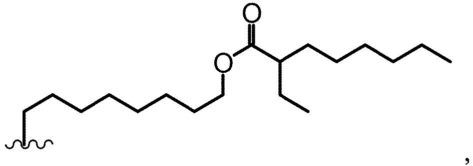 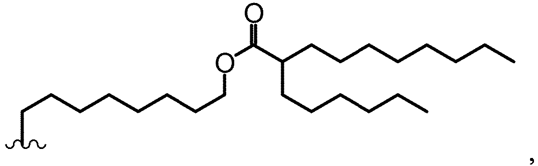 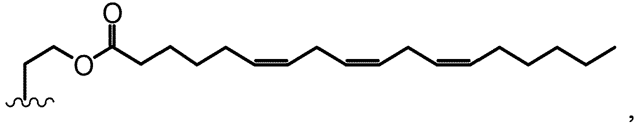 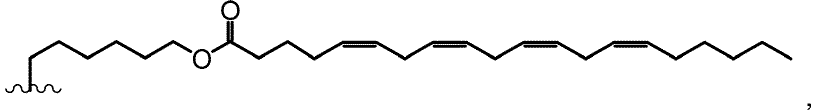 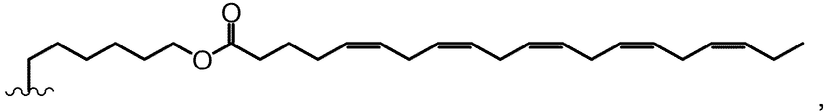 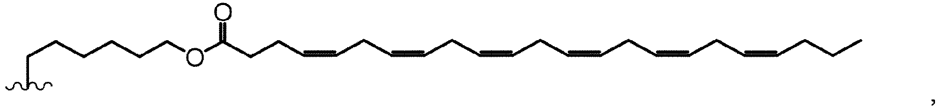 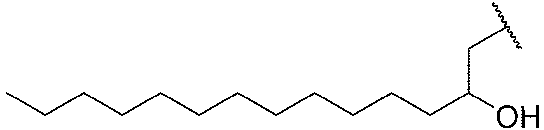 and 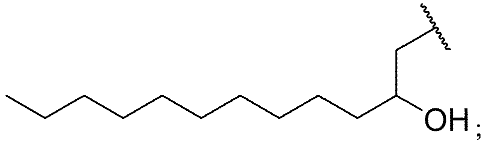
Or, R₃ and R₄ are both methyl, and R₁, R₂, L₁ and L₂ are defined by the above structures.

In some embodiments of the first aspect, L₁ and L₂ are each independently C₅-C₃₀ alkyl, C₅-C₂₄ alkenyl, or -R'-M-R"; wherein R', M, R" are defined by the above structures.

In some embodiments of the first aspect, R₁ and R₂ are each independently C₄-C₃₀ alkyl, C₂-C₂₄ alkenyl, or -R'-M-R"; or R₁ and R₂, together with their respective attached N and L₄, form a 5 to 8-membered diazacycloalkylene; wherein R', M, R" are defined by the above structures.

In some embodiments of the first aspect, R₃ and R₄ are each independently C₁-C₃₀ alkyl, C₂-C₂₄ alkenyl, or -R'-M-R"; or R₃ is H, C₁-C₃₀ alkyl, C₂-C₂₄ alkenyl, or -R'-M-R", and R₄ is C₁-C₃₀ alkyl, C₂-C₂₄ alkenyl, or -R'-M-R"; wherein R', M, R" are defined by the above structures.

In some embodiments of the first aspect, R' is C₁-C₁₂ alkylene or C₂-C₁₂ alkenylene.

In some embodiments of the first aspect, R", R^{a} and R^{b} are each independently selected from H, C₁-C₁₂ alkyl and C₂-C₁₂ alkenyl.

In some embodiments of the first aspect, the application provides a phosphatidylamine compound, or a stereoisomer, a prodrug, a pharmaceutically acceptable salt thereof, the phosphatidylamine compound is selected from one of the following compounds:

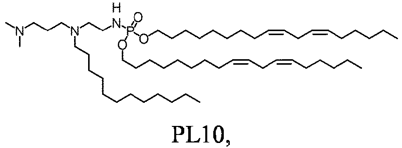

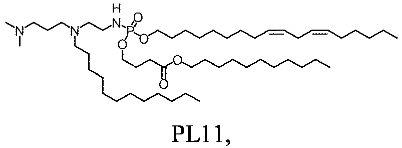

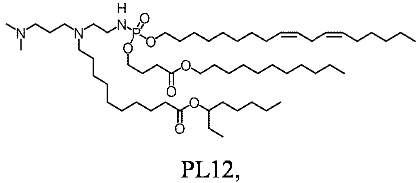

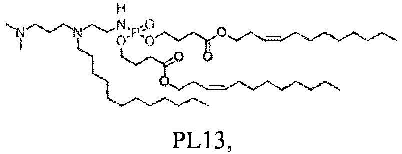

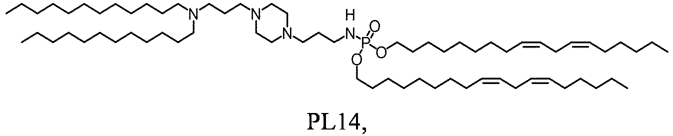

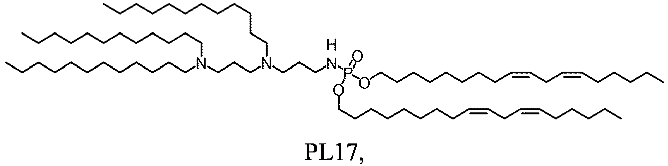

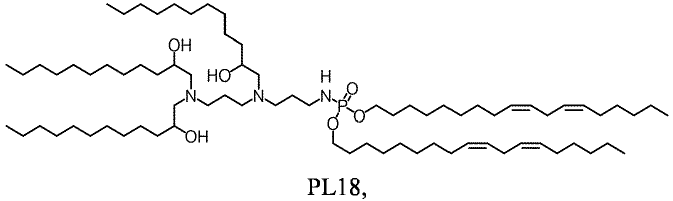

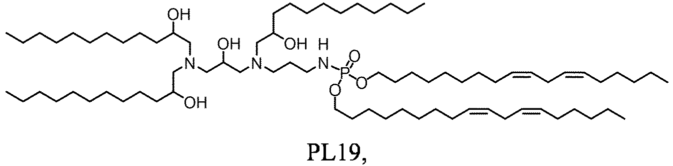

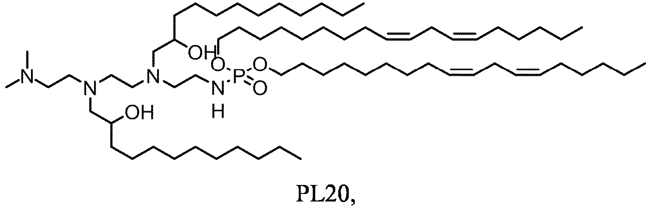

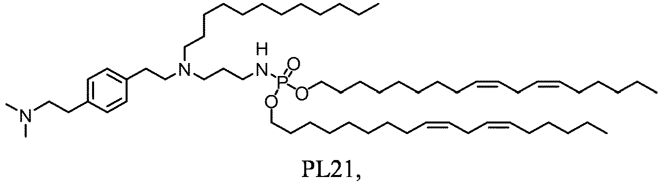

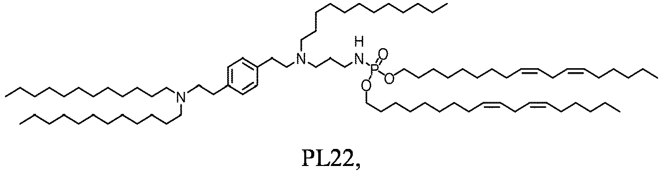

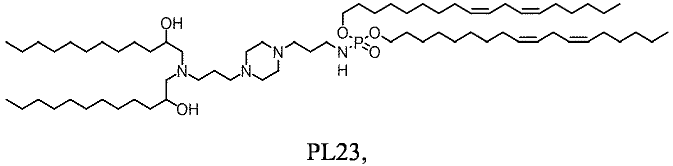

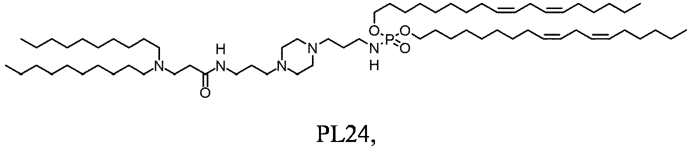

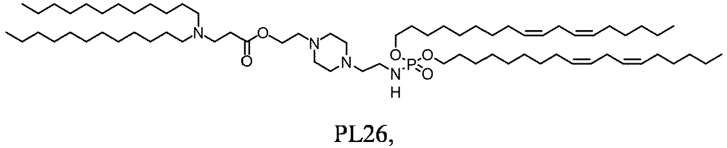

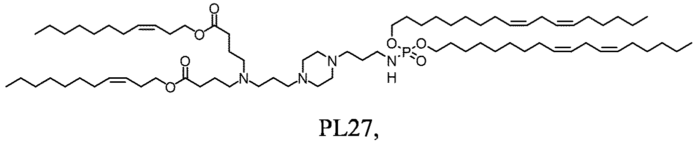

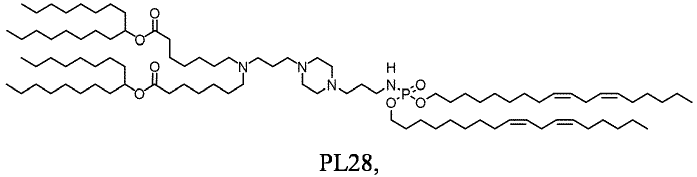

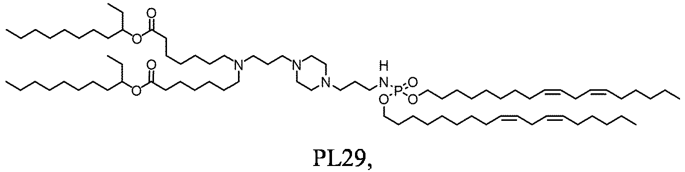

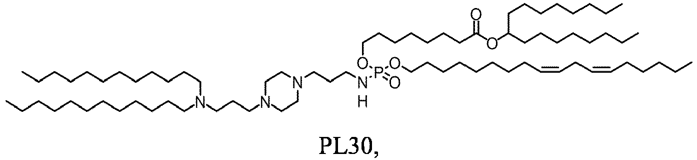

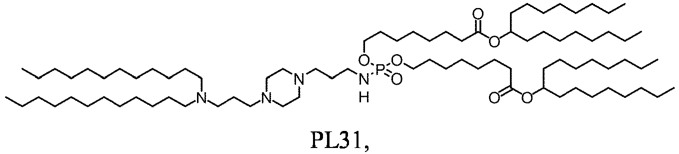

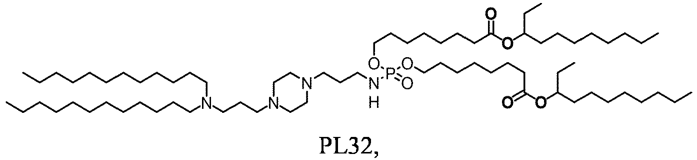

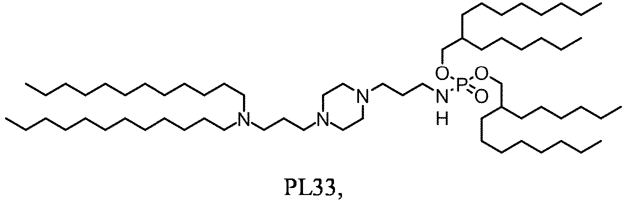

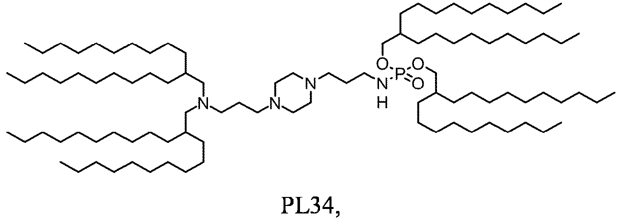

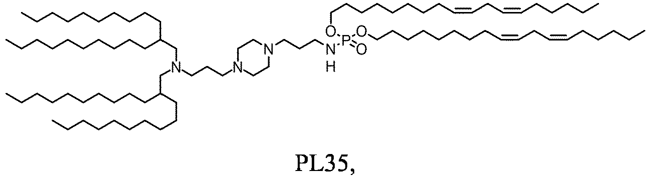

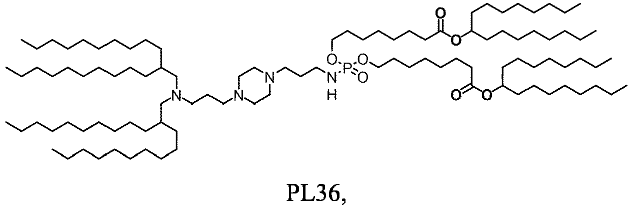

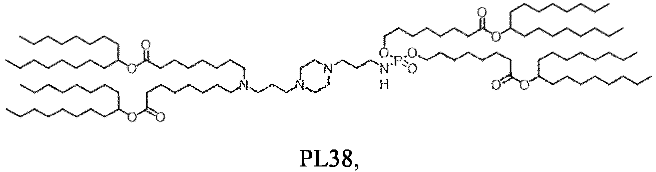

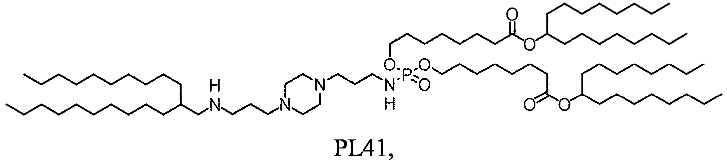

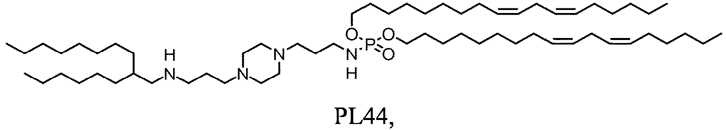

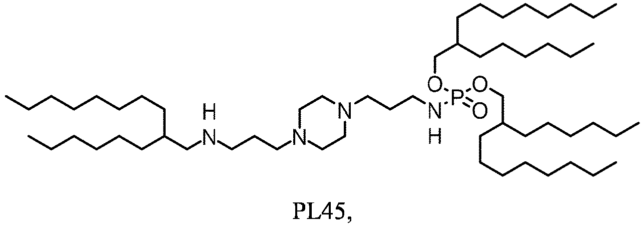

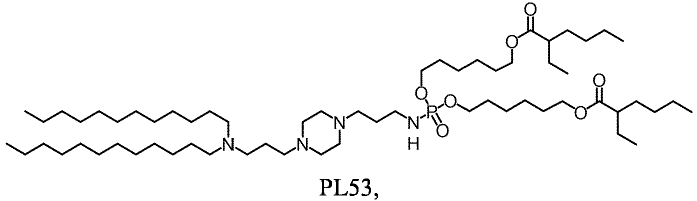

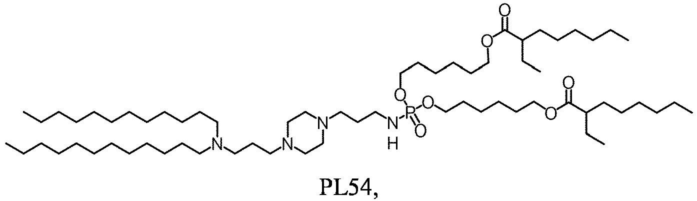

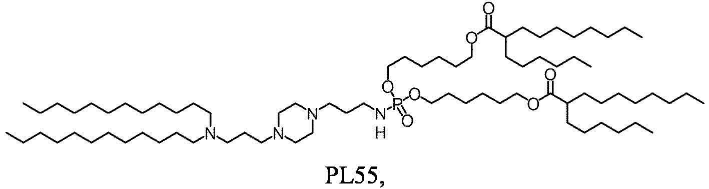

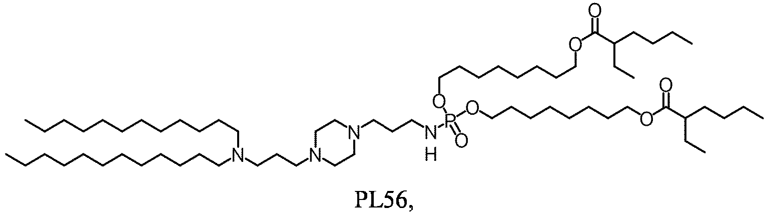

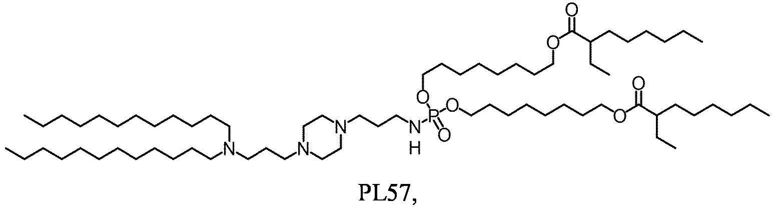

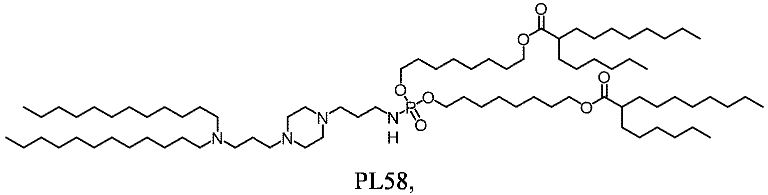

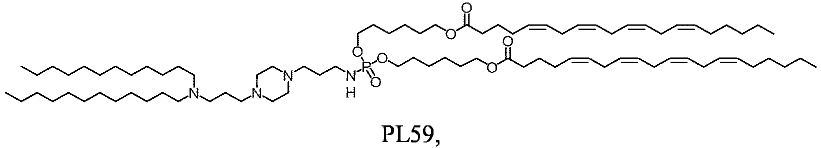

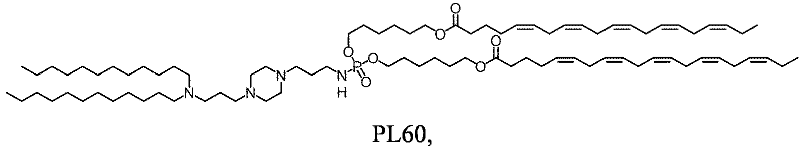

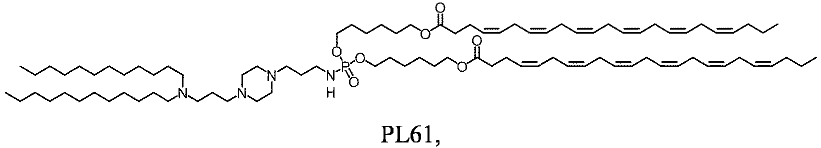

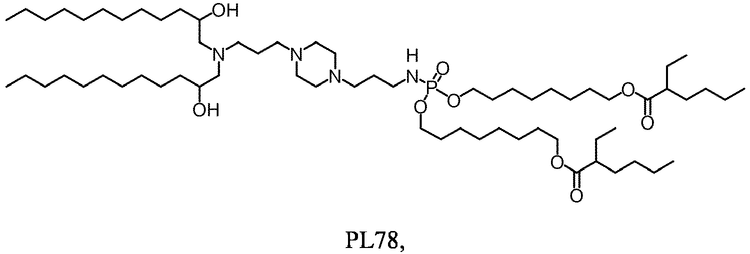

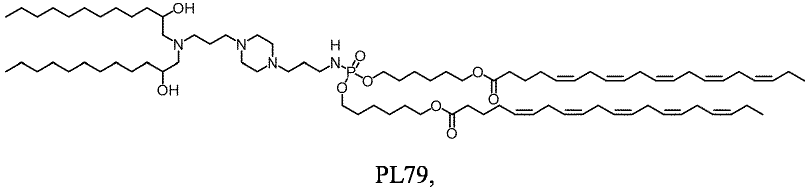

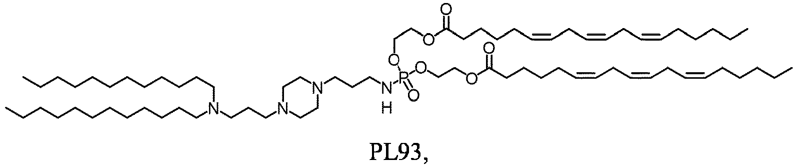

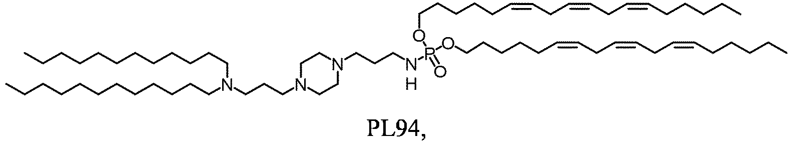

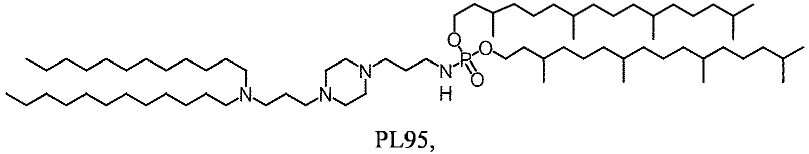

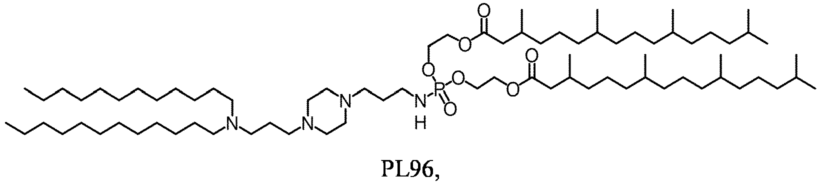

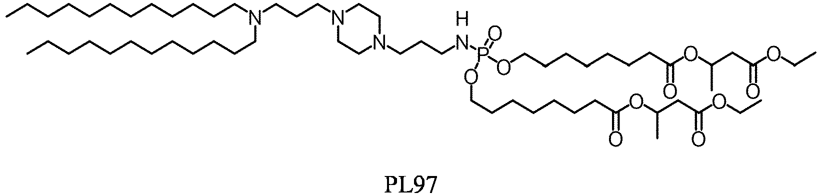

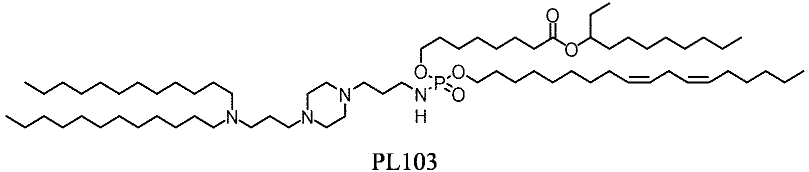

In the second aspect, this application provides a preparation method of the phosphatidylamine compound described above, the preparation method includes the following steps:
obtaining a compound represented by formula (I) by performing an amide reaction between a compound represented by formula (II) and a compound represented by formula (III): 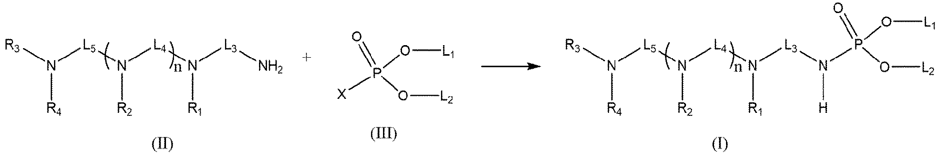
wherein X in formula (III) is a leaving group, such as chlorine, bromine, or iodine; the other groups in formula (II) and formula (III) are the same as the corresponding groups in formula (I).

In the third aspect, this application provides a lipid composition. The lipid composition includes a therapeutic agent or a prophylactic agent.

In some embodiments of the third aspect, the lipid composition further includes an additional lipid, the additional lipid being selected from one or more of a neutral lipid, a steroid, and a polymer-conjugated lipid.

In some embodiments of the third aspect, the neutral lipid is selected from one or more of DSPC, DPPC, DOPC, POPC, DOPE, DOPS, and SM, preferably, DOPE.

In some embodiments of the third aspect, a molar ratio of the neutral lipid to the phosphatidylamine compound is from 2:1 to 8:1.

In some embodiments of the third aspect, the steroid is cholesterol.

In some embodiments of the third aspect, a molar ratio of the steroid to the phosphatidylamine compound is from 5:1 to 1:1.

In some embodiments of the third aspect, the polymer-conjugated lipid is a PEGylated lipid selected from polyethylene glycol (PEG)-diacylglycerol (DAG), PEG-polyethylene (PE), PEG-sulfur ether bond (S)-diacylglycerol (DAG), and PEG-cer.

In some embodiments of the third aspect, the therapeutic agent or the prophylactic agent is selected from one or more of a nucleic acid drug, a gene vaccine, a small molecule drug, a polypeptide, and a protein drug.

In some embodiments of the third aspect, the lipid composition is lipid nanoparticles.

In the fourth aspect, this application provides a lipid nanoparticle including the phosphatidylamine compound described above or the lipid composition described above.

In the fifth aspect, this application provide a pharmaceutical composition, the pharmaceutical composition includes the phosphatidylamine compound described above, the lipid composition described above, and a pharmaceutically acceptable diluent or excipient.

In the sixth aspect, the lipid composition described above, the lipid nanoparticle described above, or the pharmaceutical composition described above are used for treating or preventing a disease in an individual in a corresponding need, or the lipid composition described above, the lipid nanoparticle described above, or the pharmaceutical composition described above is used in the preparation of a drug for treating or preventing a disease in an individual in a corresponding need, preferably, the disease includes, but is not limited to, fatty liver, obesity, tumors, arthritis, and viral infections.

In the seventh aspect, this application provides a use of a lipid composition described above, a lipid nanoparticle described above, or a pharmaceutical composition described above in a drug for vaccination against viral pathogens.

In the eighth aspect, this application provides a method for treating or preventing a disease in an individual in a corresponding need, the method includes administering the lipid composition described above, the lipid nanoparticle described above, or the pharmaceutical composition described above to the individual, the disease includes, but is not limited to, fatty liver, obesity, tumors, arthritis, or viral infections.

In the ninth aspect, this application provides a method for vaccinating an individual in a corresponding need against antiviral pathogens, the method includes administering the lipid composition described above, the lipid nanoparticle described above, or the pharmaceutical composition described above to the individual.

### SPECIFIC IMPLEMENTATIONS

In order to make the purpose, technical scheme and advantages of this application more clear, the implementation examples of this invention will be described in detail below. It should be noted that, without conflict, the embodiments in this application and the characteristics of the embodiments can be arbitrarily combined with each other.

### Abbreviations

DCM: dichloromethane
EtOH: ethanol
MeOH: methanol
DMF: N, N-Dimethylformamide
DCC: N, N'-dicyclohexylcarbodiimide
DMAP: 4-Dimethylaminopyridine
Et₃N: triethylamine
TFA: trifluoroacetic acid
(Boc)₂O: di-tert-butyl dicarbonate
Tris-EDTA: trimethylolpropane-ethylenediaminetetraacetic acid
DOPE: Dioleoyl phosphatidylethanolamine
DSPC Distearoyl phosphatidylcholine
DMG-PEG 2000: 1,2-dimyristoyl-rac-glycero-3-methoxypolyethylene glycol 2000
RT: room temperature
IPA: isopropyl alcohol
EDCI: 1-(3-dimethylaminopropyl)-3-ethylcarbodiimide
HOBt: 1-Hydroxybenzotriazole
THF: Tetrahydrofuran
PMBCl: 4-Methoxybenzyl chloride

### Experimental materials

Linolenol, 7-bromoheptanoic acid, didodecylamine, acrylic acid, 4-methoxybenzyl chloride, 2-(4-(2-aminoethyl)piperazin-1-yl)ethanol, cis-3-dodecan-1-ol, 5-bromopentanoic acid, 4-hydroxybutyric acid, 1-undecanol, phytanic acid, ethylene glycol, 3,7,11,15-tetramethylhexadecan-1-ol, γ-linolenyl alcohol, linolenic acid, 1,6-hexanediol, docosahexaenoic acid, eicosapentaenoic acid, arachidonic acid, 2-hexyl decanoic acid, 2-ethyl decanoic acid, 2-ethylhexanoic acid, ethyl 3-hydroxybutyrate, DDQ were purchased from Shanghai Bide Pharmatech Co., Ltd. 9-heptadecanol, 3-undecanol, 1,8-octanediol, 2-hexyl-1-decanol, 1-bromo-2-hexyldecane, NaH, DCC, DMAP, di-tert-butyl dicarbonate, DCC, DMAP, tert-butyl dichlorodicarbonate, ultra-dry dichloromethane, and deuterium substitute reagent were purchased from Beijing Mercury Technology Co. Ltd. Triethylamine, phosphorus oxychloride, 1,4-bis(3-aminopropyl)piperazine, and N, N-dimetgyl-1, 3-propanediamine were purchased from Beijing Innochem Technology Co. Ltd. 1-octadecanol was purchased from Alpha Aesar (China) Chemical Co. Ltd. Dialysis bags (regenerated cellulose membrane, a molecular weight cutoff: 10 kDa) and D-luciferin sodium salt were purchased from Shandong Consater Biotechnology Co. Ltd. RiboGreen reagent was purchased from invitrogen (USA). DMEM medium, penicillin-streptomycin double antibody, and 0.25% trypsin containing EDTA were purchased from Beijing Zhongke Maichen Technology Co., Ltd. Fetal bovine serum was purchased from PAN biotech (Germany). Cell culture dishes and plates were purchased from Corning Inc. (USA).

### Experimental apparatus

Electrospray Ionization Mass Spectrometer (Xevo G2 Q-TOF, Waters, USA), Nuclear Magnetic Resonance Spectrometer (AVANCE III HD400M, Bruker, Switzerland), Pure/Ultra Pure Water Integration System (Milli-Q Intergral 10, Merck Millipore, USA), Nano Particle Size and Zeta Potential Analyzer (Zetasizer Nano ZSP, MalVERN, UK), Enzyme Labeling Instrument (Synergy H1, Biotek, USA), Small Animal Live Imaging Instrument (Lumina Series III, PerkinElmer, USA), and Rotary Evaporator (Hei-VAP Value Digital, Heidolph, Germany).

### Example 1

### Synthesis of PL1

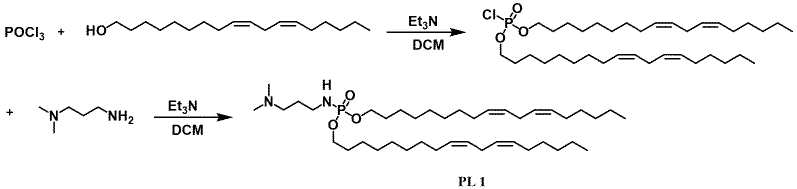

A solution was prepared by dissolving linolenol (533 mg, 2 mmol) in 5 mL of DCM and adding 2 mmol of triethylamine, and then the above solution was slowly added dropwise to phosphorus oxychloride (153 mg, 1 mmol) in an ice bath (5°C). The reaction system was protected with nitrogen and slowly warmed up to RT, followed by reaction for 4 h. After 4 h, a mixed solution of N, N-dimethyl-1,3-propanediamine (102 mg, 1 mmol) and triethylamine (1 eq) in dichloromethane was added dropwise to the system and reacted for 2 h at RM. When the reaction was complete, the crude product was obtained by washing with saturated sodium bicarbonate and concentrating under reduced pressure. A liquid compound PL1 (394 mg, 58%) was obtained by purification with column chromatography with eluting using a mixture of dichloromethane and methanol at a ratio of 15:1. ¹H NMR (400 MHz, CDCl₃): 5.41-5.29 (m, 8H), 4.01-3.91 (m, 4H), 2.96-2.89 (m, 2H), 2.78 (t, J=8Hz, 4H), 2.51-2.47 (m, 2H), 2.33 (s, 6H), 2.10-2.05 (m ,8H), 1.75-1.65 (m, 6H), 1.42-1.31 (m, 64H), 0.92 (t, J=8Hz, 6H). MS m/z (ESI): 679.59 [M+H]⁺.

### Example 2

### Synthesis of PL4

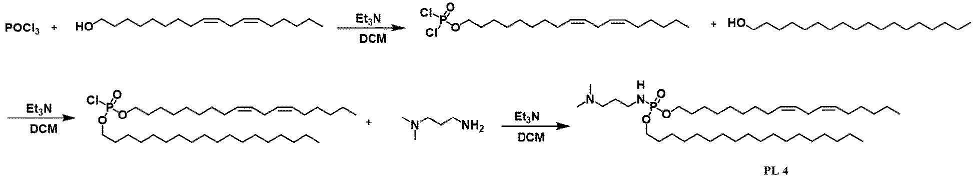

A solution was prepared by dissolving linolenol (266 mg, 1 mmol) in 5 mL of DCM and adding 1 mmol of triethylamine. Then the above solution was slowly added dropwise to phosphorus oxychloride (153 mg, 1 mmol) in an ice bath (0°C), the reaction system was protected with nitrogen, and reacted at 0°C-5°C for 2 h. Subsequently, a mixed solution of 1-octadecanol (270 mg, 1 mmol) and triethylamine (1 eq) in dichloromethane was added dropwise to the system and reacted from 5°C-RT for 2 h. Immediately thereafter, a mixed solution of N, N-dimethyl-1,3-propanediamine (102 mg, 1 mmol) and triethylamine (1 eq) in dichloromethane was added dropwise to the system and reacted at RT for 2 h. When the reaction was complete, the crude product was obtained by washing with saturated sodium bicarbonate and concentrating under reduced pressure. A compound PL4 (461 mg, 68%) was obtained by purification with column chromatography with eluting using a mixture of dichloromethane and methanol at a ratio of 15:1. ¹H NMR (400 MHz, CDCl₃): 5.40-5.31 (m, 4H), 4.01-3.95 (m, 4H), 3.54 (s, 1H), 3.03-2.96 (m, 2H), 2.79 (t, J=4Hz, 2H), 2.39 (t, J=4Hz, 2H), 2.24 (s, 6H), 2.09-2.04 (m ,4H), 1.70-1.64 (m, 6H), 1.39-1.27 (m, 46H), 0.92-0.88 (m, 9H). MS m/z (ESI): 683.62 [M+H]⁺.

### Example 3

### Synthesis of PL6

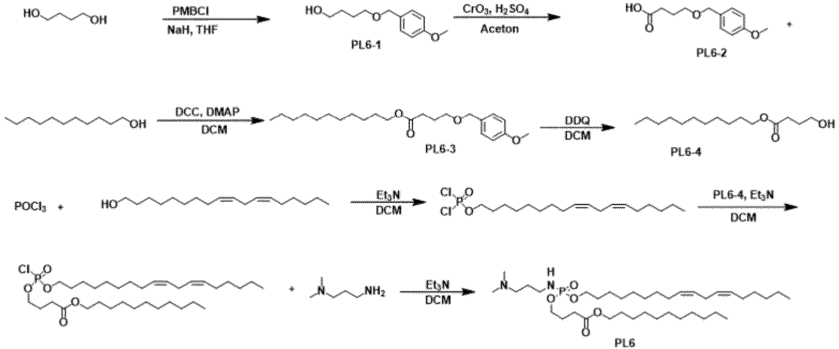

4-Hydroxybutyric acid (104 mg, 1 mmoL) and 1-undecanol (516 mg, 3 mmoL) were added to DMF solution, and DCC (618 mg, 3 mmoL) and DMAP (122 mg, 1 mmoL) were added to react at room temperature for 24 h, after the reaction, water was added and extracted with DCM, washed with saturated NaHCO₃, concentrated under reduced pressure, purified by column chromatography, and eluted with ethyl acetate : petroleum ether = 20 : 1 to obtain compound PL6-1 (178.1 mg, 69 %). ¹H NMR (400 MHz, CDCl₃) : 4.07 (t, J = 8Hz, 2H), 3.68 (t, J = 8Hz, 2H), 2.43 (t, J = 8Hz, 2H), 1.91-1.85 (m, 2H), 1.65-1.58 (m, 2H), 1.35-1.26 (m, 16H), 0.88 (t, J = 8Hz, 3H).

Linoleyl alcohol (266 mg, 1 mmoL) was dissolved in 5 mL DCM, and 1 mmol of triethylamine was added to prepare a solution, then the above solution was slowly added to phosphorus oxychloride (153 mg, 1 mmoL) in an ice bath (0 °C), with nitrogen protection, and reacted at 0 °C-5 °C for 2 h, subsequently, the mixed solution of PL6-1 (258 mg, 1 mmoL) and triethylamine (1 eq) was added to the system, and reacted at 5 °C-RT for 2 h, subsequently, a mixed solution of N, N-dimethyl-1,3-propanediamine (102 mg, 1 mmoL) and triethylamine (leq) in dichloromethane was added dropwise to the system, and the reaction was carried out at room temperature for 2 h. After the reaction is complete, it is washed with saturated sodium bicarbonate and concentrated under reduced pressure to obtain a crude product. Compound PL6 (495.8 mg, 74 %) was obtained by purification by column chromatography with eluting using a mixture of dichloromethane and methanol at a ratio of 15:1. ^{¡}H NMR (400 MHz, CDCl₃) : 5.41-5.31 (m, 4H), 4.09-3.85 (m, 4H), 3.48 (s, 1H), 3.05-3.02 (m, 2H), 2.78 (t, J = 4Hz, 2H), 2.76-2.71 (m, 2H), 2.48-2.42 (m, 10H), 2.09-1.96 (m, 6H), 1.83-1.79 (m, 2H). 1.68-1.60 (m, 4H), 1.39-1.28 (m, 32H), 0.92-0.88 (m, 6H). MS m/z (ESI):671.55 [M+H]⁺

### Example 4

### Synthesis of PL8

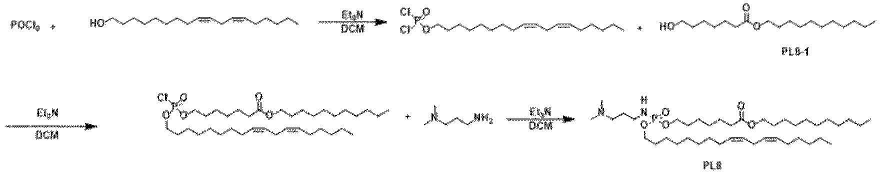

7-Hydroxyheptanoic acid (146 mg, 1 mmoL) and 1-undecanol (516 mg, 3 mmoL) were added to DMF solution, and DCC (618 mg, 3 mmoL) and DMAP (122 mg, 1 mmoL) were added respectively to react at room temperature for 24 h. After the reaction, water was added and extracted with DCM, washed with saturated NaHCO₃, concentrated under reduced pressure, purified by column chromatography, and eluted with ethyl acetate:petroleum ether = 20 : 1 to obtain compound PL8-1 (213 mg, 71 %). ¹H NMR (400 MHz, CDCl₃): 4.06 (t, J=8Hz, 2H), 3.64 (t, J=8Hz, 2H), 2.31 (t, J=8Hz, 2H),1.68-1.54 (m, 6H), 1.43-1.27 (m, 20H), 0.89 (t, J=8Hz, 3H).

A solution was prepared by dissolving linolenol (266 mg, 1 mmol) in 5 mL of DCM and adding 1 mmol of triethylamine, and then the above solution was slowly added dropwise to phosphorous oxychloride (153 mg, 1 mmol) in an ice bath (0 °C). The reaction system was protected with nitrogen and reacted at 0°C-5°C for 2 h. Subsequently, a mixed solution of PL8-1 (300 mg, 1 mmol) and triethylamine (1 eq) in dichloromethane was added dropwise to the system and reacted at 5°C-RT for 2 h. Immediately thereafter, a mixed solution of N, N-dimethyl-1,3-propanediamine (102 mg, 1 mmol) and triethylamine (1 eq) in dichloromethane was added dropwise to the system and reacted for 2 h at room temperature. When the reaction was complete, the crude product was obtained by washing with saturated sodium bicarbonate and concentrating under reduced pressure. A liquid compound PL8 (555.3 mg, 78%) was obtained by purification by column chromatography with eluting using a mixture of dichloromethane and methanol at a ratio of 15:1. ¹H NMR (400 MHz, MeOD): 5.42-5.31 (m, 4H), 4.10-3.97 (m, 6H), 2.99-2.93 (m, 2H), 2.80 (t, J=4Hz, 2H), 2.65-2.61 (m, 2H), 2.45 (s, 6H), 2.36-2.32 (m, 2H), 2.11-2.06 (m ,4H), 1.80-1.61 (m, 10H), 1.68-1.60 (m, 4H), 1.39-1.28 (m, 32H), 0.94-0.90 (m, 6H). MS m/z (ESI): 713.59 [M+H]⁺.

### Example 5

### Synthesis of PL10

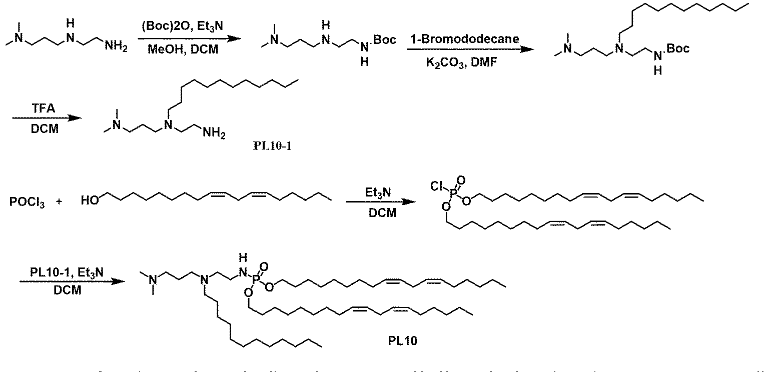

{3-[(2-aminoethyl)amino]propyl}dimethylamine (145 mg, 1 mmol) and (Boc)₂O (327 mg, 1.5 mmol) were added to a solvent mixture of DCM/MeOH, then triethylamine (1 eq) was added, and the reaction was carried out at room temperature for 3 h. After the reaction was complete, the solvent was removed by concentrating under reduced pressure, and a product was obtained by purifying by column chromatography. The obtained product was dissolved in DMF, and 1-bromododecane (498 mg, 2 mmol) and K₂CO₃ (276 mg, 2 mmol) were added and reacted at room temperature overnight. After the reaction was complete, a Boc-protected PL10-1 was obtained by adding water and extracting DCM, washing with saturated NaHCO₃, concentrating under reduced pressure, and purifying by column chromatography. Subsequent deprotection of Boc was performed with a DCM solution of TFA to obtain PL10-1. ¹H NMR (400 MHz, CDCl₃): 2.65 (t, *J*=8Hz, 2H), 2.40-2.30 (m, 6H), 2.22-2.15 (m, 2H), 2.14 (s, 6H), 1.55-1.51(m, 2H), 1.36-1.31(m, 2H), 1.25-1.19 (m, 20H), 0.81(t, *J*=8Hz, 3H). MS m/z (ESI): 314.3[M+H]⁺.

A solution was prepared by dissolving linolenol (533 mg, 2 mmol) in 5 mL of DCM and adding 2 mmol of triethylamine, and then the above solution was slowly added dropwise to phosphorus oxychloride (153 mg, 1 mmol) in an ice bath (5°C). The reaction system was protected with nitrogen and slowly warmed up to RT for reaction for 4 h. After 4 h, a mixed solution of the compound PL10-1 (314 mg, 1 mmol) and triethylamine (1 eq) in dichloromethane was added dropwise to the system and reacted for 2 h at room temperature. When the reaction was complete, the crude product was obtained by washing with saturated sodium bicarbonate and concentrating under reduced pressure. A liquid compound PL10 (729.8 mg, 82%) was obtained by purification by column chromatography with eluting using a mixture of dichloromethane and methanol at a ratio of 10:1. ¹H NMR (400 MHz, CDCl₃): 5.41-5.32 (m, 8H), 4.02-3.85 (m, 4H), 3.60 (s, 1H), 2.97-2.91 (m, 2H), 2.79 (t, J=4Hz, 4H), 2.54-2.39 (m, 8H), 2.34 (s, 6H), 2.09-2.04 (m ,8H), 1.71-1.59 (m, 6H), 1.46-1.24 (m, 52H), 0.92-0.88 (m, 9H). MS m/z (ESI): 890.82 [M+H]⁺.

### Example 6

### Synthesis of PL11

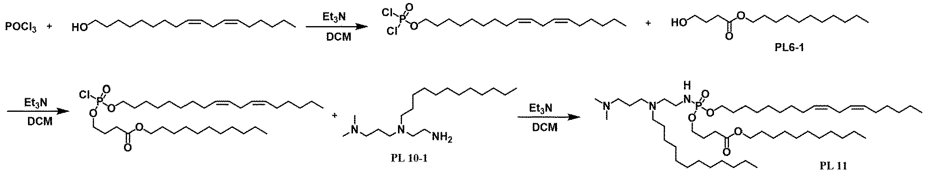

A solution was prepared by dissolving linolenol (266 mg, 1 mmol) in 5 mL of DCM and adding 1 mmol of triethylamine, and then the above solution was slowly added dropwise to phosphorus oxychloride (153 mg, 1 mmol) in an ice bath (0°C). The reaction system was protected with nitrogen and reacted at 0°C-5°C for 2 h. Subsequently, a mixed solution of PL6-1 (258 mg, 1 mmol) and triethylamine (1 eq) in dichloromethane was added dropwise to the system and reacted at 5°C-RT for 2 h. Immediately thereafter, a mixed solution of PL10-1 (313 mg, 1 mmol) and triethylamine (1 eq) in dichloromethane was added dropwise to the system and reacted for 2 h at room temperature. When the reaction was complete, the crude product was obtained by washing with saturated sodium bicarbonate and concentrating under reduced pressure. A liquid compound PL11 (450.3 mg, 52%) was obtained by purification by column chromatography with eluting using a mixture of dichloromethane and methanol at a ratio of 10:1. ¹H NMR (400 MHz, CDCl₃): 5.42-5.31 (m, 4H), 4.09-3.84 (m, 6H), 3.65 (s, 1H), 2.98-2.91 (m, 2H), 2.78 (t, *J*=4Hz, 4H), 2.55-2.40 (m, 10H), 2.36 (s, 6H), 2.09-1.99 (m ,6H), 1.72-1.59 (m, 6H), 1.44-1.28 (m, 54H), 0.92-0.88 (m, 9H). MS m/z (ESI): 882.78 [M+H]⁺.

### Example 7

### Synthesis of PL12

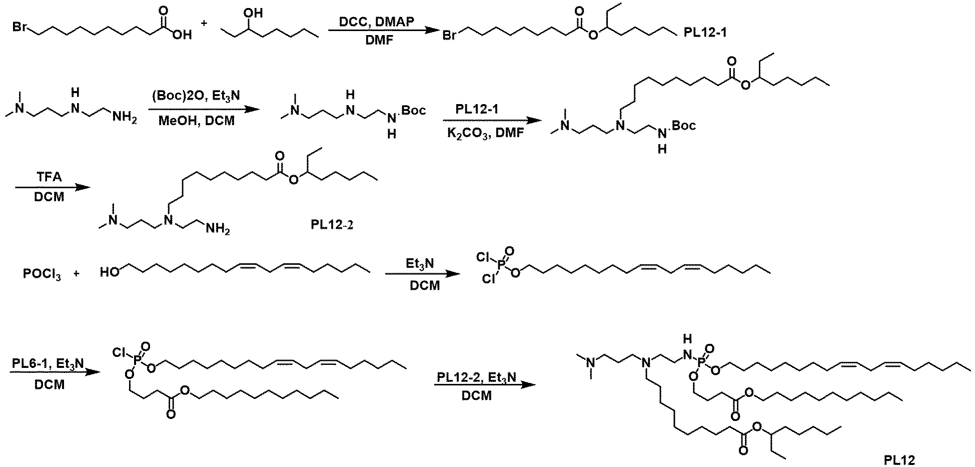

3-octanol (130 mg, 1 mmol) was added to DMF with 10-bromodecanoic acid (250 mg,1 mmol), and DCC (618 mg, 3 mmol) and then DMAP (122 mg, 1 mmol) were added for reaction at room temperature overnight. After the reaction is complete, PL12-1 was obtained by adding water, extracting with DCM, washing with saturated NaHCO₃, and purifying by column chromatography. Then {3-[(2-aminoethyl)amino]propyl}dimethylamine (145 mg, 1 mmol) and (Boc)₂O (327 mg, 1.5 mmol) were added to a solvent mixture of DCM/MeOH, triethylamine (1 eq) was then added, and the reaction was carried out at room temperature for 3 h. After the reaction was complete, a product was obtained by concentrating under reduced pressure and purifying by column chromatography. Then the obtained product was dissolved in DMF, and PL12-1 (698 mg, 2 mmol) and K₂CO₃ (276 mg, 2 mmol) were added and reacted at room temperature overnight. After the reaction is complete, a Boc-protected PL12-2 was obtained by adding water and extracting with DCM, washing with saturated NaHCO₃, concentrating under reduced pressure, and purifying by column chromatography. Subsequent deprotection of Boc was performed with a DCM solution of TFA to obtain PL12-2. ¹H NMR (400 MHz, CDCl₃) 4.83-4.80 (m, 1H), 2.71 (t, J=8Hz, 2H), 2.47-2.36 (m, 6H), 2.30-2.21 (m, 4H), 2.20 (s, 6H), 1.63-1.48(m, 8H), 1.39(t, J=8Hz, 2H).1.33-1.25(m, 16H), 0.9-0.85 (m, 6H). MS m/z (ESI): 428.4[M+H]⁺.

A solution was prepared by dissolving linolenol (266 mg, 1 mmol) in 5 mL of DCM and adding 1 mmol of triethylamine, and then the above solution was slowly added dropwise to phosphorus oxychloride (153 mg, 1 mmol) in an ice bath (0°C). The reaction system was protected with nitrogen and reacted at 0°C-5°C for 2 h. Subsequently, a mixed solution of PL6-1 (258 mg, 1 mmol) and triethylamine (1 eq) in dichloromethane was added dropwise to the system and reacted at 5°C-RT for 2 h. Immediately thereafter, a mixed solution of PL12-2 (427 mg, 1 mmol) and triethylamine (1 eq) in dichloromethane was added dropwise to the system and reacted for 2 h at room temperature. When the reaction was complete, the crude product was obtained by washing with saturated sodium bicarbonate and concentrating under reduced pressure. A liquid compound PL12 (607.6 mg, 61%) was obtained by purification by column chromatography with eluting using a mixture of dichloromethane and methanol at a ratio of 10:1. ¹H NMR (400 MHz, CDCl₃): 5.39-5.29 (m, 4H), 4.83-4.77 (m, 1H), 4.06-3.81 (m, 6H), 3.73 (s, 1H), 3.12-2.95 (m, 4H), 2.91-2.21 (m, 18H), 2.07-1.90 (m ,6H), 1.71-1.25 (m, 60H), 1.44-1.28 (m, 54H), 0.88-0.85 (m, 12H). MS m/z (ESI): 996.85 [M+H]⁺.

### Example 8

### Synthesis of PL13

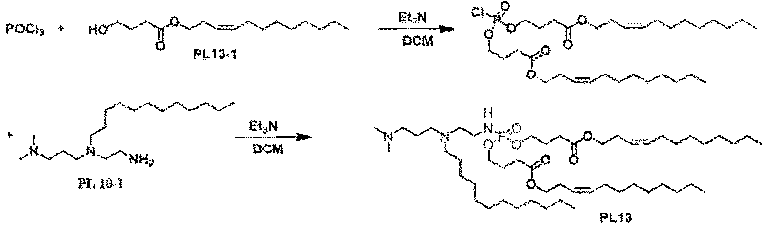

5-Hydroxyvaleric acid (118 mg, 1 mmoL) and cis-3-dodec-1-ol (552 mg, 3 mmoL) were added to DMF solution, and DCC (618 mg, 3 mmoL) and DMAP (122 mg, 1 mmoL) were added respectively to react at room temperature for 24 h. After the reaction, water was added and extracted with DCM, washed with saturated NaHCO₃, concentrated under reduced pressure, purified by column chromatography, and eluted with ethyl acetate : petroleum ether = 15 : 1 to obtain compound PL13-1. PL13-1 (540 mg, 2 mmol) was dissolved in 5 mL of DCM and adding 2 mmol of triethylamine, and then the above solution was slowly added dropwise to phosphorus oxychloride (153 mg, 1 mmol) in an ice bath (0 °C). The reaction system was protected with nitrogen and reacted at 5°C to 20°C for 2 h. A mixed solution of PL10-1 (313 mg, 1 mmol) and triethylamine (1 eq) in dichloromethane was added dropwise to the system and reacted for 2 h at room temperature. When the reaction was complete, the crude product was obtained by washing with saturated sodium bicarbonate and concentrating under reduced pressure. A liquid compound PL13 (430.1 mg, 48%) was obtained by purification by column chromatography with eluting using a mixture of dichloromethane and methanol at a ratio of 15:1. MS m/z (ESI): 463.88 [M+2H]2⁺.

### Example 9

### Synthesis of PL14

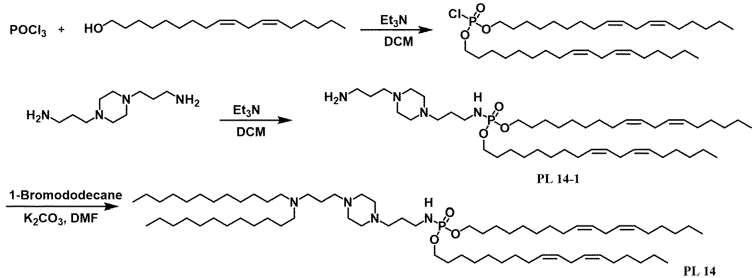

A solution was prepared by dissolving linolenol (533 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and reacted at 5°C-20°C for 2 h. Subsequently, the reaction system was added to a mixed solution of 1,4-bis(3-aminopropyl)piperazine (200 mg, 1 mmol) and triethylamine (1 eq) in dichloromethane, and reacted for 2 h at room temperature. When the reaction was complete, the crude product was obtained by washing with saturated sodium bicarbonate and concentrating under reduced pressure. A liquid compound PL14-1 was obtained by purification by column chromatography with eluting using a mixture of dichloromethane and methanol and ammonia at a ratio of 50:5:1. PL14-1 (776.67 mg,1 mmol) was dissolved in DMF, and 1-bromododecane (747 mg, 3 mmol) and K₂CO₃ (414 mg, 3 mmol) were added separately and reacted at room temperature overnight. When the reaction was complete, the crude product was obtained by quenching the reaction by adding water and extracting with dichloromethane, washing three times with water, and concentrating under reduced pressure. A liquid compound PL14 (767.97 mg, 69%) was obtained by purification by column chromatography with eluting using a mixture of dichloromethane and methanol at a ratio of 20:1. **¹H NMR** (400 MHz, CDCl₃): 5.42-5.29 (m, 8H), 4.02-3.90 (m, 4H), 3.60-3.54 (m, 1H), 3.02-2.95 (m, 4H), 2.77 (t, J=8Hz, 8H), 1.69-1.63 (m, 8H), 1.45-1.26 (m, 72H), 0.91-0.86 (m,12H) . MS m/z (ESI): 557.52[M+2H]²⁺.

### Example 10

### Synthesis of PL17

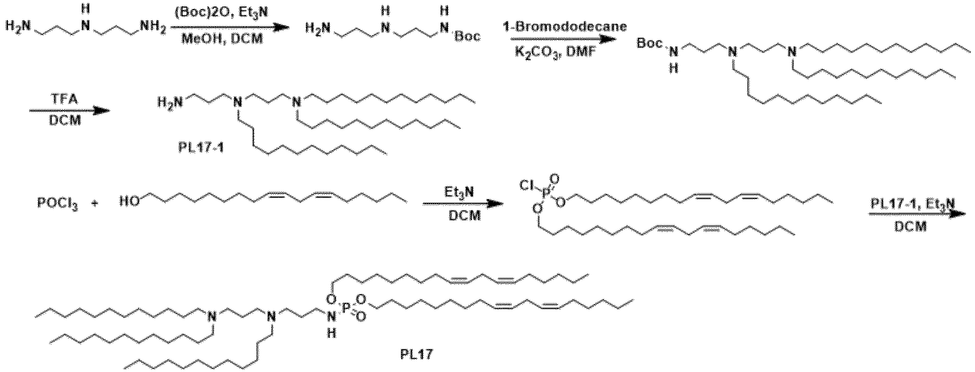

A solution was prepared by dissolving linolenol (533 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and reacted at 5°C-20°C for 2 h. Subsequently, a mixed solution of PL17-1 (635.7 mg, 1 mmol) and triethylamine (1 eq) in dichloromethane was added dropwise to the system and reacted for 2 h at room temperature. When the reaction was complete, the crude product was obtained by washing with saturated NaHCO₃ and concentrating under reduced pressure. A liquid compound PL17 (896.8mg,74%) was obtained by purification by column chromatography with eluting using a mixture of dichloromethane and methanol at a ratio of 15:1. ¹H NMR (400 MHz, CDCl₃): 5.43-5.30(m, 8H), 4.01-3.89 (m, 4H), 3.76 (s, 1H), 3.60-3.54 (m, 1H), 3.25-3.22 (m, 2H), 3.05-2.92 (m, 8H), 2.78 (t, J=8Hz,4H), 2.69-2.55 (m, 4H), 2.09-2.04 (m, 8H), 1.67-1.63 (m, 8H), 1.52-1.27 (m,92H), 0.92-0.88 (m, 15H). MS m/z (ESI):
607.08 [M+2H]²⁺.

### Example 11

### Synthesis of PL18

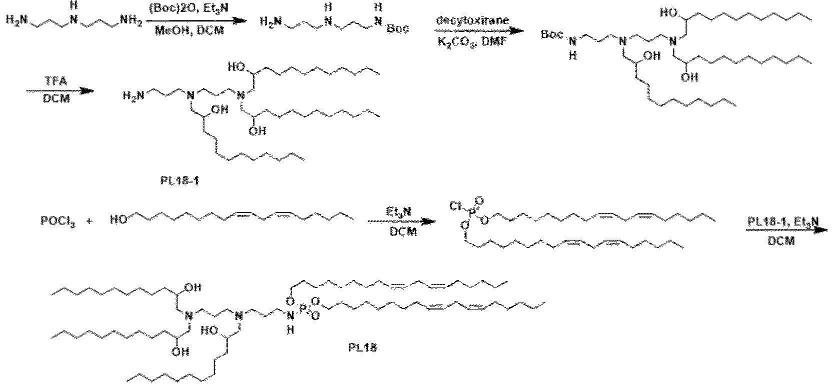

A solution was prepared by dissolving linolenol (533 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and reacted at 5°C-20°C for 2 h. Subsequently, a mixed solution of PL18-1 (684 mg, 1 mmol) and DCM (1 eq) in dichloromethane was added dropwise to the system and reacted for 2 h at room temperature. When the reaction was complete, the crude product was obtained by washing with saturated NaHCO₃ and concentrating under reduced pressure. A liquid compound PL18 (1083.6 mg, 86%) was obtained by purification by column chromatography with eluting using a mixture of dichloromethane and methanol at a ratio of 20:1. ¹H NMR (400 MHz, CDCl₃): 5.41-5.29 (m, 8H), 4.14-4.09 (m, 3H), 3.99-3.90 (m, 4H), 3.71 (s, 3H), 3.06-2.30 (m, 18H),2.07-2.02 (m ,8H),1.88-1.62 (m, 8H), 1.44-1.24 (m, 86H), 0.90-0.86 (m,15H). MS m/z (ESI): 631.08[M+2H]²⁺.

### Example 12

### Synthesis of PL23

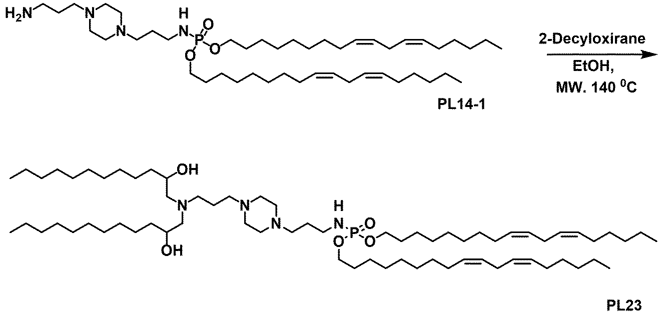

PL14-1 (776.67 mg, 1 mmol) was dissolved in EtOH, then 1,2-epoxydodecane (737.2 mg, 4 mmol) was added, and a reaction was performed in microwave at 140°C for 30 min. When the reaction was complete, the crude product was obtained by concentrating under reduced pressure. A liquid compound PL23 (732.8 mg, 64%) was obtained by purification by column chromatography with eluting using a mixture of dichloromethane and methanol at a ratio of 20:1. ¹H NMR (400 MHz, CDCl₃): 5.41-5.31 (m, 8H), 4.02-3.93 (m, 4H), 3.71-3.6 (m, 2H), 3.02-2.98 (m, 4H), 2.81-2.32 (m, 20H), 2.09-2.04 (m, 8H), 1.71-1.64 (m, 8H), 1.51-1.29 (m, 68H), 0.93-0.88 (m, 12H). MS m/z (ESI): 573.52[M+2H]²⁺.

### Example 13

### Synthesis of PL24

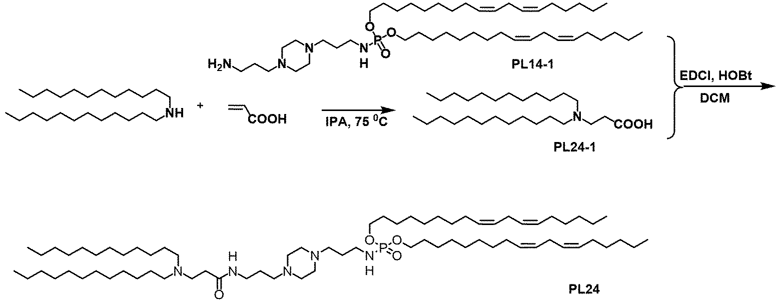

Didodecylamine (707 mg, 2 mmol) and acrylic acid (288 mg, 4 mmol) were first dissolved in 10 mL of isopropanol and reacted at 75°C overnight. After the reaction is complete, a product PL24-1 (740 mg, 87%) was obtained by concentrating under reduced pressure and purifying by column chromatography with eluting using a mixture of dichloromethane and methanol at a ratio of 30:1. 1H NMR (400 MHz, CDCl₃): 3.11 (t, *J*=8Hz, 2H), 2.88-2.84 (m, 4H), 2.63 (t, *J*=8Hz, 2H), 1.68-1.64 (m, 4H), 1.32-1.26 (m, 36H), 0.88 (t, *J*=8Hz, 6H). MS m/z (ESI): 424.42 [M-H]⁻.

PL14-1 (776.67 mg, 1 mmol) was dissolved in DCM, and PL24-1 (425.42 mg, 1 mmol), EDCI (191.7 mg, 1 mmol), and HOBt (135.1 mg, 1 mmol) were added and reacted overnight at room temperature. When the reaction was finished, the crude product was obtained by adding saturated NaCl solution and extracting with DCM, drying with anhydrous Na₂SO₄, and concentrating under reduced pressure. A liquid compound PL24 (663 mg, 56%) was obtained by purification by column chromatography with gradient eluting using a mixture of dichloromethane and methanol at a ratio of 30:1 to 15:1. ¹H NMR (400 MHz, CDCl₃): 5.44-5.31 (m, 8H), 4.01-3.93 (m, 4H), 3.32-2.28 (m, 2H), 3.02-2.81 (m, 4H), 2.09-2.04 (m, 8H), 1.74-1.58 (m, 8H), 1.38-1.27 (m, 72H), 0.93-0.88 (m, 12H).

### Example 14

### Synthesis of PL26

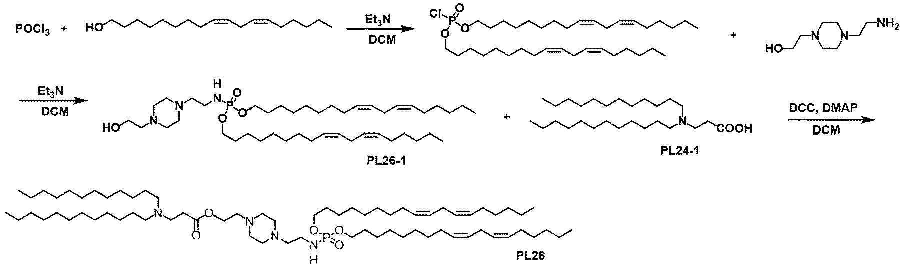

A solution was prepared by dissolving linolenol (533 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and reacted at 5°C-20 °C for 2 h. Subsequently, a mixed solution of 2-(4-(2-aminoethyl)piperazin-1-yl)ethanol (173 mg,1 mmol) and triethylamine (1 eq) in dichloromethane was added dropwise to the system and reacted for 2 h at room temperature. When the reaction was complete, the crude product was obtained by washing with saturated NaCl and concentrating under reduced pressure. A liquid compound PL26-1 was obtained by purification by column chromatography with eluting using a mixture of dichloromethane and methanol at a ratio of 15:1.

PL26-1 (749.62 mg, 1 mmol) was dissolved in DCM, and PL24-1 (425.42 mg, 1 mmol), DCC (206 mg, 1 mmol), and DMAP (12 mg, 0.1 mmol) were added to react overnight at room temperature. When the reaction was finished, the crude product was obtained by adding saturated NaCl solution and extracting with DCM, drying with anhydrous Na₂SO₄, and concentrating under reduced pressure. A liquid compound PL26 (370 mg, 32%) was obtained by purification by column chromatography with eluting using a mixture of dichloromethane and methanol at a ratio of 30:1. MS m/z (ESI): 579.52[M+2H]²⁺.

### Example 15

### Synthesis of PL27

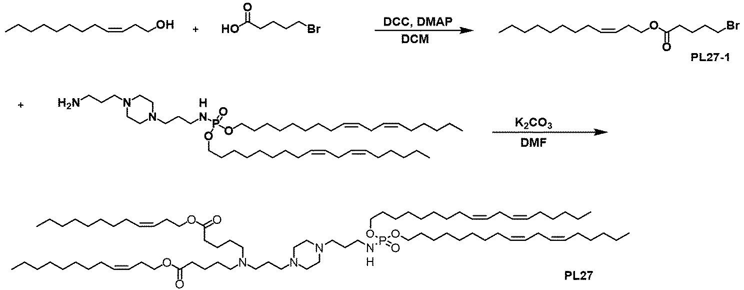

Cis-3-dodecan-1-ol (368 mg, 2 mmol) and 5-bromopentanoic acid (360 mg, 2 mmol) were first dissolved in 10 mL of DCM, followed by the addition of DCC (412 mg, 2 mmol) and DMAP (24 mg, 0.2 mmol) for the reaction at room temperature overnight. After the reaction is complete, a product PL27-1 (301 mg, 87%) was obtained by performing filtration, washing with DCM, and concentrating under reduced pressure and purifying by column chromatography with eluting using a mixture of petroleum ether and ethyl acetate at a ratio of 15:1.

PL14-1 (776.67 mg, 1 mmol) was dissolved in DMF, and PL27-1 (1038 mg, 3 mmol) and K₂CO₃ (414 mg, 3 mmol) were added for reaction overnight at room temperature. When the reaction was finished, the crude product was obtained by adding saturated NaCl solution and extracting with DCM, washing with saturated NaCl solution, drying with anhydrous Na₂SO₄, and concentrating under reduced pressure. A liquid compound PL27 (995 mg, 76%) was obtained by purification by column chromatography with eluting using a mixture of dichloromethane and methanol at a ratio of 15:1. ¹H NMR (400 MHz, CDCl₃): 5.56-5.32 (m, 12H), 4.08 (t, J=8Hz, 4H), 3.98 (t, J=8Hz, 4H), 2.10-2.03(m, 12H), 1.72-1.64 (m, 16H), 1.40-1.29(m,56H), 0.93-0.89(m,12H).

### Example 16

### Synthesis of PL28

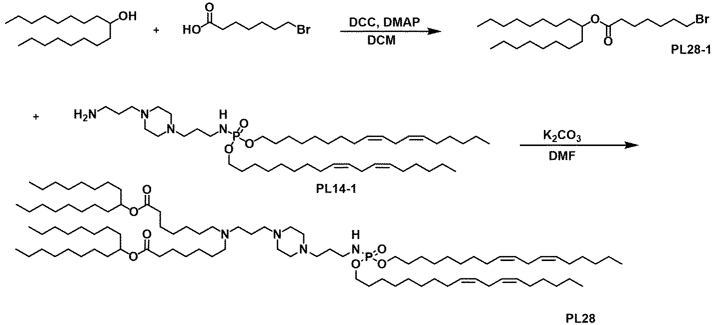

9-heptadecanol (513 mg, 2 mmol) and 7-bromoheptanoic acid (416 mg, 2 mmol) were first dissolved in 10 mL of DCM, followed by the addition of DCC (412 mg, 2 mmol) and DMAP (24 mg, 0.2 mmol) to react at room temperature overnight. After the reaction is complete, a product PL28-1 (788 mg, 88%) was obtained by performing filtration, washing with DCM, and concentrating under reduced pressure and purifying by column chromatography with eluting using a mixture of petroleum ether and ethyl acetate at a ratio of 20:1. 1H NMR (400 MHz, CDCl₃): 4.91-4.85 (m, 1H), 3.41 (t, J=4Hz, 2H), 2.30 (t, J=8Hz, 2H), 1.91-1.84 (m, 2H), 1.69-1.62 (m, 2H), 1.54-1.44 (m ,6H),1.1.41-1.27 (m, 26H), 0.89 (t, J=8Hz, 6H).

PL14-1 (776.67 mg, 1 mmol) was dissolved in DMF and PL28-1 (1338 mg, 3 mmol) and K₂CO₃ (414 mg, 3 mmol) were added to react overnight at room temperature. When the reaction was finished, the crude product was obtained by adding saturated NaCl solution and extracting with DCM, washing with saturated NaCl solution, drying with anhydrous Na₂SO₄, and concentrating under reduced pressure. A liquid compound PL28 (643 mg, 48%) was obtained by purification by column chromatography with eluting using a mixture of dichloromethane and methanol at a ratio of 20:1. ¹H NMR (400 MHz, CDCl₃): 5.43-5.31 (m, 8H), 4.90-4.84 (m, 2H), 3.02-2.27 (m, 28H), 3.03-2.77 (m, 10H), 2.09-2.04(m ,8H), 1.71-1.49 (m, 24H), 1.39-1.27(m, 92H), 0.92-0.87(m,18H).

### Example 17

### Synthesis of PL29

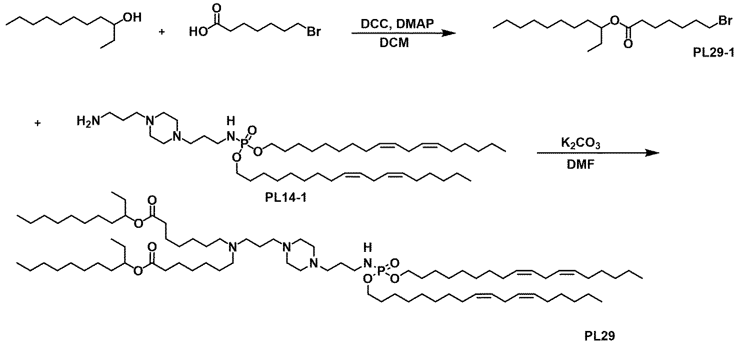

3-undecanol (344 mg, 2 mmol) and 7-bromoheptanoic acid (416 mg, 2 mmol) were first dissolved in 5 mL of DCM, followed by the addition of DCC (412 mg, 2 mmol) and DMAP (24 mg, 0.2 mmol) to react at room temperature overnight. After the reaction was complete, a product PL29-1 (596 mg, 82%) was obtained by performing filtration, washing with DCM, and concentrating under reduced pressure and purifying by column chromatography with eluting using a mixture of petroleum ether and ethyl acetate at a ratio of 20:1. **¹H NMR** (400 MHz, CDCl₃): 4.84-4.81 (m, 1H), 3.41 (t, *J*=4Hz, 2H), 2.31 (t, J=8Hz, 2H), 1.91-1.84 (m, 2H), 1.70-1.61 (m, 2H), 1.41-1.33 (m, 2H), 1.1.33-1.27 (m, 12H), 0.89 (t, J=8Hz, 6H).

PL14-1 (776.67 mg, 1 mmol) was dissolved in DMF, and PL29-1 (1086 mg, 3 mmol) and K₂CO₃ (414 mg, 3 mmol) were added to react overnight at room temperature. When the reaction was finished, the crude product was obtained by adding saturated NaCl solution and extracting with DCM, washing with saturated NaCl solution, drying with anhydrous Na₂SO₄, and concentrating under reduced pressure. A liquid compound PL29 (643 mg, 48%) was obtained by purification by column chromatography with eluting using a mixture of dichloromethane and methanol at a ratio of 20:1. ¹H NMR (400 MHz, CDCl₃): 5.44-5.31 (m, 8H), 4.86-4.79 (m, 2H), 4.04-3.94 (m, 4H), 3.04-2.77 (m, 10H), 2.58-2.24 (m ,18H), 2.09-2.08(m ,8H), 1.71-1.49 (m, 20H), 1.39-1.28(m, 68H), 0.92-0.87(m,18H).

### Example 18

### Synthesis of PL30

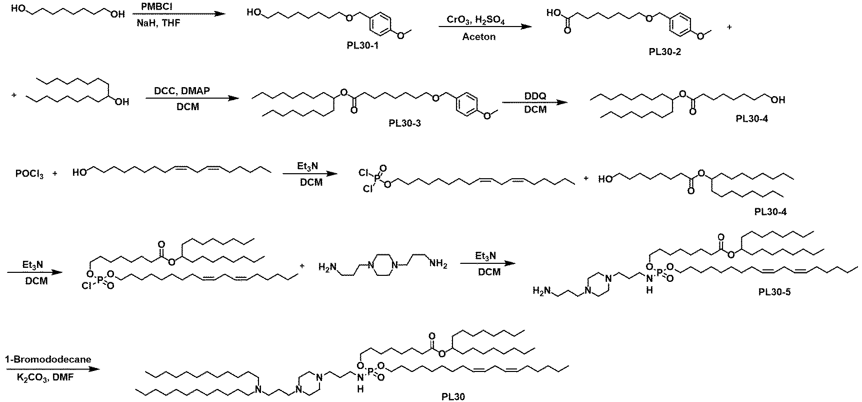

1, 8-octanediol (730 mg, 5 mmol) was first dissolved in 5 mL of THF, and then NaH (240 mg, 10 mmol) was added in an ice bath for reaction for 10 min before 4-methoxybenzyl chloride (783 mg, 5 mmol) was added dropwise to the system to react at room temperature overnight. After the reaction was complete, a product PL30-1 was obtained by quenching the reaction by adding water, extracting with DCM, concentrating under reduced pressure, purifying by column chromatography with eluting using a mixture of petroleum ether and ethyl acetate at a ratio of 5:1. PL30-1 was dissolved in acetone, and Jones reagent was added dropwise in an ice bath until the system showed brownish yellow color, and the reaction was performed at room temperature for 2 h. After the reaction was complete, a liquid compound PL30-2 was obtained by quenching the reaction with water, and extracting with DCM, concentrating under reduced pressure, purifying by column chromatography with eluting using a mixture of petroleum ether and ethyl acetate at a ratio of 5:1. PL30-2 (560 mg, 2 mmol) and 9-heptadecanol (513 mg, 2 mmol) were dissolved in 10 mL of DCM, followed by the addition of DCC (412 mg, 2 mmol) and DMAP (24 mg, 0.2 mmol) to react overnight at room temperature. After the reaction was complete, a product PL30-3 was obtained by performing filtration, washing with DCM, and concentrating under reduced pressure and purifying by column chromatography with eluting using with a mixture of petroleum ether and ethyl acetate at a ratio of 25:1. A solvent mixture of DCM/H₂O (10 mL/1 mL) was added to PL30-3 (518 mg, 1 mmol), followed by the addition of DDQ (227 mg, 1 mmol) for reaction for 4 h at room temperature. After the reaction was complete, a product PL30-4 was obtained by adding water, extracting with ethyl acetate and washing for several times with water, and then concentrating and purifying by column chromatography with eluting using a mixture of petroleum ether and ethyl acetate at a ratio of 25:1. **¹H NMR** (400 MHz, CDCl₃): 4.91-4.85 (m, 1H), 3.98 (t, J=8Hz, 2H), 2.30 (t, J=8Hz, 2H), 1.69-1.50 (m, 8H), 1.36-1.28 (m, 18H), 0.90 (t, J=8Hz, 6H).

A solution was prepared by dissolving linolenol (266 mg, 1 mmol) in 5 mL of DCM and adding 1 mmol of triethylamine, and then the above solution was slowly added dropwise to phosphorous oxychloride (153 mg, 1 mmol) in an ice bath (0°C). The reaction system was protected with nitrogen and reacted at 0°C-5°C for 2 h. Subsequently, a mixed solution of **PL30-4** (398 mg, 1 mmol) and triethylamine (1 eq) in dichloromethane was added dropwise to the system and reacted at 5°C-RT for 2 h. Immediately thereafter, the above system was added dropwise to a mixed solution of 1,4-bis(3-aminopropyl) piperazine (400 mg, 2 mmol) and triethylamine (2 eq) in dichloromethane and reacted for 2 h at room temperature. When the reaction was complete, the crude product was obtained by washing with saturated NaHCO₃ and concentrating under reduced pressure. A liquid compound PL30-5 was obtained by purification by column chromatography with eluting using a mixture of dichloromethane and methanol and ammonia at a ratio of 50:5:1. PL30-5 (908 mg,1 mmol) was dissolved in DMF and 1-bromododecane (747 mg, 3 mmol) and K₂CO₃ (414 mg, 3 mmol) were added separately to react at room temperature overnight. When the reaction was complete, the crude product was obtained by quenching the reaction by adding water and extracting with dichloromethane, washing three times with water, and concentrating under reduced pressure. A liquid compound PL30 (510.53 mg, 41%) was obtained by purification by column chromatography with eluting using a mixture of dichloromethane and methanol at a ratio of 20:1. ¹H NMR (400 MHz, MeOD): 5.44-5.32 (m, 4H), 4.91-4.85 (m, 1H), 4.00-3.94 (m, 4H), 3.02-2.28 (m, 24H), 2.08-2.04(m ,4H), 1.71-1.62 (m, 14H), 1.36-1.28 (m, 84H), 0.92-0.87 (m, 18H).

### Example 19

### Synthesis of PL31

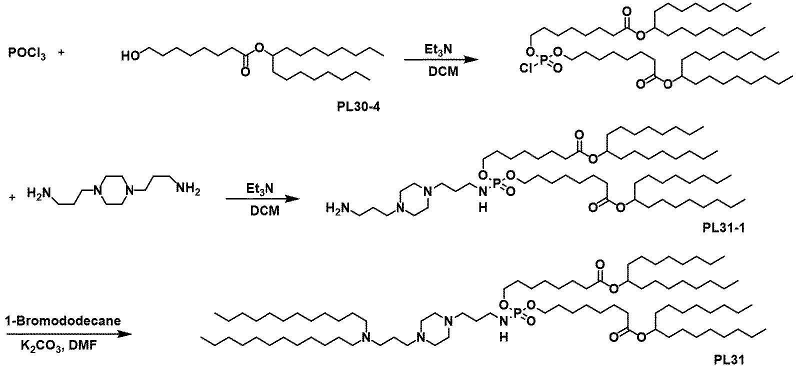

A solution was prepared by dissolving PL30-4 (796 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and reacted at 5°C-20°C for 2 h. Subsequently, a mixed solution of 1,4-bis(3-aminopropyl) piperazine (400 mg, 2 mmol) and triethylamine (2 eq) in dichloromethane was added dropwise to the system and reacted for 2 h at room temperature. When the reaction was complete, the crude product was obtained by washing with saturated NaHCO₃ and concentrating under reduced pressure. A liquid compound PL31-1 was obtained by purification by column chromatography with eluting using a mixture of dichloromethane and methanol and ammonia at ratio of 45:3: 1. PL31-1 (1041 mg, 1 mmol) was dissolved in DMF, and 1-bromododecane (747 mg, 3 mmol) and K₂CO₃ (414 mg, 3 mmol) were added separately to react at room temperature overnight. When the reaction was complete, the crude product was obtained by quenching the reaction by adding water and extracting with dichloromethane, washing three times with water, and concentrating under reduced pressure. A liquid compound PL31 (729 mg, 57%) was obtained by purification by column chromatography with eluting using a mixture of dichloromethane and methanol at a ratio of 15:1. ¹H NMR (400 MHz, CDCl₃): 4.89-4.86 (m, 2H), 4.00-3.94 (m, 4H), 2.99-2.01(m, 24H), 1.75-1.50 (m ,20H), 1.36-1.28 (m, 96H), 0.92-0.88 (m, 18H).

### Example 20

### Synthesis of PL32

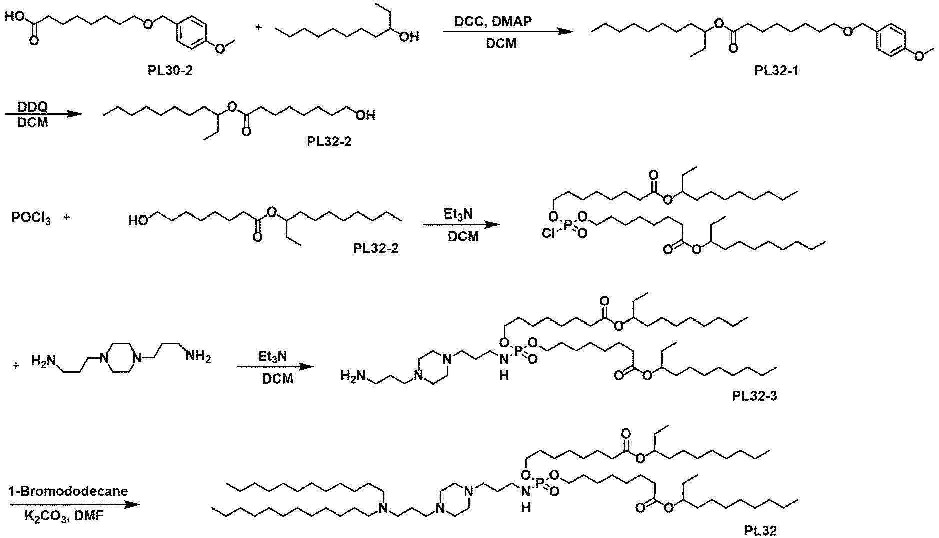

PL30-2 (560 mg, 2 mmol) and 3-undecanol (344 mg, 2 mmol) were dissolved in 10 mL of DCM, followed by the addition of DCC (412 mg, 2 mmol) and DMAP (24 mg, 0.2 mmol) to react overnight at room temperature. After the reaction was complete, a product PL32-1 was obtained by performing filtration, washing with DCM, and concentrating under reduced pressure and purifying by column chromatography with eluting using a mixture of petroleum ether and ethyl acetate at a ratio of 25:1. A solvent mixture of DCM/H₂O (10 mL/1 mL) was added to PL32-1 (434 mg, 1 mmol), followed by the addition of DDQ (227 mg, 1 mmol) to react for 4 h at room temperature. After the reaction was complete, a product PL32-2 was obtained by adding water, extracting with ethyl acetate and washing for several times with water, concentrating, and purifying by column chromatography with elution using a mixture of petroleum ether and ethyl acetate at a ratio of 25:1. ¹H NMR (400 MHz, CDCl₃): 4.86-4.80 (m, 1H), 3.98 (t, J=8Hz, 2H), 2.31 (t, *J*=8Hz, 2H), 1.69-1.51 (m, 8H), 1.36-1.28 (m, 18H), 0.91-0.87 (m, 6H).

A solution was prepared by dissolving PL32-2 (628 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and reacted at 5°C-20°C for 2 h. Subsequently, the above system was added dropwise to a mixed solution of 1,4-bis(3-aminopropyl) piperazine (400 mg, 2 mmol) and triethylamine (2 eq) in dichloromethane for reaction at room temperature for 2 h. When the reaction was complete, the crude product was obtained by washing with saturated NaHCO₃ and concentrating under reduced pressure. A liquid compound PL32-3 was obtained by purification by column chromatography with eluting using a mixture of dichloromethane and methanol and ammonia at a ratio of 45:3:1. PL32-3 (873 mg,1 mmol) was dissolved in DMF, and 1-bromododecane (747 mg, 3 mmol) and K₂CO₃ (414 mg, 3 mmol) were added separately to react at room temperature overnight. When the reaction was complete, the crude product was obtained by quenching the reaction by adding water and extracting with dichloromethane, washing three times with water, and concentrating under reduced pressure. A liquid compound PL32 (665.43 mg, 55%) was obtained by purification by column chromatography with eluting using a mixture of dichloromethane and methanol at a ratio of 20:1. ¹H NMR (400 MHz, CDCl₃): 4.85-4.81 (m, 2H), 4.00-3.94 (m, 4H), 3.03-2.96 (m, 4H), 2.47-2.29(m, 20H), 1.69-1.51 (m ,20H), 1.36-1.28 (m, 76H), 0.92-0.87 (m, 18H).

### Example 21

### Synthesis of PL33

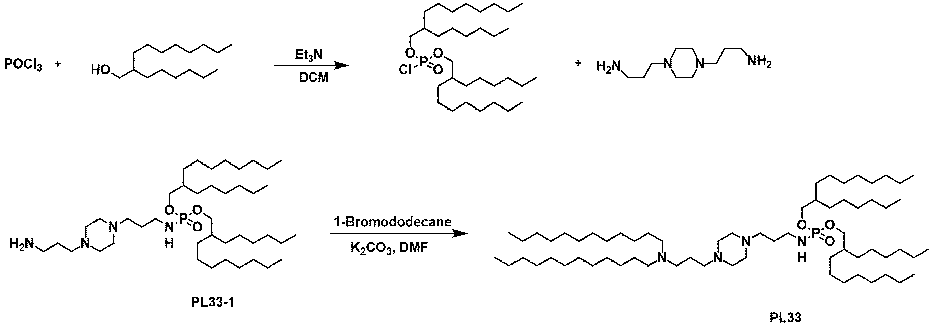

A solution was prepared by dissolving 2-hexyl-1-decanol (484 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and reacted at 5°C-20°C for 2 h. Subsequently, the above reaction system was added dropwise to a mixed solution of 1,4- bis(3-aminopropyl) piperazine (400 mg, 2 mmol) and triethylamine (2 eq) in dichloromethane to react for 2 h at room temperature. When the reaction was complete, the crude product was obtained by washing with saturated NaHCO₃ and concentrating under reduced pressure. A liquid compound PL33-1 was obtained by purification by column chromatography with eluting using a mixture of dichloromethane and methanol and ammonia at a ratio of 50:5:1. PL33-1 (729 mg,1 mmol) was dissolved in DMF, and 1-bromododecane (747 mg, 3 mmol) and K₂CO₃ (414 mg, 3 mmol) were added separately to react at room temperature overnight. When the reaction was complete, the crude product was obtained by quenching the reaction by adding water and extracting with dichloromethane, washing three times with water, and concentrating under reduced pressure. A liquid compound PL33 (734.85 mg, 69%) was obtained by purification by column chromatography with eluting using a mixture of dichloromethane and methanol at a ratio of 20:1. **¹H NMR** (400 MHz, CDCl₃): 3.93-3.83 (m, 4H), 3.02-2.27 (m, 20H), 1.69-1.61 (m, 6H), 1.48-1.14 (m, 88H), 0.90 (t, J=8Hz 18H).

### Example 22

### Synthesis of PL36

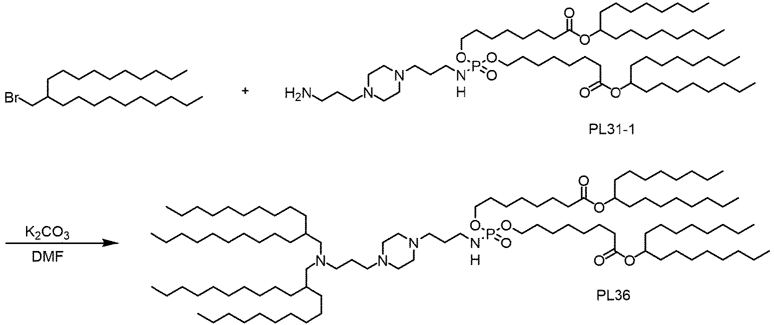

PL31-1 (1041 mg,1 mmol) was dissolved in DMF, and 11-(bromomethyl) heneicosane (1165 mg, 3 mmol) and K₂CO₃ (414 mg, 3 mmol) were added separately to react at room temperature overnight. When the reaction was complete, the crude product was obtained by quenching the reaction by adding water and extracting with dichloromethane, washing three times with water, and concentrating under reduced pressure. A liquid compound PL36 (499.13 mg, 37%) was obtained by purification by column chromatography with eluting using a mixture of dichloromethane and methanol at a ratio of 15:1. ¹H NMR (400 MHz, CDCl₃): 4.88 (t, J=8Hz, 2H), 3.99-3.94 (m, 4H), 3.02-2.00 (m, 24H), 1.71-1.50 (m, 20H), 1.36-1.23 (m, 134H), 0.91-0.88 (m, 24H).

### Example 23

### Synthesis of PL38

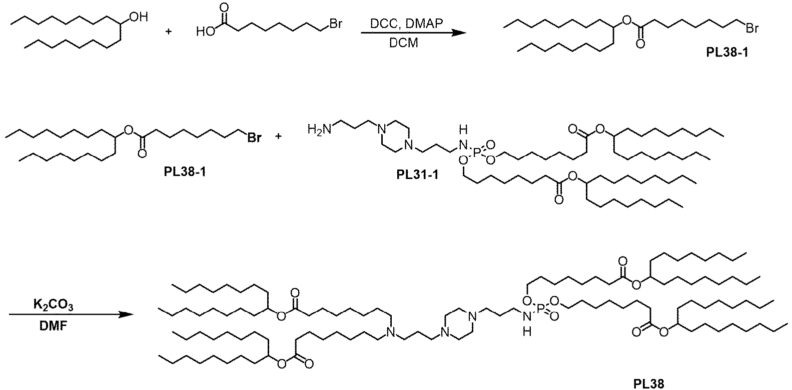

9-heptadecanol (513 mg, 2 mmol) and 8-bromooctanoic acid (444 mg, 2 mmol) were first dissolved in 10 mL of DCM, followed by the addition of DCC (412 mg, 2 mmol) and DMAP (24 mg, 0.2 mmol) to react at room temperature overnight. After the reaction was complete, a product PL38-1 was obtained by performing filtration, washing with DCM, and concentrating under reduced pressure and purifying by column chromatography with eluting using a mixture of petroleum ether and ethyl acetate at a ratio of 20:1. PL31-1 (1041 mg, 1 mmol) was dissolved in DMF, followed by the addition of PL38-1 (1385 mg, 3 mmol) and K₂CO₃ (414 mg, 3 mmol) to react overnight at room temperature. After the reaction was finished, the crude product was obtained by adding saturated NaCl solution and extracting with DCM, washing with saturated NaCl solution, drying with anhydrous Na₂SO₄, and concentrating under reduced pressure. A liquid compound PL38 (643 mg, 48%) was obtained by purification by column chromatography with eluting using a mixture of dichloromethane and methanol at ratio of 20:1. ¹H NMR (400 MHz, CDCl₃): 4.90-4.84 (m, 4H), 4.02-3.94 (m, 4H), 3.02-2.27 (m, 28H), 1.81-1.49 (m, 36H), 1.37-1.27(m, 120H), 0.92-0.87 (m, 24H).

### Example 24

### Synthesis of PL41

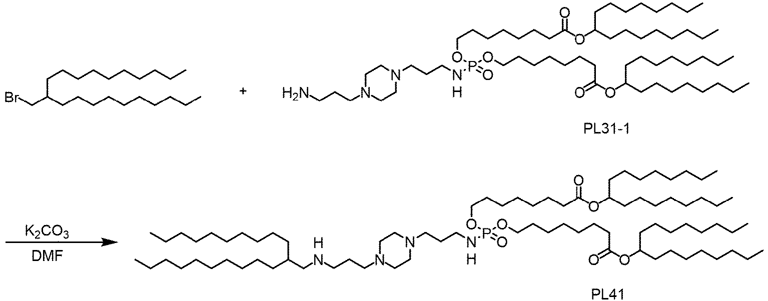

PL31-1 (1040 mg, 1 mmol) was dissolved in DMF and 11-(bromomethyl) heneicosane (582 mg, 1.5 mmol) and K₂CO₃ (414 mg, 3 mmol) were added separately to react at room temperature overnight. After the reaction was complete, the crude product was obtained by quenching the reaction by adding water and extracting with dichloromethane, washing three times with water, and concentrating under reduced pressure. A liquid compound PL41 (499.13 mg, 37%) was obtained by purification by column chromatography with eluting using a mixture of dichloromethane and methanol at a ratio of 15:1. ¹H NMR (400 MHz, CDCl₃): 4.89-4.86 (m, 2H), 4.00-3.94 (m, 4H), 3.12-2.18 (m, 22H), 1.69-1.50 (m ,24H), 1.40-1.28 (m, 88H), 0.92-0.88 (m, 18H).

### Example 25

### Synthesis of PL44

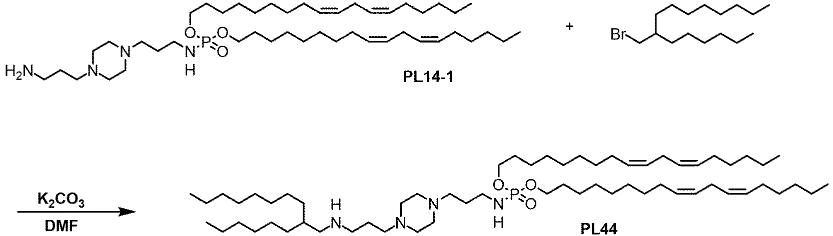

PL14-1 (776.67 mg, 1 mmol) was first dissolved in 5 mL of DMF and 1-bromo-2-hexyldecane (913 mg, 3 mmol) and K₂CO₃ (414 mg, 3 mmol) were added to react at room temperature overnight. After the reaction was finished, the crude product was obtained by adding saturated NaCl solution and extracting with DCM, washing with saturated NaCl solution, drying with anhydrous Na₂SO₄, and concentrating under reduced pressure. A liquid compound PL44 (380.38 mg, 38%) was obtained by purification by column chromatography with eluting using a mixture of dichloromethane and methanol at a ratio of 30:1. ¹H NMR (400 MHz, CDCl₃): 5.44-5.32 (m, 8H), 4.02-3.94 (m, 4H), 3.14-2.53 (m, 22H), 2.16-2.04 (m, 8H), 1.72-1.64 (m, 8H), 1.41-1.28 (m, 57H), 0.93-0.89 (m, 12H)..

### Example 26

### Synthesis of PL45

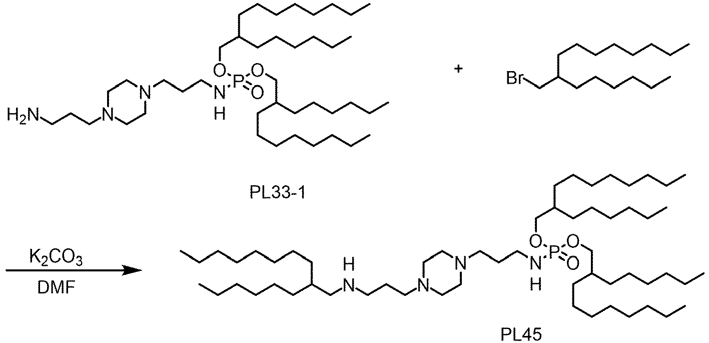

PL33-1 (729 mg, 1 mmol) was first dissolved in DMF, and 1-bromo-2-hexyldecane (913 mg, 3 mmol) and K₂CO₃ (414 mg, 3 mmol) were added to react at room temperature overnight. After the reaction was complete, the crude product was obtained by quenching the reaction by adding water and extracting with dichloromethane, washing three times with water, and concentrating under reduced pressure. A liquid compound PL45 (295 mg, 31%) was obtained by purification by column chromatography with eluting using a mixture of dichloromethane and methanol at a ratio of 20:1. ¹H NMR (400 MHz, CDCl₃): 3.92-3.85 (m, 4H), 3.03-2.43 (m, 18H), 1.71-1.60 (m, 6H), 1.44-1.29 (m, 73H), 0.90 (t, J=8Hz 18H).

### Example 27

### Synthesis of PL53

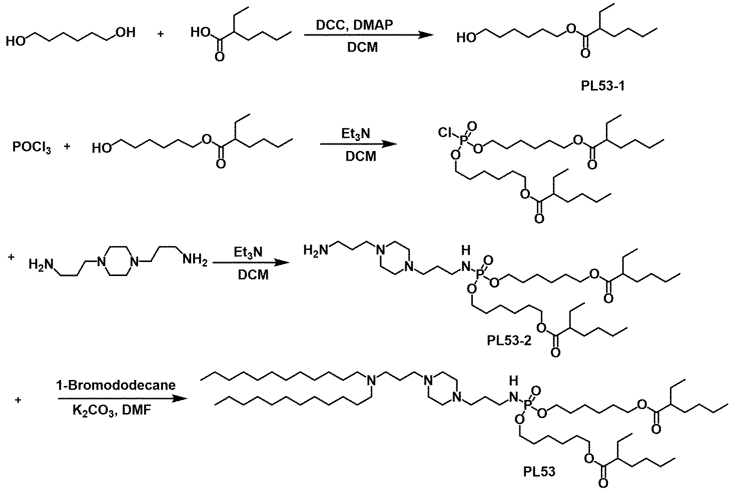

1,6-hexanediol (1.18 g, 10 mmol) and 2-ethylhexanoic acid (1.44 g, 10 mmol) were first dissolved in 20 mL of DCM, followed by the addition of DCC (2.06 g, 10 mmol) and DMAP (122 mg, 1 mmol) to react at room temperature overnight. After the reaction was complete, a product PL53-1 was obtained by performing filtration, washing with DCM, and concentrating under reduced pressure and then purifying by column chromatography with eluting using a mixture of petroleum ether and ethyl acetate at a ratio of 25:1.

A solution was prepared by dissolving PL53-1 (488 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and reacted at 5°C-20°C for 2 h. Subsequently, the above system was added dropwise to a mixed solution of 1,4-bis(3-aminopropyl) piperazine (400 mg, 2 mmol) and triethylamine (2 eq) in dichloromethane for reaction for 2 h at room temperature. After the reaction was complete, the crude product was obtained by washing with saturated NaHCO₃ and concentrating under reduced pressure. A liquid compound PL53-2 was obtained by purification by column chromatography with eluting using a mixture of dichloromethane and methanol and ammonia at a ratio of 45:3:1. PL53-2 (732 mg, 1 mmol) was dissolved in DMF and 1-bromododecane (747 mg, 3 mmol) and K₂CO₃ (414 mg, 3 mmol) were added separately to react at room temperature overnight. After the reaction was complete, the crude product was obtained by quenching the reaction by adding water and extracting with dichloromethane, washing three times with water, and concentrating under reduced pressure. A liquid compound PL53 (555.88 mg, 52%) was obtained by purification by column chromatography with eluting using a mixture of dichloromethane and methanol at a ratio of 15:1. ¹H NMR (400 MHz, CDCl₃): 4.09 (t, *J*=8Hz, 4H), 4.02-3.93 (m, 4H), 3.02-2.23 (m, 22H), 1.71-1.38 (m, 28H), 1.25-1.23 (m ,48H), 0.92-0.89 (m, 18H).

### Example 28

### Synthesis of PL54

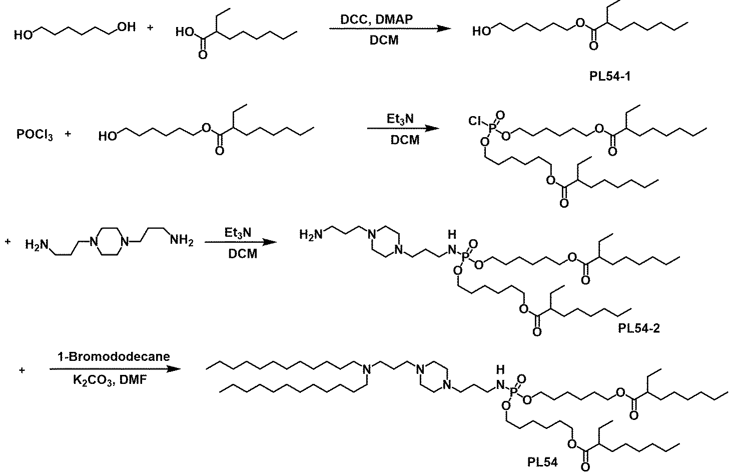

1,6-hexanediol (1.18 g, 10 mmol) and 2-ethyloctanoic acid (1.72 g, 10 mmol) were first dissolved in 20 mL of DCM, followed by the addition of DCC (2.06 g, 10 mmol) and DMAP (122 mg, 1 mmol) to react at room temperature overnight. After the reaction was complete, a product PL54-1 was obtained by performing filtration, washing with DCM, and concentrating under reduced pressure and then purifying by column chromatography with the eluting using a mixture of petroleum ether and ethyl acetate at a ratio of 25:1.

A solution was prepared by dissolving PL54-1 (544 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and reacted at 5°C-20 °C for 2 h. Subsequently, the above system was added dropwise to a mixed solution of 1,4-bis(3-aminopropyl) piperazine (400 mg, 2 mmol) and triethylamine (2 eq) in dichloromethane for 2 h at room temperature. After the reaction was complete, the crude product was obtained by washing with saturated NaCO₃ and concentrating under reduced pressure. A liquid compound PL54-2 was obtained by purification by column chromatography with eluting using a mixture of dichloromethane and methanol and ammonia at a ratio of 45:3:1. PL54-2 (789 mg, 1 mmol) was dissolved in DMF and 1-bromododecane (747 mg, 3 mmol) and K₂CO₃ (414 mg, 3 mmol) were added separately to react at room temperature overnight. After the reaction was complete, the crude product was obtained by quenching the reaction by adding water and extracting with dichloromethane, washing three times with water, and concentrating under reduced pressure. A liquid compound PL54 (753.08 mg, 67%) was obtained by purification by column chromatography with eluting using a mixture of dichloromethane and methanol at a ratio of 15:1. ¹H NMR (400 MHz, CDCl₃): 4.09 (t, J=8Hz, 4H), 4.01-3.93 (m, 4H), 3.02-2.22 (m, 22H), 1.73-1.39 (m, 32H), 1.33-1.28 (m ,52H), 0.92-0.88(m, 18H).

### Example 29

### Synthesis of PL55

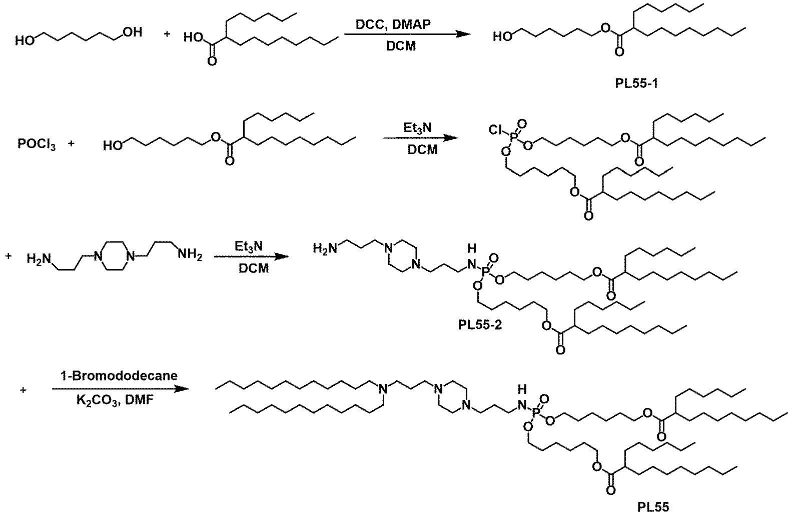

1,6-hexanediol (1.18 g, 10 mmol) and 2-hexanedecanoic acid (2.56 g, 10 mmol) were first dissolved in 20 mL of DCM, followed by the addition of DCC (2.06 g, 10 mmol) and DMAP (122 mg, 1 mmol) to react at room temperature overnight. After the reaction was complete, a product PL55-1 was obtained by performing filtration, washing with DCM, and concentrating under reduced pressure and purifying by column chromatography with eluting using a mixture of petroleum ether and ethyl acetate at a ratio of 25:1.

A solution was prepared by dissolving PL55-1 (712 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and reacted at 5°C-20°C for 2 h. Subsequently, the above system was added dropwise to a mixed solution of 1,4-bis(3-aminopropyl) piperazine (400 mg, 2 mmol) and triethylamine (2 eq) in dichloromethane to react for 2 h at room temperature. After the reaction was complete, the crude product was obtained by washing with saturated NaHCO₃ and concentrating under reduced pressure. A liquid compound PL55-2 was obtained by purification by column chromatography with eluting using a mixture of dichloromethane and methanol and ammonia at a ratio of 45:3:1. PL55-2 (957 mg, 1 mmol) was dissolved in DMF and 1-bromododecane (747 mg, 3 mmol) and K₂CO₃ (414 mg, 3 mmol) were added separately to react at room temperature overnight. After the reaction was complete, the crude product was obtained by quenching the reaction by adding water and extracting with dichloromethane, washing three times with water, and concentrating under reduced pressure. A liquid compound PL55 (853.38 mg, 66%) was obtained by purification by column chromatography with eluting using a mixture of dichloromethane and methanol at a ratio of 15:1. ¹H NMR (400 MHz, CDCl₃): 4.08 (t, J=8Hz, 4H), 4.00-3.97 (m, 4H), 3.00-2.29 (m, 22H), 1.73-1.40 (m, 32H), 1.33-1.05 (m, 76H), 0.91-0.88 (m, 18H).

### Example 30

### Synthesis of PL56

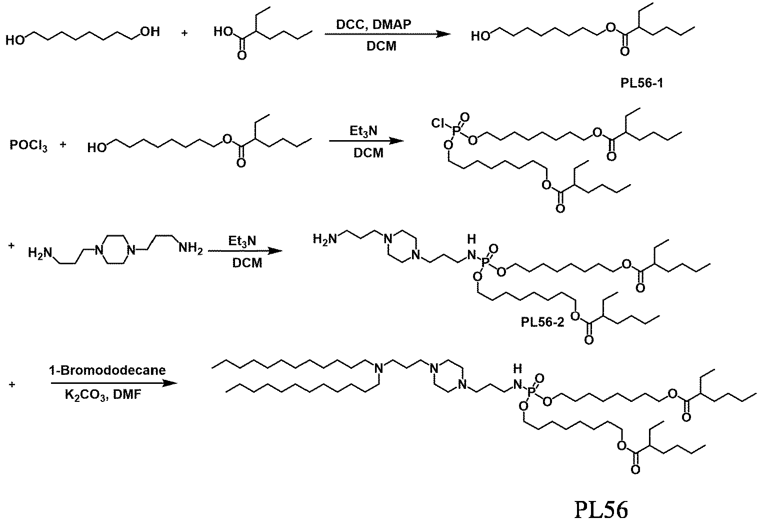

1,8-octanediol (1.46 g, 10 mmol) and 2-ethylhexanoic acid (1.44 g, 10 mmol) were first dissolved in 20 mL of DCM, followed by the addition of DCC (2.06 g, 10 mmol) and DMAP (122 mg, 1 mmol) to react at room temperature overnight. After the reaction was complete, a product PL56-1 was obtained by performing filtration, washing with DCM, and concentrating under reduced pressure and purifying by column chromatography with eluting using a mixture of petroleum ether and ethyl acetate at a ratio of 25: 1.

A solution was prepared by dissolving PL56-1 (544 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and reacted at 5°C-20°C for 2 h. Subsequently, the above system was added dropwise to a mixed solution of 1,4-bis(3-aminopropyl) piperazine (400 mg, 2 mmol) and triethylamine (2 eq) in dichloromethane to react for 2 h at room temperature. After the reaction was complete, the crude product was obtained by washing with saturated NaHCO₃ and concentrating under reduced pressure. A liquid compound PL56-2 was obtained by purification by column chromatography with eluting using a mixture of dichloromethane and methanol and ammonia at a ratio of 45:3:1. PL56-2 (789 mg,1 mmol) was dissolved in DMF and 1-bromododecane (747 mg, 3 mmol) and K₂CO₃ (414 mg, 3 mmol) were added separately to react at room temperature overnight. After the reaction was complete, the crude product was obtained by quenching the reaction by adding water and extracting with dichloromethane, washing three times with water, and concentrating under reduced pressure. A liquid compound PL56 (798.75 mg, 71%) was obtained by purification by column chromatography with eluting using a mixture of dichloromethane and methanol at a ratio of 15:1. ¹H NMR (400 MHz, CDCl₃): 4.08 (t, J=8Hz, 4H), 4.01-3.94 (m, 4H), 3.03-2.23 (m, 22H), 2.0(s, 2H), 1.71-1.43 (m, 24H), 1.37-1.25 (m, 60H), 0.92-0.88 (m, 18H).

### Example 31

### Synthesis of PL57

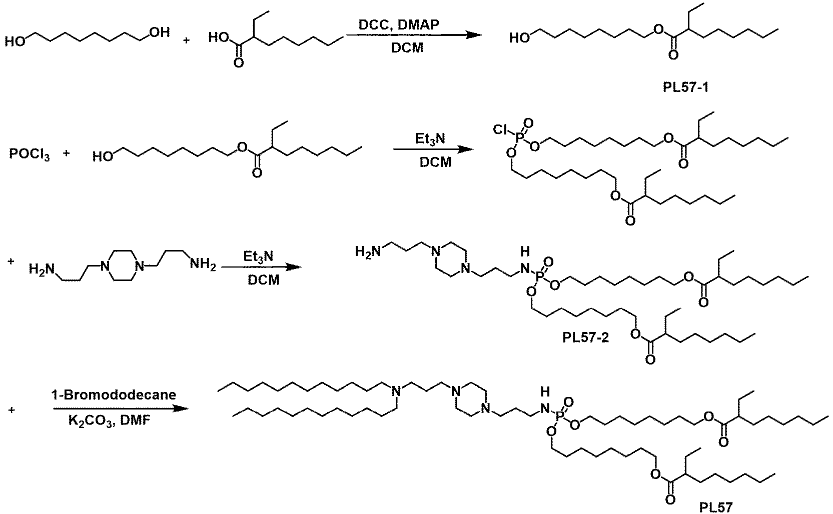

1,8-octanediol (1.46 g, 10 mmol) and 2-ethyloctanoic acid (1.72 g, 10 mmol) were first dissolved in 20 mL of DCM, followed by the addition of DCC (2.06 g, 10 mmol) and DMAP (122 mg, 1 mmol) to react at room temperature overnight. After the reaction was complete, a product PL57-1 was obtained by performing filtration, washing with DCM, and concentrating under reduced pressure and purifying by column chromatography with eluting using a mixture of petroleum ether and ethyl acetate at a ratio of 25: 1.

A solution was prepared by dissolving PL57-1 (601 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and the reacted at 5°C-20°C for 2 h. Subsequently, the above system was added dropwise to a mixed solution of 1,4-bis(3-aminopropyl) piperazine (400 mg, 2 mmol) and triethylamine (2 eq) in dichloromethane to react for 2 h at room temperature. After the reaction was complete, the crude product was obtained by washing with saturated NaHCO₃ and concentrating under reduced pressure. A liquid compound PL57-2 was obtained by purification by column chromatography with eluting using a mixture of dichloromethane and methanol and ammonia at a ratio of 45:3:1. PL57-2 (845 mg,1 mmol) was dissolved in DMF and 1-bromododecane (747 mg, 3 mmol) and K₂CO₃ (414 mg, 3 mmol) were added separately to react at room temperature overnight. After the reaction was complete, the crude product was obtained by quenching the reaction by adding water and extracting with dichloromethane, washing three times with water, and concentrating under reduced pressure. A liquid compound PL57 (732.22 mg, 62%) was obtained by purification by column chromatography with eluting using a mixture of dichloromethane and methanol at a ratio of 15:1. ¹H NMR (400 MHz, CDCl₃): 4.09 (t, J=8Hz, 4H), 4.01-3.94 (m, 4H), 3.01-2.24 (m, 22H), 2.0(s, 2H), 1.72-1.44 (m, 24H), 1.43-1.28 (m, 68H), 0.92-0.88 (m, 18H).

### Example 32

### Synthesis of PL58

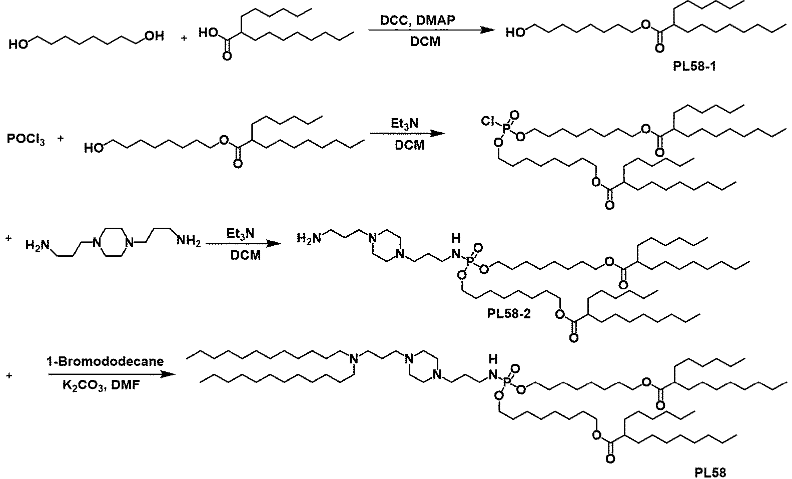

1,8-octanediol (1.46 g, 10 mmol) and 2-hexanedecanoic acid (2.56 g, 10 mmol) were first dissolved in 20 mL of DCM, followed by the addition of DCC (2.06 g, 10 mmol) and DMAP (122 mg, 1 mmol) to react at room temperature overnight. After the reaction was complete, a product PL58-1 was obtained by performing filtration, washing with DCM, and concentrating under reduced pressure and then purifying by column chromatography with eluting using a mixture of petroleum ether and ethyl acetate at a ratio of 25:1.

A solution was prepared by dissolving PL58-1 (769 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and reacted at 5°C-20°C for 2 h. Subsequently, the above system was added dropwise to a mixed solution of 1,4-bis(3-aminopropyl) piperazine (400 mg, 2 mmol) and triethylamine (2 eq) in dichloromethane to react for 2 h at room temperature. After the reaction was complete, the crude product was obtained by washing with saturated NaHCO₃ and concentrating under reduced pressure. A liquid compound PL58-2 was obtained by purification by column chromatography with eluting using a mixture of dichloromethane and methanol and ammonia at a ratio of 45:3:1. PL58-2 (1013 mg, 1 mmol) was dissolved in DMF and 1-bromododecane (747 mg, 3 mmol) and K₂CO₃ (414 mg, 3 mmol) were added separately to react at room temperature overnight. After the reaction was complete, the crude product was obtained by quenching the reaction by adding water and extracting with dichloromethane, washing three times with water, and concentrating under reduced pressure. A liquid compound PL58 (769.5 mg, 57%) was obtained by purification by column chromatography with eluting using a mixture of dichloromethane and methanol at a ratio of 15:1. ¹H NMR (400 MHz, CDCl₃): 4.09 (t, J=8Hz, 4H), 4.00-3.96 (m, 4H), 2.99-2.23 (m, 20H), 2.0 (s, 2H), 1.78-1.45 (m, 28H), 1.41-1.21 (m, 88H), 0.92-0.88 (m, 18H).

### Example 33

### Synthesis of PL59

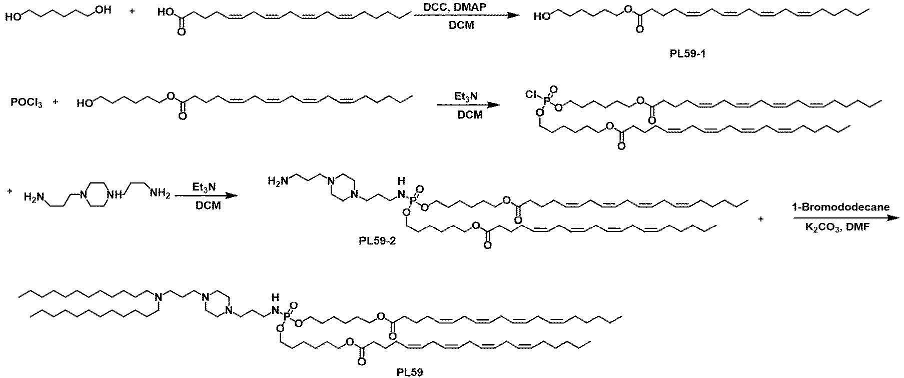

1,6-hexanediol (1.18 g, 10 mmol) and arachidonic acid (3.04 g, 10 mmol) were first dissolved in 20 mL of DCM, followed by the addition of DCC (2.06 g, 10 mmol) and DMAP (122 mg, 1 mmol) to react at room temperature overnight. After the reaction was complete, a product PL59-1 was obtained by performing filtration, washing with DCM, and concentrating under reduced pressure and then purifying by column chromatography with eluting using a mixture of petroleum ether and ethyl acetate at a ratio of 25:1.

A solution was prepared by dissolving PL59-1 (808 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and reacted at 5°C-20°C for 2 h. Subsequently, the above system was added dropwise to a mixed solution of 1,4-bis(3-aminopropyl) piperazine (400 mg, 2 mmol) and triethylamine (2 eq) in dichloromethane to react for 2 h at room temperature. After the reaction was complete, the crude product was obtained by washing with saturated NaHCO₃ and concentrating under reduced pressure. A liquid compound PL59-2 was obtained by purification by column chromatography with eluting using a mixture of dichloromethane and methanol and ammonia at a ratio of 45:3:1. PL59-2 (1053 mg,1 mmol) was dissolved in DMF and 1-bromododecane (747 mg, 3 mmol) and K₂CO₃ (414 mg, 3 mmol) were added separately to react at room temperature overnight. After the reaction was complete, the crude product was obtained by quenching the reaction by adding water and extracting with dichloromethane, washing three times with water, and concentrating under reduced pressure. A liquid compound PL59 (945.2 mg, 68%) was obtained by purification by column chromatography with eluting using a mixture of dichloromethane and methanol at a ratio of 15:1. ¹H NMR (400 MHz, CDCl₃): 5.46-5.32 (m, 16H), 4.08 (t, J=8Hz, 4H), 3.99-3.91 (m, 4H), 3.28-2.39 (m, 32H), 2.33 (t, J=8Hz, 4H), 2.16-2.05(m, 8H), 1.87-1.59(m, 24H), 1.43-1.28 (m ,52H), 0.93-0.88 (m, 12H)

### Example 34

### Synthesis of PL60

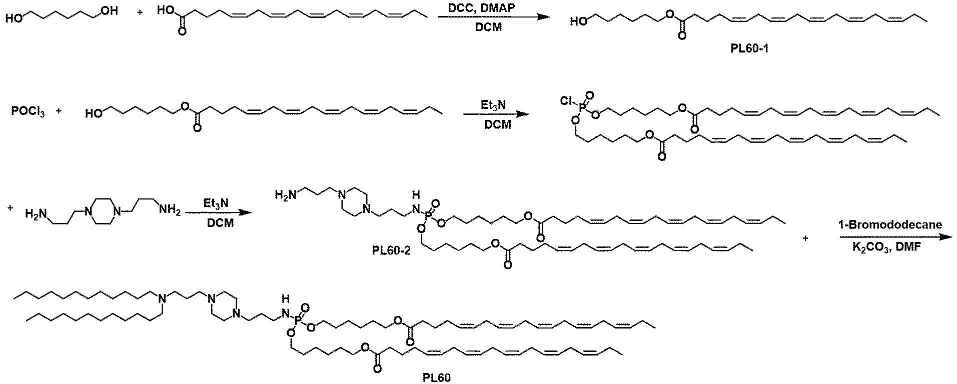

1,6-hexanediol (1.18 g, 10 mmol) and eicosapentaenoic acid (3.02 g, 10 mmol) were first dissolved in 20 mL of DCM, followed by the addition of DCC (2.06 g, 10 mmol) and DMAP (122 mg, 1 mmol) to react at room temperature overnight. After the reaction was complete, a product PL60-1 was obtained by performing filtration, washing with DCM, and concentrating under reduced pressure and then purifying by column chromatography with a mixture of petroleum ether and ethyl acetate at a ratio of 25:1.

A solution was prepared by dissolving PL60-1 (804 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and reacted at 5°C-20 °C for 2 h. Subsequently, the above system was added dropwise to a mixed solution of 1,4-bis(3-aminopropyl) piperazine (400 mg, 2 mmol) and triethylamine (2 eq) in dichloromethane to react for 2 h at room temperature. After the reaction was complete, the crude product was obtained by washing with saturated NaHCO₃ and concentrating under reduced pressure. A liquid compound PL60-2 was obtained by purification by column chromatography with eluting using a mixture of dichloromethane and methanol and ammonia at a ratio of 45:3:1. PL60-2 (1049 mg,1 mmol) was dissolved in DMF and 1-bromododecane (747 mg, 3 mmol) and K₂CO₃ (414 mg, 3 mmol) were added separately to react at room temperature overnight. After the reaction was complete, the crude product was obtained by quenching the reaction by adding water and extracting with dichloromethane, washing three times with water, and concentrating under reduced pressure. A liquid compound PL60 (747.9 mg, 54%) was obtained by purification by column chromatography with eluting using a mixture of dichloromethane and methanol at a ratio of 15:1. ¹H NMR (400 MHz, CDCl₃): 5.45-5.32 (m, 20H), 4.08 (t, J=8Hz, 4H), 4.04-3.94 (m, 4H), 3.02-2.45 (m, 32H), 2.33 (t, J=8Hz, 4H), 2.16-2.06(m, 8H), 1.76-1.59(m, 24H), 1.42-1.28 (m ,40H), 0.99 (t, J=8Hz, 4H), 0.90 (t, J=8Hz, 4H).

### Example 35

### Synthesis of PL61

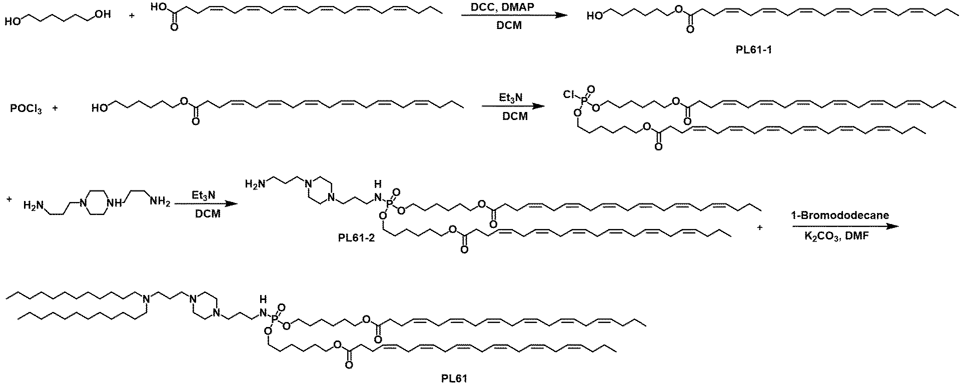

1,6-hexanediol (1.18 g, 10 mmol) and tricosahahexaenoic acid (3.42 g, 10 mmol) were first dissolved in 20 mL of DCM, followed by the addition of DCC (2.06 g, 10 mmol) and DMAP (122 mg, 1 mmol) to react at room temperature overnight. After the reaction was complete, a product PL61-1 was obtained by performing filtration, washing with DCM, and concentrating under reduced pressure and purifying by column chromatography with eluting using a mixture of petroleum ether and ethyl acetate at a ratio of 25:1.

A solution was prepared by dissolving PL61-1 (885 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and reacted at 5°C-20 °C for 2 h. Subsequently, the above system was added dropwise to a mixed solution of 1,4-bis(3-aminopropyl) piperazine (400 mg, 2 mmol) and triethylamine (2 eq) in dichloromethane to react for 2 h at room temperature. After the reaction was complete, the crude product was obtained by washing with saturated NaHCO₃ and concentrating under reduced pressure. A liquid compound PL61-2 was obtained by purification by column chromatography with eluting using a mixture of dichloromethane and methanol and ammonia at a ratio of 45:3:1. PL61-2 (1129 mg, 1 mmol) was dissolved in DMF and 1-bromododecane (747 mg, 3 mmol) and K₂CO₃ (414 mg, 3 mmol) were added separately to react at room temperature overnight. After the reaction was complete, the crude product was obtained by quenching the reaction by adding water and extracting with dichloromethane, washing three times with water and concentrating under reduced pressure. A liquid compound PL61 (673.9 mg, 46%) was obtained by purification by column chromatography with eluting using a mixture of dichloromethane and methanol at a ratio of 15:1. ¹H NMR (400 MHz, CDCl₃): 5.43-5.32 (m, 24H), 4.08 (t, J=8Hz, 4H), 4.02-3.94 (m, 4H), 3.01-2.32 (m, 48H), 2.13-2.06(m, 4H), 1.79-1.64(m, 22H), 1.42-1.28 (m ,44H), 0.98 (t, J=8Hz, 4H), 0.90 (t, *J*=8Hz, 4H).

### Example 36

### Synthesis of PL78

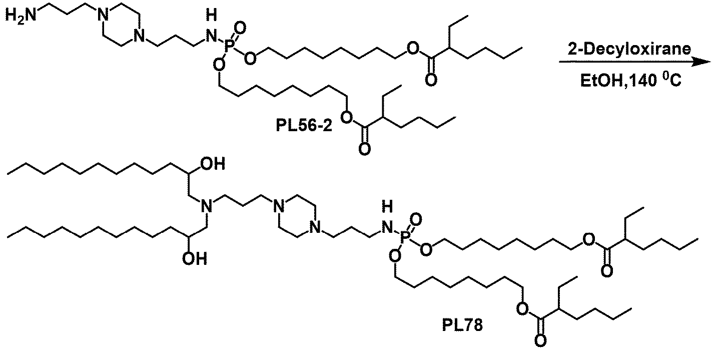

PL56-2 (789 mg,1 mmol) was dissolved in EtOH, 1,2-epoxydodecane (737 mg, 4 mmol) was added, and the reaction was carried out in microwave at 140°C for 30 min. After the reaction was finished, the crude product was obtained by concentrating under reduced pressure. A liquid compound PL78 (728.91 mg, 63%) was obtained by purification by column chromatography with eluting using a mixture of dichloromethane and methanol at a ratio of 20:1. ¹H NMR (400 MHz, CDCl₃): 4.08 (t, J=8Hz, 4H), 4.03-3.91 (m, 4H), 3.69-3.66 (m, 2H), 3.03-2.23 (m, 22H), 1.69-1.43(m, 20H), 1.40-1.22 (m, 60H), 0.92-0.88 (m, 18H).

### Example 37

### Synthesis of PL79

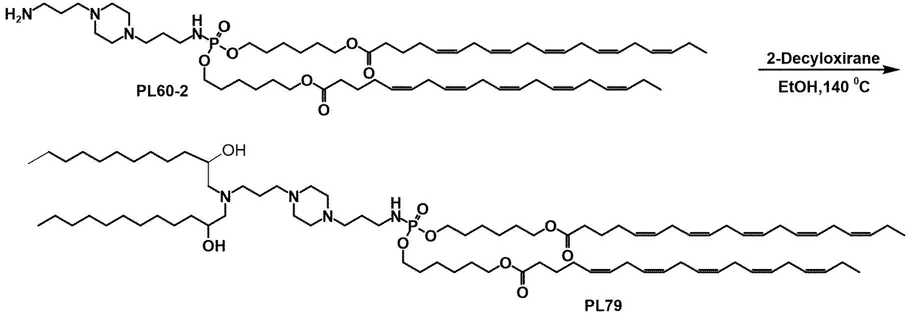

PL60-2 (1049 mg,1 mmol) was dissolved in EtOH, 1,2-epoxydodecane (737.2 mg, 4 mmol) was added, and the reaction is carried out in microwave at 140°C for 30 min. After the reaction was finished, the crude product was obtained by concentrating under reduced pressure. A liquid compound PL79 (935.88 mg, 66%) was obtained by purification by column chromatography with eluting using a mixture of dichloromethane and methanol at a ratio of 20:1. ¹H NMR (400 MHz, CDCl₃): 5.44-5.30 (m, 20H), 4.07 (t, J=8Hz, 4H), 4.01-3.94 (m, 4H), 3.67-3.61 (m, 2H), 3.00-2.29 (m, 40H), 2.16-2.06 (m, 8H), 1.76-1.61(m, 16H), 1.49-1.22 (m, 44H), 0.99 (t, J=8Hz, 4H), 0.90 (t, J=8Hz, 4H).

### Example 38

### Synthesis of PL93

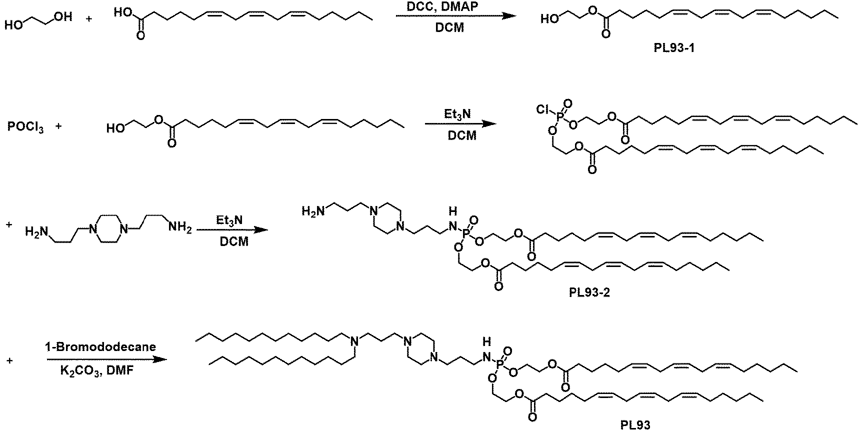

Ethylene glycol (0.62 g, 10 mmol) and linolenic acid (2.78 g, 10 mmol) were first dissolved in 20 mL of DCM, followed by the addition of DCC (2.06 g, 10 mmol) and DMAP (122 mg, 1 mmol) to react at room temperature overnight. After the reaction was complete, a product PL93-1 was obtained by performing filtration, washing with DCM, and concentrating under reduced pressure and then purifying by column chromatography with eluting using a mixture of petroleum ether and ethyl acetate at a ratio of 25: 1.

A solution was prepared by dissolving PL93-1 (645 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and reacted at 5°C-20°C for 2 h. Subsequently, the above system was added dropwise to a mixed solution of 1,4-bis(3-aminopropyl) piperazine (400 mg, 2 mmol) and triethylamine (2 eq) in dichloromethane to react for 2 h at room temperature. After the reaction was complete, the crude product was obtained by washing with saturated NaHCO₃ and concentrating under reduced pressure. A liquid compound PL93-2 was obtained by purification by column chromatography with eluting using a mixture of dichloromethane and methanol and ammonia at a ratio of 45:3:1. PL93-2 (889 mg, 1 mmol) was dissolved in DMF and 1-bromododecane (747 mg, 3 mmol) and K₂CO₃ (414 mg, 3 mmol) were added separately to react at room temperature overnight. After the reaction was complete, the crude product was obtained by quenching the reaction by adding water and extracting with dichloromethane, washing three times with water, and concentrating under reduced pressure. A liquid compound PL93 (821.3 mg, 73%) was obtained by purification by column chromatography with eluting using a mixture of dichloromethane and methanol at a ratio of 15:1. ¹H NMR (400 MHz, CDCl₃): 5.43-5.35 (m, 12H), 4.30 (t, *J*=8Hz, 4H), 4.21-4.16 (m, 4H), 3.05-2.81 (m, 12H), 2.48-2.35 (m, 20H), 2.13-2.05 (m, 8H), 1.71-1.63 (m, 8H), 1.46-1.28 (m, 56H), 0.93-0.88 (m, 12H)..

### Example 39

### Synthesis of PL94

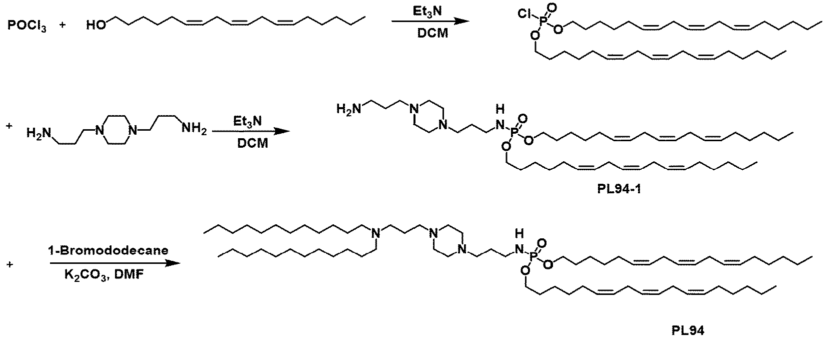

A solution was prepared by dissolving γ-linolenyl alcohol (528 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and reacted at 5°C-20°C for 2 h. Subsequently, the above system was added dropwise to a mixed solution of 1,4-bis(3-aminopropyl) piperazine (400 mg, 2 mmol) and triethylamine (2 eq) in dichloromethane to react for 2 h at room temperature. After the reaction was complete, the crude product was obtained by washing with saturated NaHCO₃ and concentrating under reduced pressure. A liquid compound PL94-1 was obtained by purification by column chromatography with eluting using a mixture of dichloromethane and methanol and ammonia at a ratio of 45:3:1. PL94-1 (773 mg,1 mmol) was dissolved in DMF and 1-bromododecane (747 mg, 3 mmol) and K₂CO₃ (414 mg, 3 mmol) were added separately to react at room temperature overnight. After the reaction was complete, the crude product was obtained by quenching the reaction by adding water and extracting with dichloromethane, washing three times with water, and concentrating under reduced pressure. A liquid compound PL94 (765.2 mg, 69%) was obtained by purification by column chromatography with eluting using a mixture of dichloromethane and methanol at a ratio of 15:1. ¹H NMR (400 MHz, CDCl₃): 5.43-5.34 (m, 12H), 4.06-3.95 (m, 4H), 3.30-2.22 (m, 28H), 2.10-2.06 (m, 8H), 1.73-1.57 (m, 12H), 1.43-1.28 (m, 56H), 0.93-0.88 (m, 12H).

### Example 40

### Synthesis of PL95

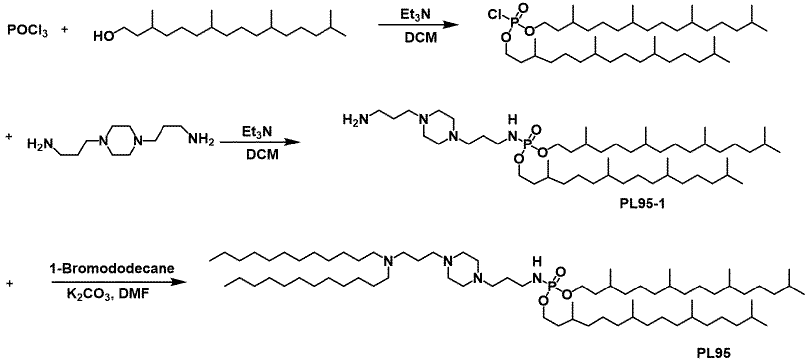

A solution was prepared by dissolving 3,7,11,15-tetramethylhexadecan-1-ol (596 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and reacted at 5°C-20°C for 2 h. Subsequently, the above system was added dropwise to a mixed solution of 1,4-bis(3-aminopropyl) piperazine (400 mg, 2 mmol) and triethylamine (2 eq) in dichloromethane to react for 2 h at room temperature. After the reaction was complete, the crude product was obtained by washing with saturated NaHCO₃ and concentrating under reduced pressure. A liquid compound PL95-1 was obtained by purification by column chromatography with eluting using a mixture of dichloromethane and methanol and ammonia at a ratio of 45:3:1. PL95-1 (840 mg,1 mmol) was dissolved in DMF and 1-bromododecane (747 mg, 3 mmol) and K₂CO₃ (414 mg, 3 mmol) were added separately to react at room temperature overnight. After the reaction was complete, the crude product was obtained by quenching the reaction by adding water and extracting with dichloromethane, washing three times with water, and concentrating under reduced pressure. A liquid compound PL95 (918.06 mg, 78%) was obtained by purification by column chromatography with eluting using a mixture of dichloromethane and methanol at a ratio of 15:1. ¹H NMR (400 MHz, CDCl₃): 4.08-3.98 (m, 4H), 3.04-2.38 (m, 20H), 1.76-1.49 (m, 16H), 1.40-1.05 (m, 76H), 1.10 -0.85 (m, 36H).

### Example 41

### Synthesis of PL96

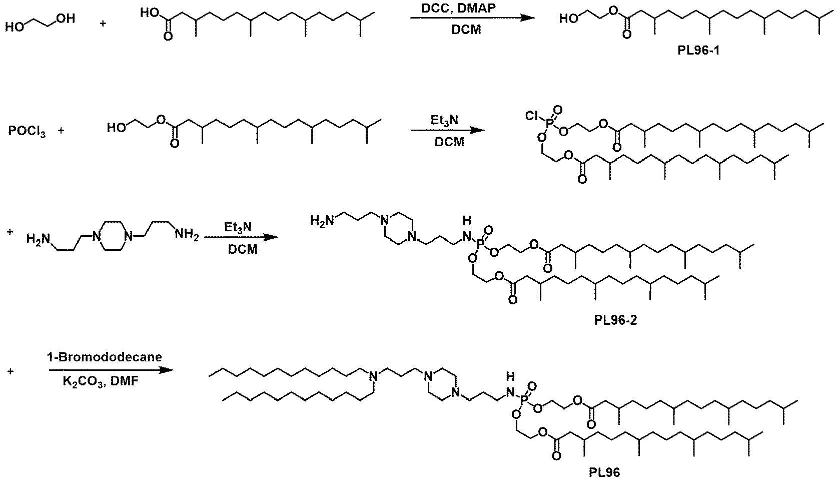

Ethylene glycol (0.62 g, 10 mmol) and phytanic acid (3.12 g, 10 mmol) were first dissolved in 20 mL of DCM, followed by the addition of DCC (2.06 g, 10 mmol) and DMAP (122 mg, 1 mmol) to react at room temperature overnight. After the reaction was complete, a product PL96-1 was obtained by performing filtration, washing with DCM, and concentrating under reduced pressure and then purifying by column chromatography with eluting using a mixture of petroleum ether and ethyl acetate at a ratio of 25:1.

A solution was prepared by dissolving PL96-1 (712 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and reacted at 5°C-20°C for 2 h. Subsequently, the above system was added dropwise to a mixed solution of 1,4-bis(3-aminopropyl) piperazine (400 mg, 2 mmol) and triethylamine (2 eq) in dichloromethane to react for 2 h at room temperature. After the reaction was complete, the crude product was obtained by washing with saturated NaHCO₃ and concentrating under reduced pressure. A liquid compound PL96-2 was obtained by purification by column chromatography with eluting using a mixture of dichloromethane and methanol and ammonia at a ratio of 45:3:1. PL96-2 (957 mg,1 mmol) was dissolved in DMF and 1-bromododecane (747 mg, 3 mmol) and K₂CO₃ (414 mg, 3 mmol) were added separately to react at room temperature overnight. After the reaction was complete, the crude product was obtained by quenching the reaction by adding water and extracting with dichloromethane, washing three times with water, and concentrating under reduced pressure. A liquid compound PL96 (821.3 mg, 73%) was obtained by purification by column chromatography with eluting using a mixture of dichloromethane and methanol at a ratio of 15:1. ¹H NMR (400 MHz, CDCl₃): 4.30 (t, *J*=8Hz, 4H), 4.21-4.17 (m, 4H), 3.05-2.12 (m, 24H), 2.01-1.95 (m, 2H), 1.69-1.43 (m, 10H), 1.40-1.05 (m, 76H), 0.96-0.85 (m, 36H).

### Example 42

### Synthesis of PL97

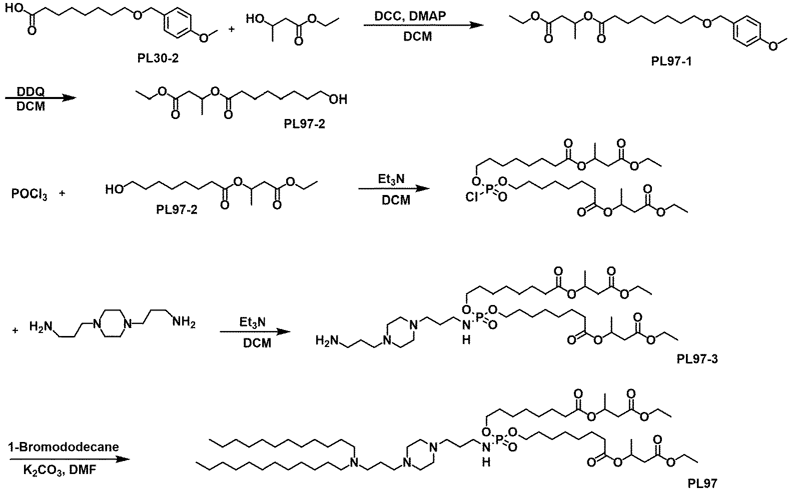

PL30-2 (560 mg, 2 mmol) and ethyl 3-hydroxybutyrate (264 mg, 2 mmol) were dissolved in 10 mL of DCM, followed by the addition of DCC (412 mg, 2 mmol) and DMAP (24 mg, 0.2 mmol) to react at room temperature overnight. After the reaction was complete, a product PL97-1 was obtained by performing filtration, washing with DCM, and concentrating under reduced pressure and purifying by column chromatography with eluting using a mixture of petroleum ether and ethyl acetate at a ratio of 25: 1. A solvent mixture of DCM/H₂O (10 mL/1 mL) was added to PL97-1 (394 mg, 1 mmol), followed by the addition of DDQ (227 mg, 1 mmol) to react for 4 h at room temperature. After the reaction was complete, a product PL97-2 was obtained by adding water and extracting with ethyl acetate, washing for several times with water, and concentrating the organic phase and purifying by column chromatography with eluting using a mixture of petroleum ether and ethyl acetate at a ratio of 25:1. ¹H NMR (400 MHz, CDCl₃): 5.55-5.47 (m, 1H), 4.16(q, J=8Hz 2H), 3.62 (t, J=8Hz, 2H), 2.82-2.61 (m, 2H), 2.35 (t, J=8Hz, 2H), 1.63-1.59 (m, 4H), 1.34-1.21 (m, 12H).

A solution was prepared by dissolving PL97-2 (548 mg, 2 mmol) in 5 mL of DCM and adding 2 mmol of triethylamine, and then the above solution was slowly added dropwise to phosphorous oxychloride (153 mg, 1 mmol) in an ice bath (0°C). The reaction system was protected with nitrogen and reacted at 5°C-20°C for 2 h. Subsequently, the above system was added dropwise to a mixed solution of 1,4-bis(3-aminopropyl) piperazine (400 mg, 2 mmol) and triethylamine (2 eq) in dichloromethane to react for 2 h at room temperature. After the reaction was complete, the crude product was obtained by washing with saturated NaHCO₃ and concentrating under reduced pressure. A liquid compound PL97-3 was obtained by purification by column chromatography with eluting using a mixture of dichloromethane and methanol and ammonia at a ratio of 45:3:1. PL97-3 (792 mg,1 mmol) was dissolved in DMF and 1-bromododecane (747 mg, 3 mmol) and K₂CO₃ (414 mg, 3 mmol) were added separately to react at room temperature overnight. After the reaction was complete, the crude product was obtained by quenching the reaction by adding water and extracting with dichloromethane, washing three times with water, and concentrating under reduced pressure. A liquid compound PL97 (665.43 mg, 55%) was obtained by purification by column chromatography with eluting using a mixture of dichloromethane and methanol at a ratio of 20:1. ¹H NMR (400 MHz, CDCl₃): 5.32-5.29 (m, 4H), 4.16 (q, J=8Hz 4H), 4.01-3.90 (m, 4H), 3.07-2.48 (m, 24H), 2.28 (t, J=8Hz, 4H), 1.79-1.60 (m ,16H), 1.35-1.26 (m, 60H), 0.90 (t, J=8Hz, 6H).

### Example 43

### Synthesis of PL103

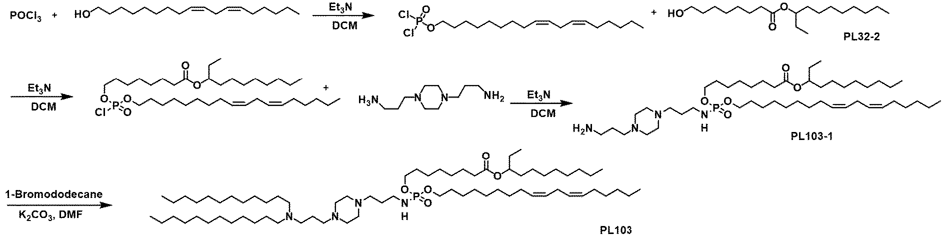

A solution was prepared by dissolving linolenol (266 mg, 1 mmol) in 5 mL of DCM and adding 1 mmol of triethylamine, and then the above solution was slowly added dropwise to phosphorous oxychloride (153 mg, 1 mmol) in an ice bath (0°C). The reaction system was protected with nitrogen and reacted at 0°C-5°C for 2 h. Subsequently, a mixed solution of PL32-2 (314 mg, 1 mmol) and triethylamine (1 eq) in dichloromethane was added dropwise to the system and reacted at 5°C-RT for 2 h. Immediately thereafter, the above system was added dropwise to a mixed solution of 1,4-bis(3-aminopropyl) piperazine (400 mg, 2 mmol) and triethylamine (2 eq) in dichloromethane to react for 2 h at room temperature. After the reaction was complete, the crude product was obtained by washing with saturated NaHCO₃ and concentrating under reduced pressure. A liquid compound PL103-1 was obtained by purification by column chromatography with eluting using a mixture of dichloromethane and methanol and ammonia at a ratio of 50:5:1. PL103-1 (824 mg,1 mmol) was dissolved in DMF and 1-bromododecane (747 mg, 3 mmol) and K₂CO₃ (414 mg, 3 mmol) were added separately to react at room temperature overnight. After the reaction was complete, the crude product was obtained by quenching the reaction by adding water and extracting with dichloromethane, washing three times with water, and concentrating under reduced pressure. A liquid compound PL103 (592.11 mg, 51%) was obtained by purification by column chromatography with eluting using a mixture of dichloromethane and methanol at a ratio of 20:1. ¹H NMR (400 MHz, MeOD): 5.44-5.32 (m, 4H), 4.86-4.80 (m, 1H), 4.00-3.96 (m, 4H), 3.02-2.29 (m, 24H), 2.10-2.04 (m, 4H), 1.70-1.51 (m, 14H), 1.38-1.28 (m, 72H), 0.92-0.87 (m, 18H).

### Example 44

### Preparation of lipid nanoparticles

The prepared cationic compounds (PL1, PL4, PL8, PL10, PL11, PL12, PL13, PL14, PL17, PL18, PL23, PL24, PL26, PL27, PL28, PL29, PL30, PL31, PL32, PL33, PL36, PL38, PL41, PL44, PL45, PL53, PL54, PL55, PL56, PL57, PL58, PL59, PL60, PL61, PL78, PL79, PL93, PL94, PL95, PL96, PL97, PL103), DOPE/DSPC, cholesterol, and DMG-PEG2000 are prepared with ethanol in a molar ratio of 35:16:46.5:2.5 to form the ethanol phase, and RNA is formulated into the aqueous phase with 50 mM citrate buffer solution at pH = 4. Lipid nanoparticles are obtained by microfluidic mixing at aqueous phase: ethanol phase = 3:1 (v/v), followed by dialysis purification in PBS at pH 7.4 overnight to remove ethanol.

### Example 45

### Determination of particle size of lipid nanoparticles

The particle size of the lipid nanoparticles and the distribution (size, PDI) were determined by Malvern Zetasizer Nano ZS laser particle sizer according to dynamic light scattering (DLS). The samples were diluted 100-fold with PBS before measurement, and 1 mL was taken for measurement. The measurement was repeated three times in parallel for each sample.

### Example 46

### Determination of total RNA concentration and encapsulation efficiency of lipid nanoparticles

The total RNA concentration and the encapsulation efficiency were determined using a RiboGreen assay. Determination process included: taking a black 96-well plate, adding 50 µL TE buffer/Triton X to A1-A8/B1-B8, respectively (A1-A6/B1-B6 for the standard curve, A7-8/B7-8 for samples), then adding the RNA TE (Tris-EDTA) dilution solutions (2 ng/µL, 1 ng/µL, 0.5 ng/µL, 0.25 ng/µL, 0.125 ng/µL, 0 ng/µL) to the above wells A1-A6/B1-B6 as the standard curve, adding 50 µL nanoparticle dilution solution to A7-A8/B7-B8, and shaking at 37°C for 15 min, subsequently, adding 100 uL of RiboGreen solution (diluted 400-fold with TE buffer) to each well, and shaking for 3-5 min, and detecting the fluorescence intensity of each well by a microplate reader at an excitation wavelength of 485 nm and an emission wavelength of 515 nm.

### Example 47

### Determination of transfection efficiency of lipid nanoparticles in cells

Determination process included: preparing a 96-well plate, inoculating cells (4000 cells/well), adding 100 µL of medium, incubating at 37°C overnight; after 24 h, adding lipid nanoparticles diluted with medium (achieving the final concentration of RNA of 10 µg/mL), incubating at 37°C for 24 h, and then adding 50 µL of specialized luciferase substrate to each well, followed by detection using by a microplate reader (chemiluminescence).

### Example 48

### Determination of in vivo transfection efficiency of lipid nanoparticles

6 h after intramuscular injection of mRNA LNPs, mice were injected intraperitoneally with 0.2 mL D-fluorescein (20 mg/mL PBS solution). After 10 min, mice were anesthetized with isoflurane in oxygen in a ventilated anesthesia room and imaged with an imaging system (IVIS, PerkinElmer), and luminescence was quantified using Living Image software (PerkinElmer).

### Example 49

### Animal experiments

For obesity prevention study, male or female mice were fed an HFD and injected intraperitoneally once a week with LNPs in phosphate-buffered saline (PBS) (30 mg per mouse). For the obesity treatment study, obesity was induced in male mice after ten weeks of HFD feeding, followed by intraperitoneal injections of LNPs (40 µg mRNA per mouse) every four days for the next six weeks. Weight was monitored weekly and body composition was determined by QMR06-090H before euthanasia.

### Example 50

### Metabolic phenotype

For indirect calorimetry study, after injection of LNPs, an integrated laboratory animal monitoring system (Columbus Instruments) was used to monitor a separate group of male mice from the start of HFD feeding. For a glucose tolerance test (GTT), mice were fasted for 16 hours in clean padded cages and then injected intraperitoneally with glucose (2 g per kg body weight). Blood glucose was measured using an ACCU-CHEK active glucose meter (Roche) at specific time points. For an insulin tolerance test (ITT), mice were fasted for four hours and injected intraperitoneally with insulin (0.75 U per kg body weight).

### Example 51

### Immunocytochemistry

Cells were washed with PBS, fixed with 4% paraformaldehyde (PFA) for 15 min at room temperature and permeabilized with 0.1% Triton-X 100 for 10 min. Cells were blocked in blocking buffer (2% BSA) for 30 min and incubated with primary antibody diluted in the blocking buffer at room temperature for 30 min. The cells were then washed three times with PBS and incubated with the secondary antibody (diluted at 1:500 in blocking buffer) at room temperature for 15 min. After three washes with PBS, the cells were incubated with 4,6-diamidino-2-phenylindole (DAPI; 0.5 µg/ml) for 10 min. Imaging was performed using an an LSM880 confocal microscope (Carl Zeiss).

### Example 52

### In situ determination of pKa by TNS

The apparent pKa of each cationic lipid was determined using the fluorescent probe 2-(p-toluidino)-6-naphthalenesulfonic acid (TNS) and an LNP consisting of cationic lipid/DOPE/cholesterol/PEG-lipid (35:16:35:2.5 mol%) in PBS (total lipid concentration of above 6 mM). TNS was first prepared as a 100 µM stock solution. LNP was diluted to a total lipid concentration of 100 µM with 90 µL of buffer solution (in triplicate). 10 µL of TNS stock solution was added to a LNP solution and mixed thoroughly in a black 96-well plate, and fluorescence intensity was monitored in a Tecan Pro200 plate reader at an excitation wavelength of 321 nm and an emission wavelength of 445 nm. Using the obtained fluorescence values, an s-plot of fluorescence against pH of buffer was created. The logarithm of the inflection point of this curve was the apparent pKa of the LNP.

### Example 53

### Histological evaluation

Immediately after dissection, eWAT, iWAT, and liver were immobilized in 10% formalin buffer solution. After fixation and dehydration, tissues were embedded in paraffin, stained with antibodies against F4/80 (CST, #70076), hematoxylin-eosin (H&E), or periodate-Schiff, and photographed under a microscope (Olympus IX71).

### Example 54

### Determination of concentrations of total triglyceride (TG) and total cholesterol (TC) in blood

After incubation of the plasma for 1 h at room temperature, the supernatant was obtained by centrifuging at 3,500 rpm and 4°C for 15 min. TG and TC were measured by a triglyceride assay kit and a total cholesterol assay kit (Nanjing Jiancheng Bioengineering Institute Co., Ltd.).

### Experimental Results

For compounds PL1, PL4, PL6, PL8, PL10, PL11, PL12, lipid nanoparticles are prepared by DOPE and DSPC, respectively, according to an ethanol injection method (Example 44). The transfection efficiency of these two groups of lipid nanoparticles in cells is first evaluated, and murine fibroblasts NIH-3T3 are selected for incubation (Example 47), and the results are shown in FIG. 1. FIG. 1A shows the DOPE group and FIG. 1B shows the DSPC group, indicating that the transfection efficiency of the addition of DOPE is significantly better than that of the addition of DSPC. In addition, as can be seen from FIG. 1A, PL1, PL10, and PL12 exhibit excellent transfection efficiency and are much higher than a control group MC3, whereas as can be seen from FIG. 1B, the transfection efficiency of PL11 and PL12 is not as good as that of the DOPE group, but the transfection efficiency is still better than that of the control group MC3. The experimental results, therefore, demonstrate that PL lipid nanoparticles have excellent cell transfection efficiency, in which PL1, PL10 and PL12 of the DOPE group and PL11 and PL12 of the DSPC group have good effects, all of which are significantly better than MC3 LNP, which are 16-fold, 22-fold, 7-fold and 3-fold and 1.5-fold that of MC3 LNP, respectively. PL10, PL11, and PL12 all include two tertiary amino groups.

PL1, PL10, and PL12 from the DOPE group, and PL11 and PL12 from the DSPC group are selected to further explore the transfection efficiency in human hepatocellular carcinoma HepG2 cells. LNP is first prepared using microfluidic approach (Example 44) and the encapsulation efficiency of LNP is measured (Example 46), and the results are shown in FIG. 2A. The encapsulation efficiency of PL12 (DOPE), PL11 (DSPC), and PL12 (DSPC) are all above 70%, and the encapsulation efficiency of PL10 (DOPE) is around 50%, indicating that all have good encapsulation efficiency and may be used for RNA encapsulation. Immediately following cell transfection experiments (Example 47), the results are shown in FIG. 2B, which shows excellent transfection efficiency in HepG2 cells. PL10 has the best effect, which is 400-fold of that of LNPs in the control group MC3. The experiment also further illustrates that PL lipids have excellent cell transfection efficiency and stable effect in different cell lines.

According to the above excellent cell transfection efficiency, to further improve the encapsulation efficiency of PL10 and the transfection efficiency in vitro and in vivo, the effect of a ratio of ionizable lipids to RNA on the encapsulation efficiency and the transfection efficiency are further explored. Specific operations include adjusting weight ratios of the cationic PL lipid/RNA (5:1, 10:1, and 15:1), and determining the encapsulation efficiency and the particle sizes. The results are shown in FIG. 3. The encapsulation efficiency is further increased with the addition of more lipids, from more than 30% to about 70%. The particle size is the smallest when the weight ratio of lipid nanoparticle/RNA is 15: 1, and the particle size is from 200 nm to 150 nm when the weight ratio of lipid nanoparticle/RNA is from 5:1 to 15:1. This is also consistent with the literature report that with the elevation of the ratio of N/P, the particle sizes of lipid nanoparticles become smaller and the encapsulation efficiency increases.

The transfection efficiency of PL10 lipid nanoparticles with different N/P ratios in vivo is further explored, and the results are as shown in FIG.4. It shows that there is obvious fluorescent signals when the weight ratios of lipid nanoparticles/RNA are 5:1 and 10:1, which is 3-fold and 10-fold of expression of MC3 LNP, respectively. The fluorescent signal is the strongest when the weight ratio of cationic lipid/RNA is 10:1, while there is no fluorescence signal when the weight ratio of cationic lipid/RNA is 15:1. It indicates that the higher the N/P ratio, the higher the nanoparticle encapsulation efficiency, and the smaller the particle size, indicating PL10 lipid nanoparticles have better RNA encapsulation ability, but are unfavorable to the release of RNA, thus affecting the transfection efficiency of RNA. When the weight ratio of lipid nanoparticle/RNA is 5:1, the encapsulation efficiency is low, which cannot encapsulate RNA well, affecting the transfection later. Therefore, at the weight ratio of lipid nanoparticles/RNA of 10:1, it achieves a kind of equilibrium of RNA encapsulation and release, which may realize a better transfection efficiency.

According to the results of the above experiments, PL1, PL10, PL12, PL13, PL14, PL17, PL18, and PL23, PL24, PL26, PL27, PL28, PL29, PL30, PL31, PL32, PL33, L41, PL44, PL45, PL53, PL54, PL55, PL56, PL57, PL58, PL103 in the DOPE group, and PL11, PL12, and MC3 in the DSPC group are prepared at a weight ratio of lipid nanoparticle/RNA of 10:1, and further explored for transfection in vivo. Microfluidic control is used to prepare the LNP, and the results are as shown in FIG. 5. It shows that the LNP has excellent transfection efficiency in vivo. It can be seen that PL1, PL10, PL11, PL12, PL13, PL14, PL17, PL18, PL23, PL24, PL26, PL27, PL28, PL29, PL30, PL31, PL32, PL33, PL41, PL53, PL54, PL55, PL56, PL57, PL58, and PL103 are all expressed. PL10, PL14, PL24, PL26, PL29, PL30, PL31, PL53, PL55, PL56, and PL57 in the DOPE group have better effects, and their expression in vivo is better than that of control MC3. Especially, PL14, PL53, PL55, PL56, and PL57 have an order of magnitude enhancement with respect to control MC3, while the expression level of PL11 in DOPE group is close to that of MC3. The above 27 groups are all phospholipid structures including two tertiary amino groups, demonstrating that PL-associated lipids including two or more tertiary aminos have excellent in vivo transfection efficiency, which can be used for efficient in vivo delivery of RNA.

Subsequently, the physical properties of lipid nanoparticles PL10, PL14, PL17, PL18, PL23, PL56, and PL60 are evaluated (as shown in FIG. 6). It can be seen that the particle size tends to become smaller as more tertiary aminos are added, and the particle sizes of PL LNPs including a plurality of tertiary aminos are all less than 200 nm, and the zeta potential first becomes negative and then tends to be neutral, and pKa is at 5.5-7.0, indicating that protonation can occur in the acidic environment of lysosomes. In addition, most of the lipid encapsulation rate is around 60%, which may encapsulate mRNA better. In the plasma stability assay, it can be found that the cationic lipids including the plurality of tertiary aminos have better plasma stability, indicating that these lipids may be further applied to in vivo experiments.

In addition, the feasibility of local intra-articular administration of PL lipids is evaluated, and the initially optimized PL10, PL14, and PL17 are selected for local intra-articular administration. The PL14 LNPs have higher and more long-lasting expression efficiencies compared to both PL10 and PL17 LNPs (FIG.7). However, PL14 LNPs are able to be distributed and expressed non-specifically in the liver in addition to localized protein expression. A plurality of administrations may induce hepatotoxicity. Therefore, the formulation prescription is optimized on the basis of PL14 DOPE LNPs by adjusting an oil-to-water ratio, a PEG ratio, a N/P ratio, and a concentration of a buffer solution. When decreasing the proportion of PEG, and increasing the volume of the aqueous phase, it is possible to tune the nanoparticle particle size to around 200 nm, which in turn reduces expression in the liver and enhances the localized expression time of PL14 LNPs (FIG.8). Furthermore, it is verified that the optimized formulation can be expressed in the joint cavity and the concentration does not affect the expression of LNP. Finally, an optimized WG-PL14 prescription is compared to a commercial MC3 prescription. The optimized formulation and prescription are able to enhance the concentration and duration of mRNA in local expression, which is about 30-fold relative to the MC3 prescription (FIG.9).

Targeting and expression of PL lipids on adipose tissue after intraperitoneal administration is also evaluated, enrichment in adipose tissue can be enhanced by altering the structure of the lipids. The PL lipid nanoparticles including a plurality of tertiary amino group structures are significantly fat-enriched and greatly reduced the expression in the liver (FIG.10). At the same time, expression of PL lipid nanoparticles with the plurality of tertiary amino groups in obese mice is also validated (FIG.11). the results show that the PL lipid nanoparticles with the plurality of tertiary amino groups have the effect of lowering the adipose tissue content, reducing the body weight of the obese mice, and improving the metabolism of the obese mice (FIG.12).

Finally, the expression levels of lipid nanoparticles with a plurality of tertiary amine groups from the application after intramuscular injection, white adipocyte administration, and intraperitoneal injection are summarized, and compared with lipid nanoparticles prepared from commercially available cationic lipids MC3 and SM102. The intramuscular injection results shows that the lipid nanoparticles with multiple tertiary amine groups is basically better than that of MC3 and is more than twice as high. SM102 lipids are the positive lipids with the highest reported clinical expression, and its expression in most cells and tissues is at least ten-fold higher than that of MC3. However, the expression rates of the phosphatidylamine lipids with a plurality of tertiary amine groups from embodiments of the present disclosure are higher than that of SM102. The white adipocyte administration results show that the expression of PL17, PL20, PL21, PL29, PL53, PL54, PL55, PL56, PL57, PL59, PL78, and PL79 is superior to that of the control SM102. The intraperitoneal injection results show that the expression of PL20, PL54, PL55, PL56, PL57, PL78, PL93, PL94, and PL95 is superior to that of the control SM102.

### Summary of Lipid Expression

| Compound in the present disclosure | Intramuscular injection (PL/MC3) | White adipocyte administration (PL/SM102) | Intraperitoneal injection (PL/SM102) | Summary of the strongest expressions |
|---|---|---|---|---|
| PL10 | A | C | D | A |
| PL11 | C | C | | C |
| PL12 | A | D | | A |
| PL13 | A | D | | A |
| PL14 | A | C | | A |
| PL17 | A | B | C | A |
| PL18 | A | C | D | A |
| PL19 | | C | D | C |
| PL20 | | B | B | B |
| PL21 | | B | C | B |
| PL22 | | C | D | C |
| PL23 | A | C | C | A |
| PL24 | A | C | C | A |
| PL26 | A | C | | A |
| PL27 | A | | | A |
| PL28 | A | C | | A |
| PL29 | A | B | | A |
| PL30 | A | D | | A |
| PL31 | A | D | | A |
| PL32 | A | | | A |
| PL33 | A | | | A |
| PL36 | | C | | C |
| PL38 | | C | D | C |
| PL41 | A | C | | A |
| PL44 | C | C | | C |
| PL45 | C | C | | C |
| PL53 | A | B | C | A |
| PL54 | A | B | B | A |
| PL55 | A | B | B | A |
| PL56 | A | B | A | A |
| PL57 | A | B | A | A |
| PL58 | A | C | C | A |
| PL59 | | A | D | A |
| PL60 | | C | D | C |
| PL61 | | C | D | C |
| PL78 | | B | B | B |
| PL79 | | B | C | B |
| PL93 | | | A | A |
| PL94 | | | A | A |
| PL95 | | | A | A |
| PL96 | | | C | C |
| PL97 | | | C | C |
| PL103 | A | | | A |

Instructions of expression strength levels:
NA = not tested;
A: ≥2;
B: ≥1 and <2;
C: ≥0.1 and <1;
D: <0.1.

While the embodiments disclosed in the present disclosure are as described above, what is described is only an embodiment to facilitate the understanding of the present disclosure and is not intended to limit the present application. Any person skilled in the art to which this disclosure belongs, without departing from the spirit and scope of the embodiments disclosed herein, may make any modifications and variations in the form and details of the embodiments. However, the scope of protection of this disclosure shall still be subject to the scope defined in the appended claims.

## Claims

1. A phosphatidylamine compound comprising a plurality of tertiary amino group structures, wherein the phosphatidylamine compound is a compound represented by formula (I) or a stereoisomer, a prodrug, a pharmaceutically acceptable salt thereof: 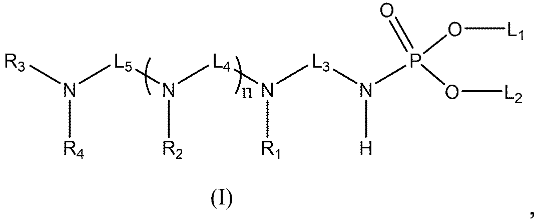
wherein n is an integer from 0-5;
L₁ and L₂ are each independently C₅-C₃₀ alkyl, C₅-C₄₀ alkenyl, or -R'-M-R";
R₁ and R₂ are each independently C₄-C₃₀ alkyl, C₂-C₄₀ alkenyl, or -R'-M-R"; or R₁ and R₂, together with their respective attached N and L₄, form a 5 to 8-membered diazacycloalkylene;
R₃ and R₄ are each independently C₁-C₃₀ alkyl, C₂-C₄₀ alkenyl, or -R'-M-R"; or R₃ is H, C₁-C₃₀ alkyl, C₂-C₄₀ alkenyl, or -R'-M-R", and R₄ is C₁-C₃₀ alkyl, C₂-C₄₀ alkenyl, or -R'-M-R";
R' is C₁-C₂₄ alkylene or C₂-C₂₄ alkenylene;
M is selected from -C(O)-, -C(O)O-, -OC(O)-, -OC(O)O-, -C(S)-, -C(O)S-, -SC(O)-, -C(S)S-, -SC(S)-, -S(O)₂-, -S-S-, -C(O)N(R^{a})-, -N(R^{a})C(O)-, -OC(O)N(R^{a})-, -N(R^{a})C(O)O-, -N(R^{a})C(O)N(R^{a})-, -C(O-C(O)-R^{a})(H)-C₁₋₆ alkylene-O-C(O)-, -C(O)O-C₁₋₆ alkylene-C(O)-O-, -CH(OH)-, -P(O)(OR^{b})O-, C₆-C₁₀ arylene, and 5 to 10-membered heteroarylene ;
R", R^{a} and R^{b} are each independently selected from H, C₁-C₃₀ alkyl, and C₂-C₄₀ alkenyl; and
L₃, L₄, and L₅ are each independently C₁-C₁₂ alkylene, C₂-C₁₂ alkenylene, C₃-C₈ cycloalkylene, -A-Y-B-, -A-Y-, or -Y-B-, wherein A and B are each independently C₁-C₁₂ alkylene, or C₂-C₁₂ alkenylene, and Y is -C(OH)-, -C(O)-, -C(O)O-, -OC(O)-, -C(O)N(H)-, -N(H)C(O)-, arylene, or heteroarylene.

2. The phosphatidylamine compound of claim 1, wherein n is 0 and the compound represented by the formula (I) is a compound represented by formula (I-1): 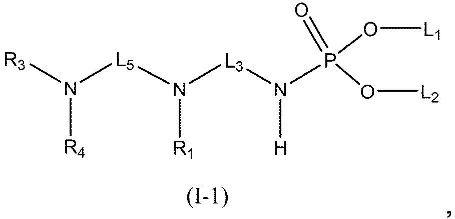
wherein L₁ and L₂ in formula (I-1) are each independently C₅-C₃₀ alkyl, C₅-C₄₀ alkenyl, or -R'-M-R";
R₁ is C₄-C₃₀ alkyl, C₂-C₄₀ alkenyl, or -R'-M-R";
R₃ and R₄ are each independently C₁-C₃₀ alkyl, C₂-C₄₀ alkenyl, or -R'-M-R"; or R₃ is H, C₁-C₃₀ alkyl, C₂-C₄₀ alkenyl, or -R'-M-R", and R₄ is C₁-C₃₀ alkyl, C₂-C₄₀ alkenyl, or -R'-M-R";
R' is C₁-C₂₄ alkylene or C₂-C₂₄ alkenylene;
M is selected from -C(O)-, -C(O)O-, -OC(O)-, -OC(O)O-, -C(S)-, -C(O)S-, -SC(O)-, -C(S)S-, -SC(S)-, -S(O)₂-, -S-S-, -C(O)N(R^{a})-, -N(R^{a})C(O)-, -OC(O)N(R^{a})-, -N(R^{a})C(O)O-, -N(R^{a})C(O)N(R^{a})-, -C(OC(O)R^{a})-C₁₋₆ alkylene-O-C(O)-, -C(O)-C₁₋₆ alkylene-C(O)-O-, -CH(OH)-, -P(O)(OR^{b})O-, C₆-C₁₀ arylene, and 5 to 10-membered heteroarylene;
R", Rₐ and R_{b} are each independently selected from H, C₁-C₃₀ alkyl, and C₂-C₄₀ alkenyl; and
L₃ and L₅ are each independently C₁-C₁₂ alkylene, C₂-C₁₂ alkenylene, C₃-C₈ cycloalkylene, -A-Y-B-, -A-Y-, or -Y-B-, wherein A and B are each independently C₁-C₁₂ alkylene or C₂-C₁₂ alkenylene, and Y is -C(OH)-, -C(O)-, -C(O)O-, -OC(O)-, -C(O)N(H)-, -N(H)C(O)-, arylene , or heteroarylene.

3. The phosphatidylamine compound of claim 1, wherein n is 1 and the compound represented by the formula (I) is a compound represented by formula (I-2): 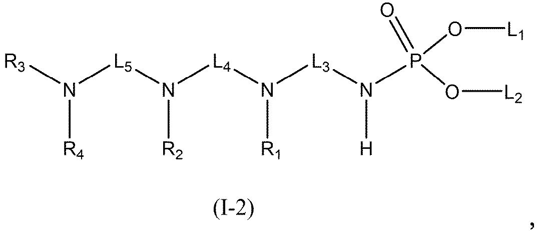
wherein L₁ and L₂ are each independently C₅-C₃₀ alkyl, C₅-C₄₀ alkenyl, or -R'-M-R";
R₁ and R₂, together with their respective attached N and L₄, form a 5 to 8-membered diazacycloalkylene; or R₁ and R₂ are each independently C₄-C₃₀ alkyl, C₂-C₄₀ alkenyl, or -R'-M-R";
L₄ is C₁-C₁₂ alkylene, C₂-C₁₂ alkenylene, C₃-C₈ cycloalkylene, -A-Y-B-, -A-Y-, or -Y-B-, wherein A and B are each independently C₁-C₁₂ alkylene or C₂-C₁₂ alkenylene, and Y is -C(OH)-, -C(O)-, -C(O)O-, -OC(O)-, -C(O)N(H)-, - N(H)C(O)-, arylene, or heteroarylene;
R₃ and R₄ are each independently C₁-C₃₀ alkyl, C₂-C₄₀ alkenyl, or -R'-M-R"; or R₃ is H, C₁-C₃₀ alkyl, C₂-C₄₀ alkenyl, or -R'-M-R", and R₄ is C₁-C₃₀ alkyl, C₂-C₄₀ alkenyl, or -R'-M-R";
R' is C₁-C₂₄ alkylene or C₂-C₂₄ alkenylene;
M is selected from -C(O)-, -C(O)O-, -OC(O)-, -OC(O)O-, -C(S)-, -C(O)S-, -SC(O)-, -C(S)S-, -SC(S)-, -S(O)₂-, -S-S-, -C(O)N(R^{a})-, -N(R^{a})C(O)-, -OC(O)N(R^{a})-, -N(R^{a})C(O)O-, -N(R^{a})C(O)N(R^{a})-, -C(OC(O)R^{a})-C₁₋₆ alkylene-O-C(O)-, -C(O)-C₁₋₆ alkylene-C(O)-O-, -CH(OH)-, -P(O)(OR^{b})O-, C₆-C₁₀ arylene, and 5 to 10-membered heteroarylene ;
R", R^{a}, and R^{b} are each independently selected from H, C₁-C₃₀ alkyl, and C₂-C₄₀ alkenyl; and
L₃ and L₅ are each independently C₁-C₁₂ alkylene, C₂-C₁₂ alkenylene, C₃-C₈ cyclo alkylene, -A-Y-B-, -A-Y-, or -Y-B-, wherein A and B are each independently C₁-C₁₂ alkylene or C₂-C₁₂ alkenylene, and Y is -C(OH)-, -C(O)-, -C(O)O-, -OC(O)-, -C(O)N(H)-, -N(H)C(O)-, arylene, or heteroarylene .

4. The phosphatidylamine compound of any of the claims 1-3, wherein L₁ and L₂ are each independently C₆-C₃₀ alkyl, C₆-C₄₀ alkenyl, or -R'-M-R", wherein R' is C₁-C₂₄ alkylene or C₂-C₂₄ alkenylene, M is selected from -C(O)-, -C(O)O -, -OC(O)-, -OC(O)O-, -C(S)-, -C(O)S-, -SC(O)-, -C(S)S-, -SC(S)-, -S(O)₂-, -S-S-, -C(O)N(R^{a})-, -N(R^{a})C(O)-, -OC(O)N(R^{a})-, -N(R^{a})C(O)O-, -N(R^{a})C(O)N(R^{a})-, -C(OC(O)R^{a})-CH₂-O-C(O)-, -C(O)O-CH(CH₃)CH₂-C(O)-O-, -CH(OH)-, -P(O)(OR^{b})O-, C₆-C₁₀ arylene, and 5 to 10-membered heteroarylene, R" is C₁-C₃₀ alkyl or C₂-C₄₀ alkenyl, and R^{a} and R^{b} are each independently selected from H, C₁-C₃₀ alkyl, and C₂-C₄₀ alkenyl; or L₁ and L₂ are each independently C₆-C₃₀ alkyl, C₆-C₄₀ alkenyl, or -R'-M-R", wherein R' is C₁-C₂₄ alkylene or C₂-C₂₄ alkenylene, M is -C(O)-, -C(O)O-, -OC(O)-, -C(O)N(H)-, -N(H)C(O)-, -C(OC(O)R^{a})-CH₂-O-C(O)-, -C(O)O-CH(CH₃)CH₂-C(O)-O-, or -CH(OH)-, and R^{a} or R" is C₁-C₃₀ alkyl or C₂-C₄₀ alkenyl; or
L₁ and L₂ are each independently one of the following structures: 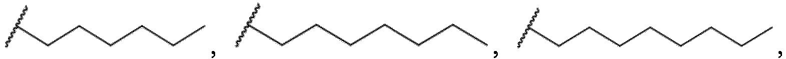 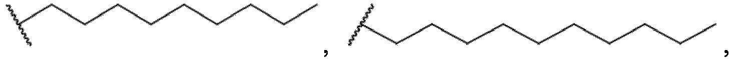 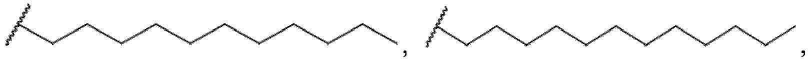 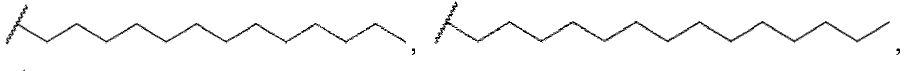 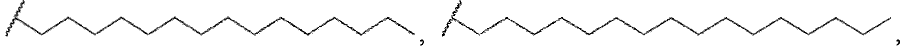 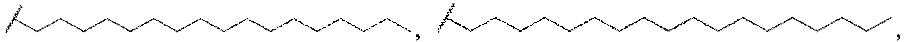 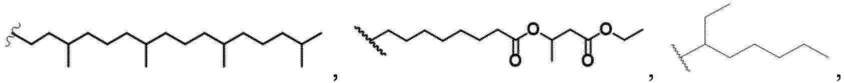 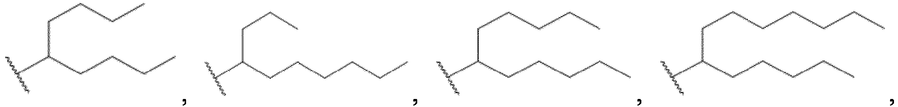 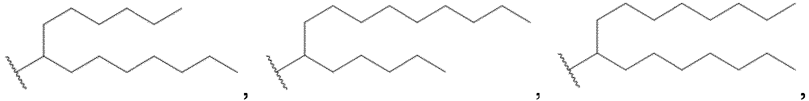 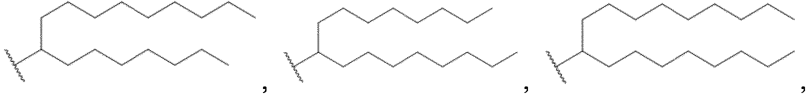 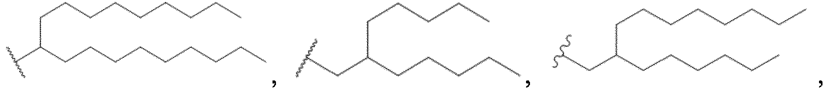 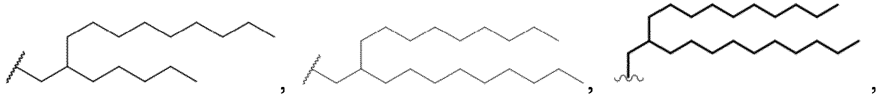 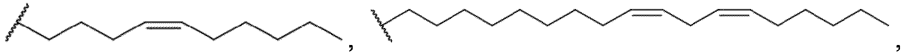 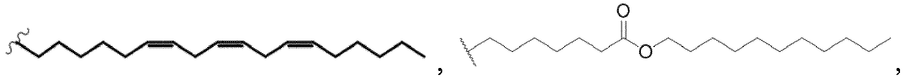 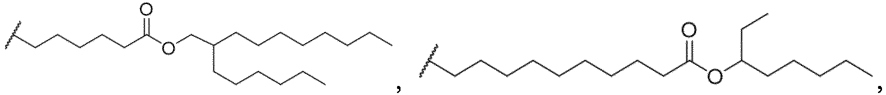 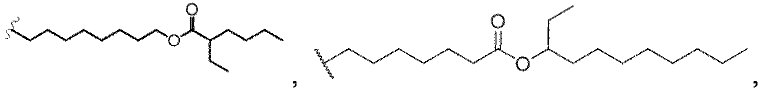 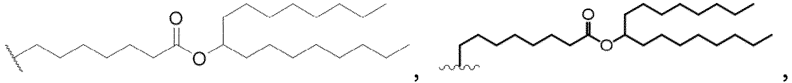 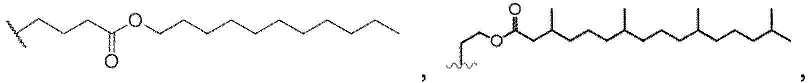 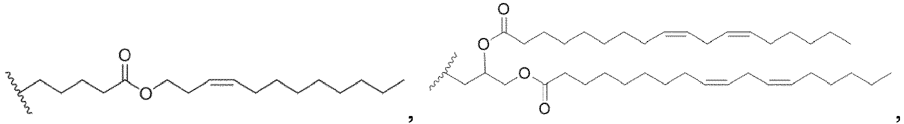 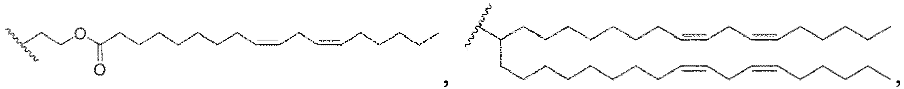 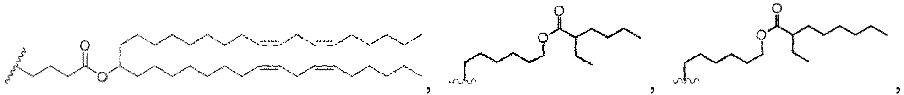 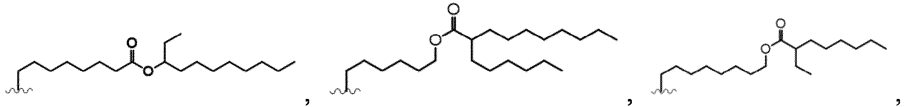 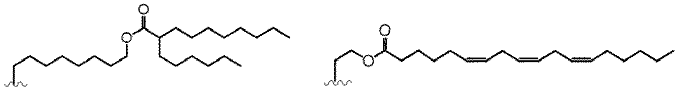 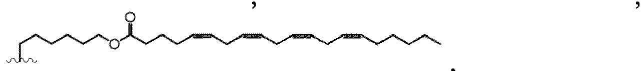 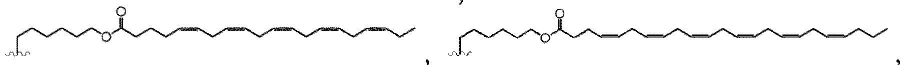 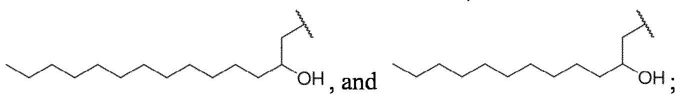
L₁ and L₂ are each independently one of the following structures: 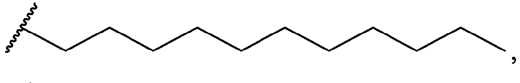 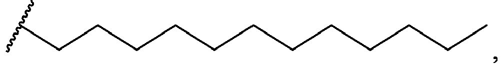 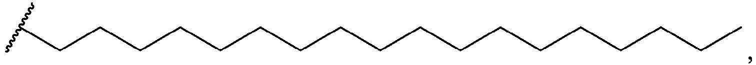 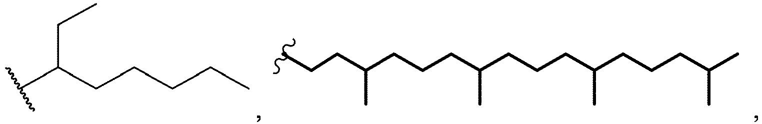 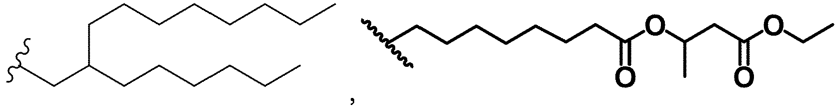 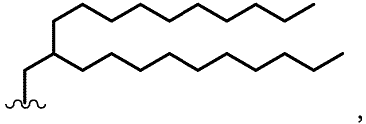 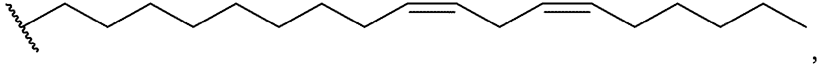 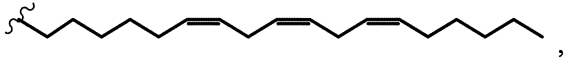 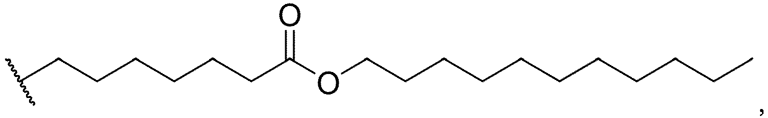 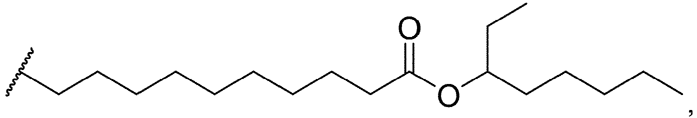 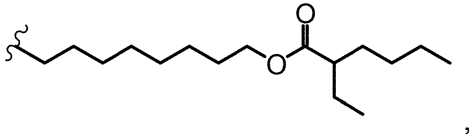 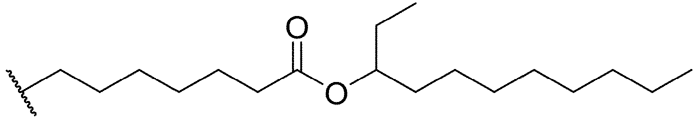 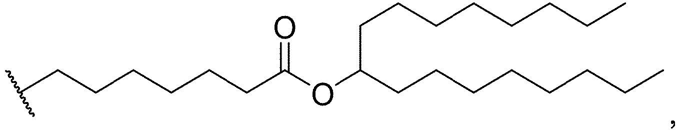 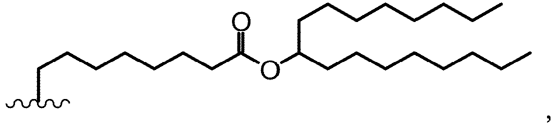 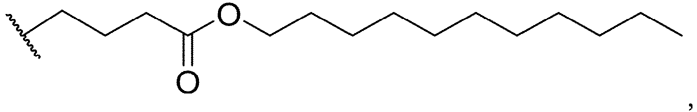 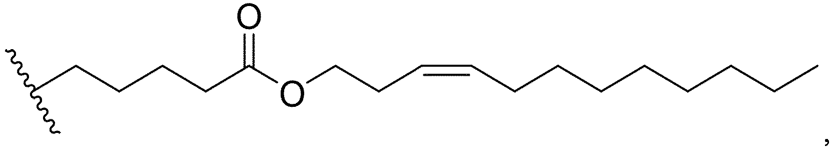 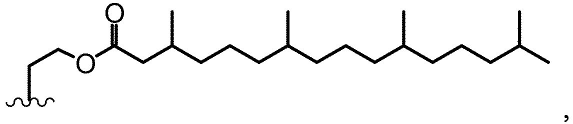 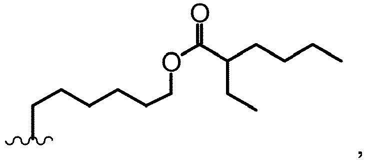 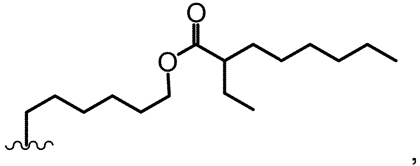 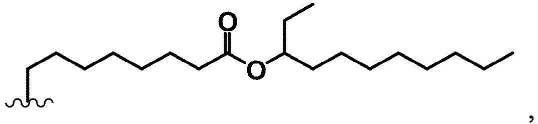 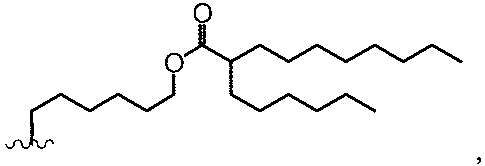 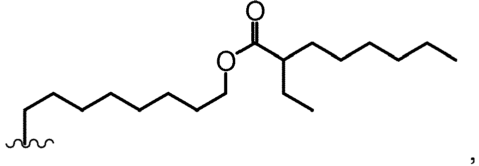 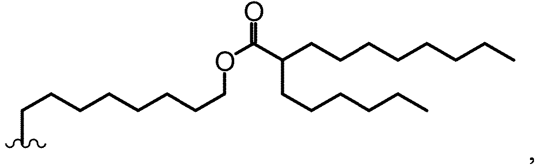 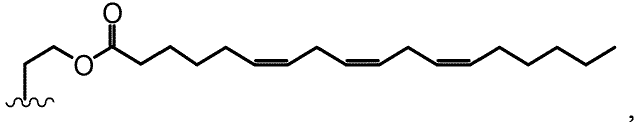 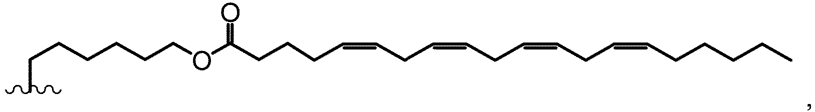 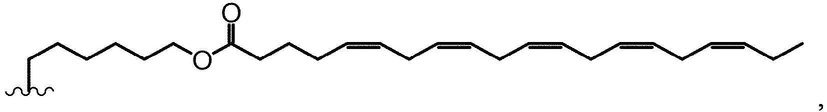 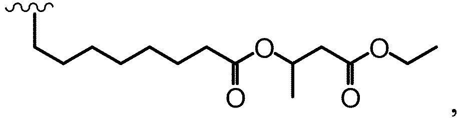 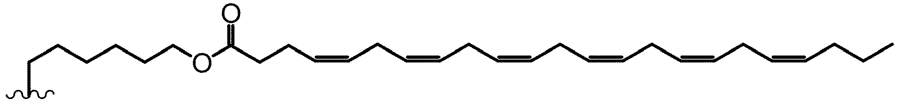
and

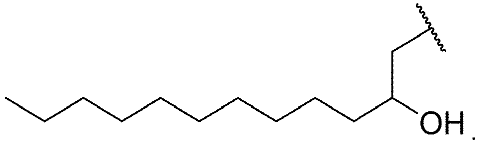

5. The phosphatidylamine compound of any of the claims 1-4, wherein R₁ is one of following structures, or when R₂ is present, R₁ and R₂ are each independently one of the following structures: 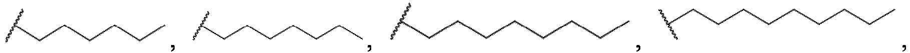 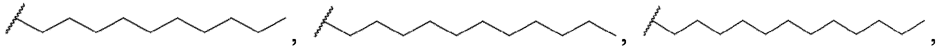 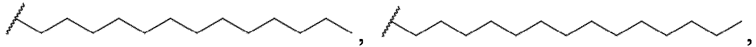 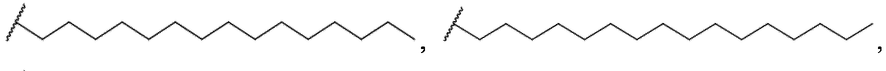 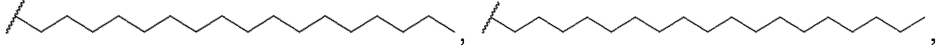 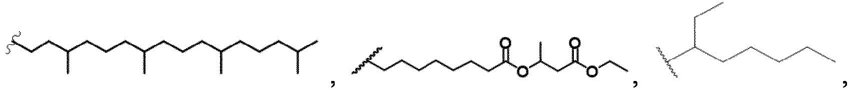 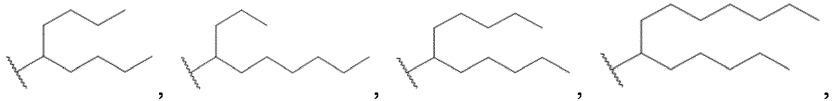 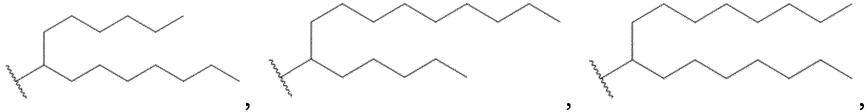 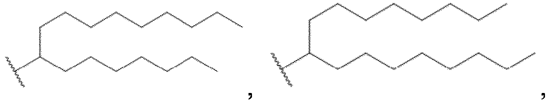 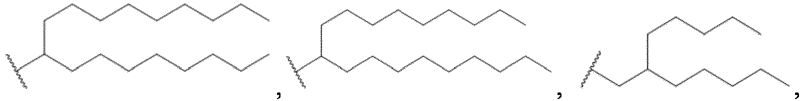 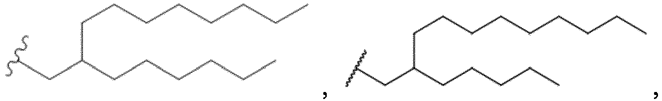 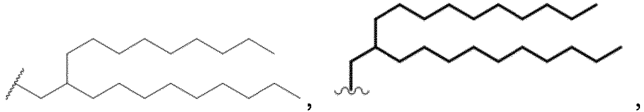 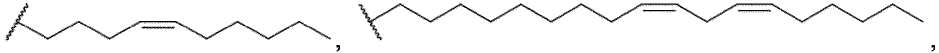 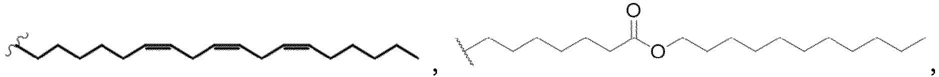 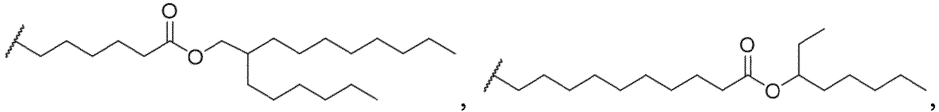 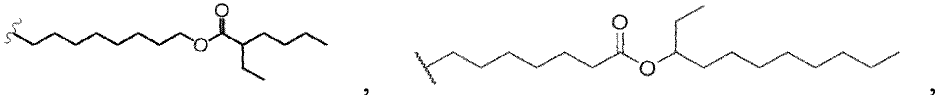 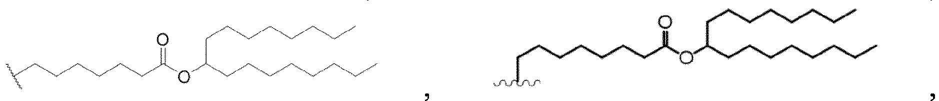 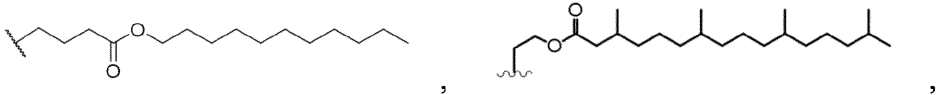 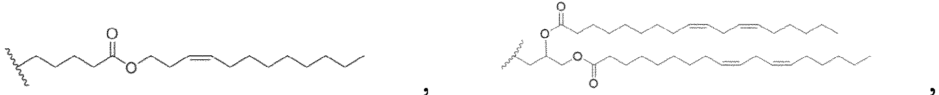 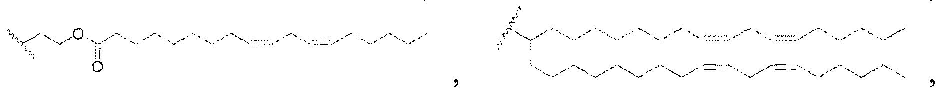 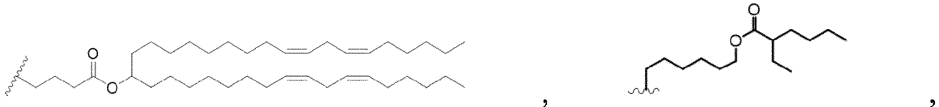 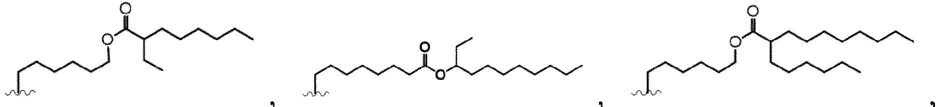 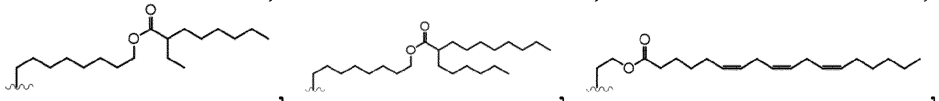 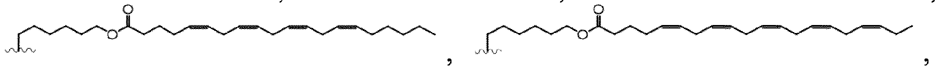 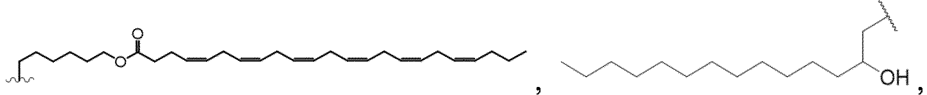 and 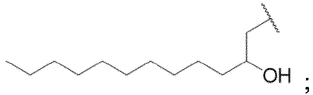 or R₁ and R₂ are each independently one of the following structures

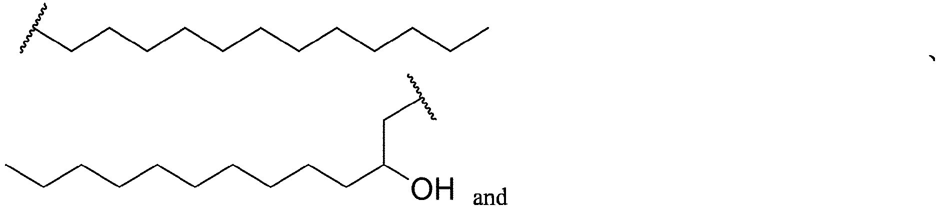

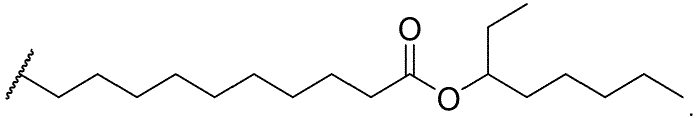

6. The phosphatidylamine compound of any of the claims 1, 3-4, wherein R₁ and R₂, together with their respective attached N and L₄, form one of the following groups that is optionally substituted: 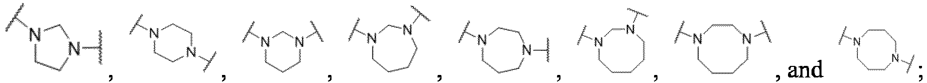 or
R₁ and R₂, together with their respective attached N and L₄, form the following group that is optionally substituted:

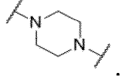

7. The phosphatidylamine compound of any of the claims 1-6, wherein L₃ and L₅ or L₃, L₄, and L₅ (when L₄ is present) are each independently methylene, ethylene, propylene, butylene, pentylene, hexylene, vinylidene, propenylene, butenylene, pentenylene, hexenylene, cyclopropylene, cyclobutylene, cyclopentylene, cyclohexylene, cycloheptylene, -A-Y-B-, -A-Y-, or -Y-B-, wherein A and B are each independently methylene, ethylene, propylene, butylene, pentylene, hexylene, vinylidene, propenylene, butenylene, pentenylene, or hexenylene, and Y is -C(OH)-, -C(O)-, -C(O)O-, -OC(O)-, -C(O)N(H)-, -N(H)C(O)-, phenylene, pyrroloylene, imidazolylene, thiazolylene, thienylene, furylene, pyridylene, or pyrimidylene; or
L₃ and L₅ or L₃, L₄, and L₅ (when L₄ is present) are each independently -CH₂-, -CH₂CH₂-, -CH(CH₃)CH₂-, -CH₂CH(CH₃)-, -CH₂CH₂CH₂-, -CH(CH₃)CH₂CH₂-, -CH₂CH(CH₃)CH₂-, -CH₂CH₂CH(CH₃)-, -CH₂CH₂CH₂CH₂-, -CH(CH₃)CH₂CH₂CH₂-, -CH₂CH(CH₃)CH₂CH₂-, -CH₂CH₂CH₂CH(CH₃)-, -A-Y-B-, -A-Y-, or -Y-B-, wherein A and B are each independently -CH₂-, -CH₂CH₂-, -CH(CH₃)CH₂-, -CH₂CH(CH₃)-, -CH₂CH₂CH₂-, -CH(CH₃)CH₂CH₂-, -CH₂CH(CH₃)CH₂-, or -CH₂CH₂CH(CH₃)-, and Y is -C(OH)-, -C(O)-, -C(O)O-, -OC(O)-, -C(O)N(H)-, -N(H)C(O)-, or phenylene; or
L₃ is -CH₂-, -CH₂CH₂-, -CH(CH₃)CH₂-, -CH₂CH(CH₃)-, -CH₂CH₂CH₂-, -CH(CH₃)CH₂CH₂-, -CH₂CH(CH₃)CH₂-, -CH₂CH₂CH(CH₃)-, -CH₂CH₂CH₂CH₂-, -CH(CH₃)CH₂CH₂CH₂-, -CH₂CH(CH₃)CH₂CH₂-, -CH₂CH₂CH₂CH(CH₃)-, -A-Y-B-,-A-Y-, or -Y-B-, wherein A and B are each independently -CH₂-, -CH₂CH₂-, -CH(CH₃)CH₂-, -CH₂CH(CH₃)-, -CH₂CH₂CH₂-, -CH(CH₃)CH₂CH₂-, -CH₂CH(CH₃)CH₂-, or -CH₂CH₂CH(CH₃)-, Y is -C(OH)-, -C(O)-, -C(O)O-, -OC(O)-, -C(O)N(H)-, or -N(H)C(O)-; or
L₃ is -CH₂CH₂-,-CH₂CH₂CH₂-, or
L₄ is-CH₂-, -CH₂CH₂-, -CH(CH₃)CH₂-, -CH₂CH(CH₃)-, -CH₂CH₂CH₂-, -CH(CH₃)CH₂CH₂-, -CH₂CH(CH₃)CH₂-, -CH₂CH₂CH(CH₃)-, -CH₂CH₂CH₂CH₂-, -CH(CH₃)CH₂CH₂CH₂-, -CH₂CH(CH₃)CH₂CH₂-, -CH₂CH₂CH₂CH(CH₃)-, -A-Y-B-, -A-Y-, or -Y-B-, wherein A and B are each independently -CH₂-, -CH₂CH₂-, -CH(CH₃)CH₂-, -CH₂CH(CH₃)-, -CH₂CH₂CH₂-, -CH(CH₃)CH₂CH₂-, -CH₂CH(CH₃)CH₂-, or -CH₂CH₂CH(CH₃)-, Y is-C(OH)-, -C(O)-, -C(O)O-, -OC(O)-, -C(O)N(H)-, or -N(H)C(O)-; or,
L₄ is -CH₂CH₂-,-CH₂CH₂CH₂-, or
L₅ is -CH₂-, -CH₂CH₂-, -CH(CH₃)CH₂-, -CH₂CH(CH₃)-, -CH₂CH₂CH₂-, -CH(CH₃)CH₂CH₂-, -CH₂CH(CH₃)CH₂-, -CH₂CH₂CH(CH₃)-, -CH₂CH₂CH₂CH₂-, -CH(CH₃)CH₂CH₂CH₂-, -CH₂CH(CH₃)CH₂CH₂-, -CH₂CH₂CH₂CH(CH₃)-, -A-Y-B-, -A-Y-, or -Y-B-, wherein A and B are each independently -CH₂-, -CH₂CH₂-, -CH(CH₃)CH₂-, -CH₂CH(CH₃)-, -CH₂CH₂CH₂-, -CH(CH₃)CH₂CH₂-, -CH₂CH(CH₃)CH₂-, or -CH₂CH₂CH(CH₃)-, Y is -C(OH)-, -C(O)-, -C(O)O-, -OC(O)-, -C(O)N(H)-, -N(H)C(O)-, or phenylene; or
L₅ is -CH₂CH₂-, -CH₂CH₂CH₂-, -CH₂CH₂-C(O)O-CH₂CH₂CH₂-, -CH₂CH₂-C(O)N(H)-CH₂CH₂CH₂-, -CH₂CH(OH)CH₂-, or

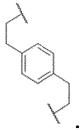

8. The phosphatidylamine compound of claim 1, wherein
n is 0;
R₁, R₃, R₄, L₁, and L₂ are each independently one of following structures: 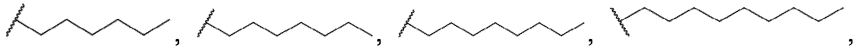 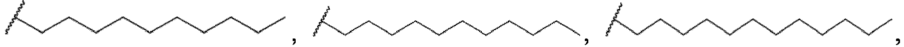 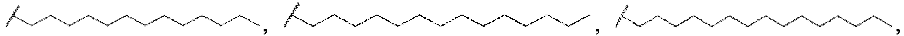 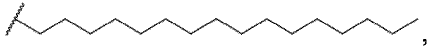 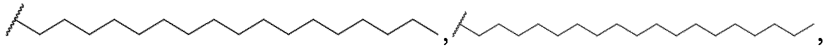 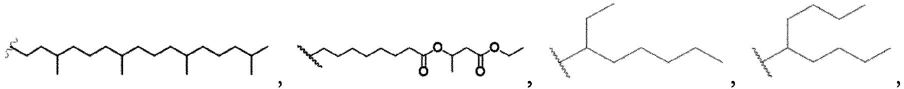 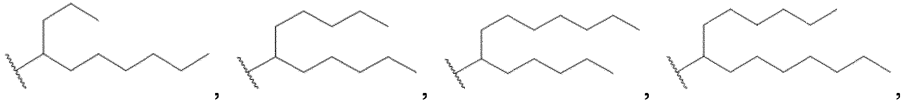 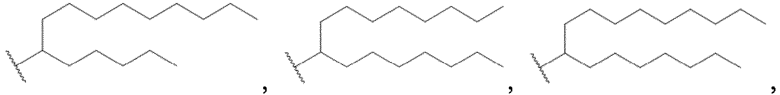 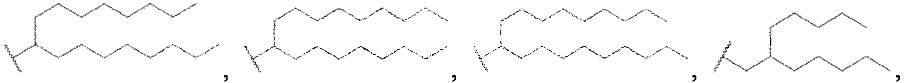 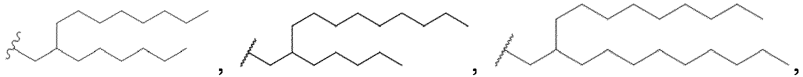 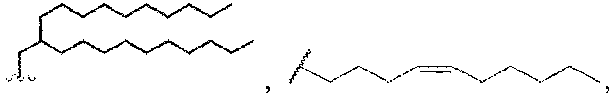 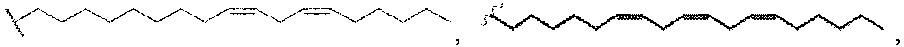 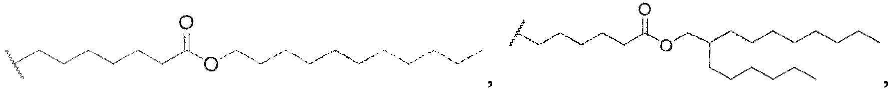 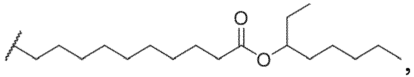 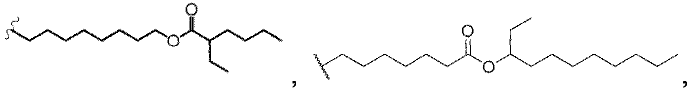 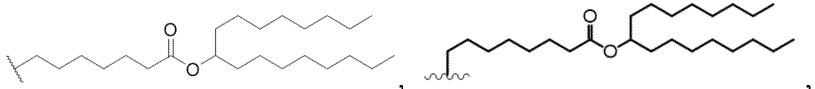 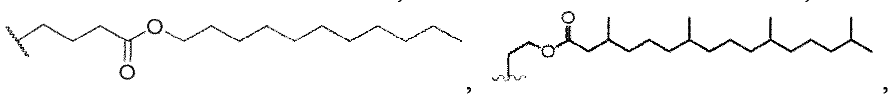 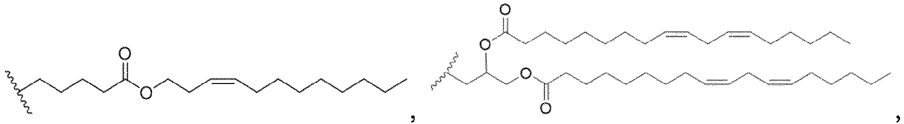 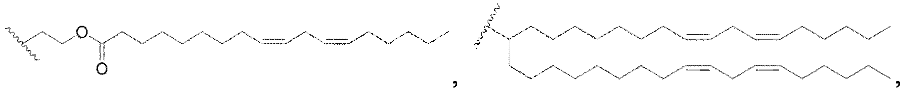 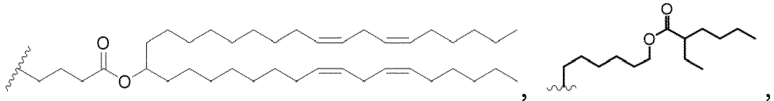 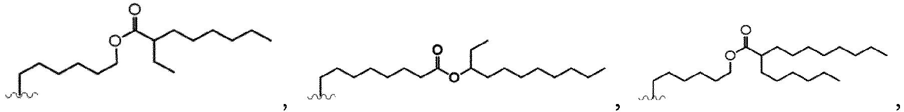 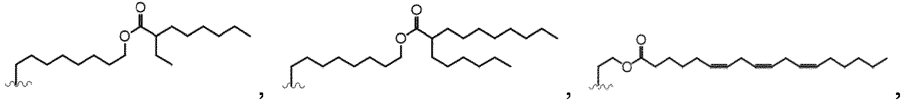 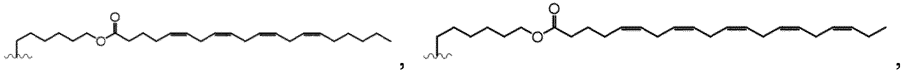 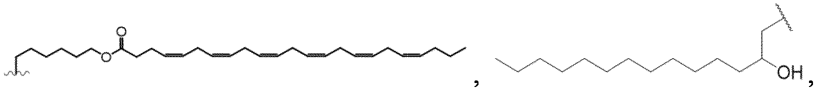 and 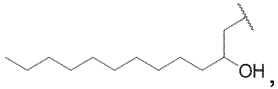 or R₃ and R₄ are both methyl and R₁, L₁ and L₂ are defined by the above structures;
L₃ is -CH₂CH₂- or -CH₂CH₂CH₂-; and
L₅ is -CH₂CH₂-, -CH₂CH₂CH₂-, -CH₂CH₂-C(O)O-CH₂CH₂CH₂-, -CH₂CH₂-C(O)N(H)-CH₂CH₂CH₂-, -CH₂CH(OH)CH₂-, or

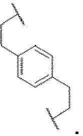

9. The phosphatidylamine compound of claim 1, wherein n is 1; R₁ and R₂, together with their respective attached N and L₄, form the following group that is optionally substituted: 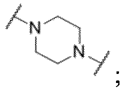
L₃ and L₄ are independently -CH₂CH₂- or -CH₂CH₂CH₂-;
L₅ is -CH₂CH₂-, -CH₂CH₂CH₂-, -CH₂CH₂-C(O)O-CH₂CH₂CH₂-, -CH₂CH₂-C(O)N(H)-CH₂CH₂CH₂-, or -CH₂CH(OH)CH₂-, and
R₃, R₄, L₁, and L₂ are each independently one of following structures:

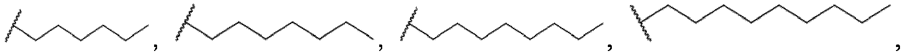

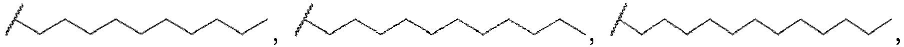

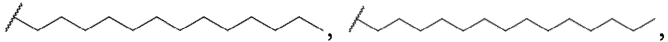

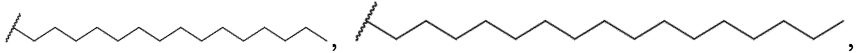

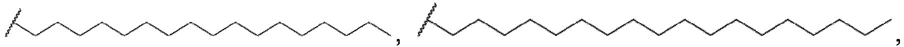

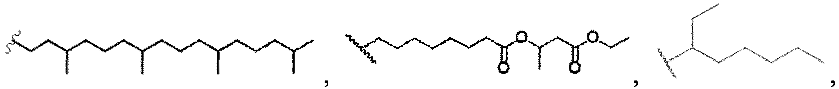

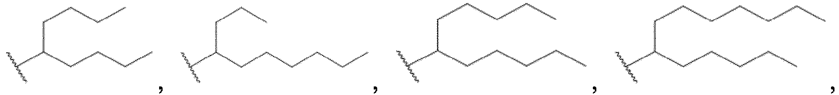

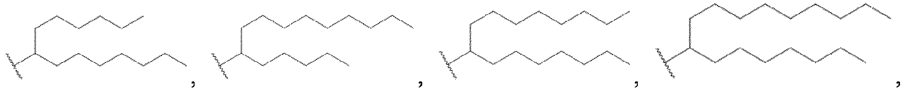

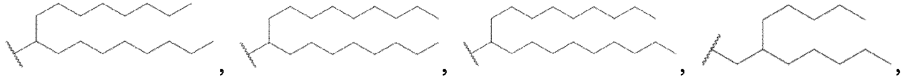

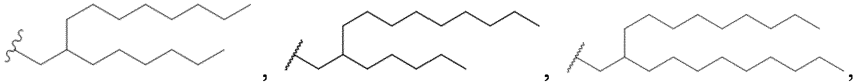

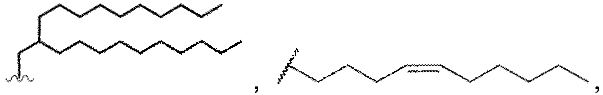

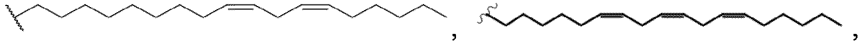

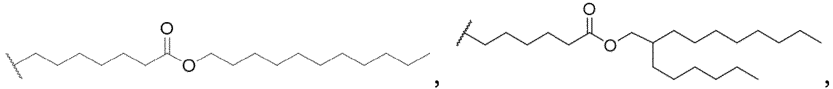

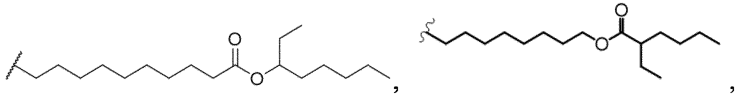

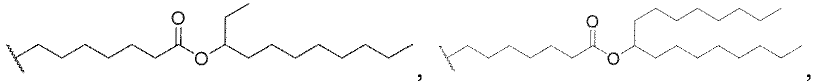

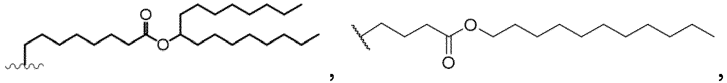

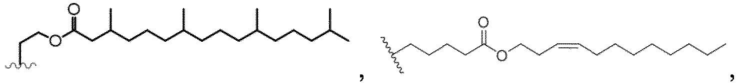

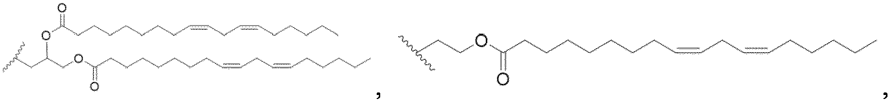

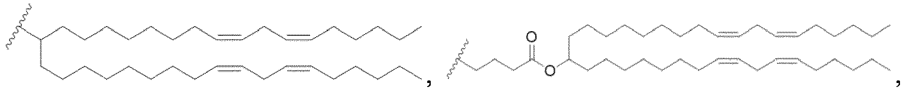

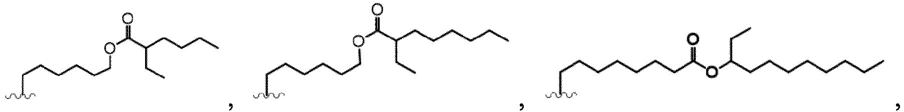

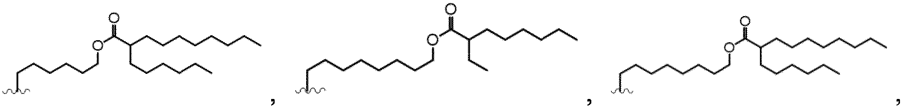

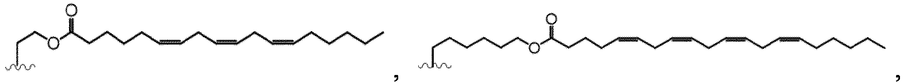

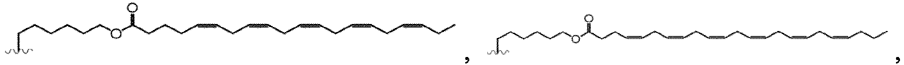

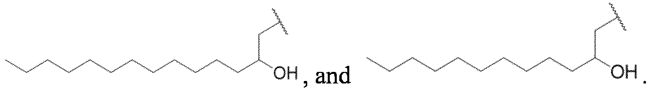

10. The phosphatidylamine compound of claim 1, wherein n is 1;
L₃ and L₄ are independently -CH₂CH₂- or -CH₂CH₂CH₂-;
L₅ is -CH₂CH₂-, -CH₂CH₂CH₂-, -CH₂CH₂-C(O)O-CH₂CH₂CH₂-, -CH₂CH₂-C(O)N(H)-CH₂CH₂CH₂-, or -CH₂CH(OH)CH₂-, and
R₁, R₂, R₃, R₄, L₁, and L₂ are each independently one of following structures:

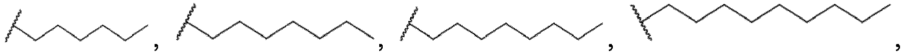

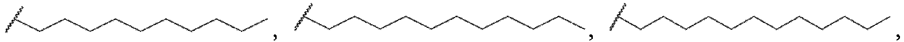

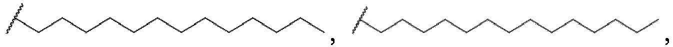

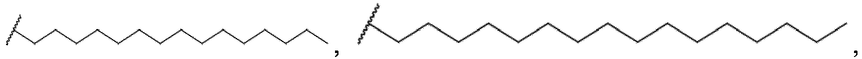

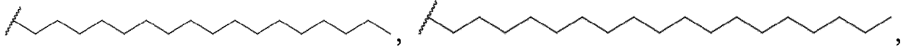

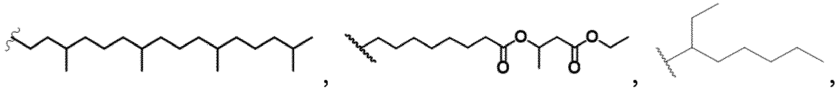

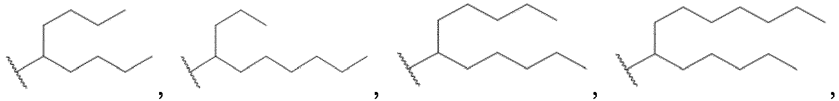

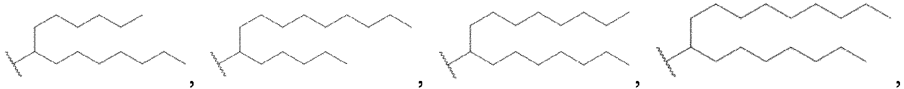

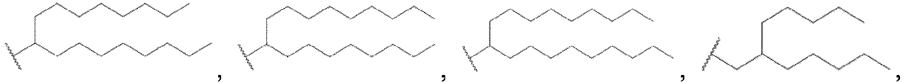

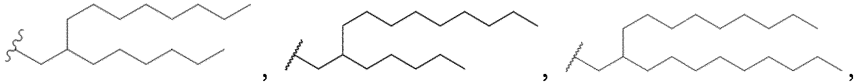

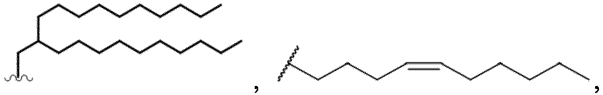

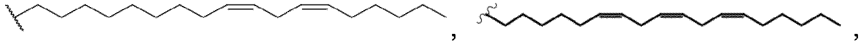

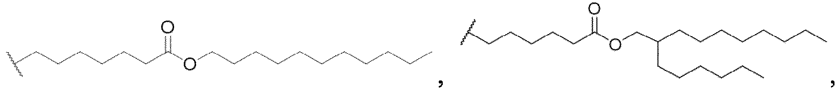

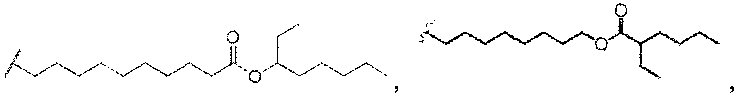

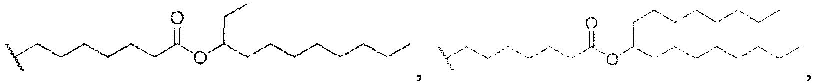

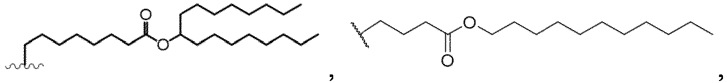

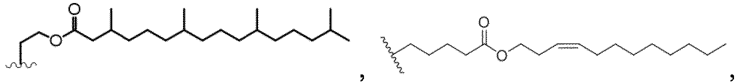

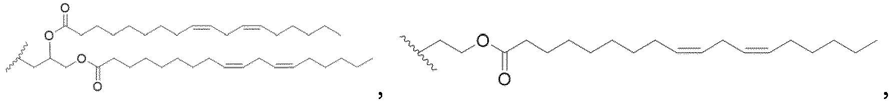

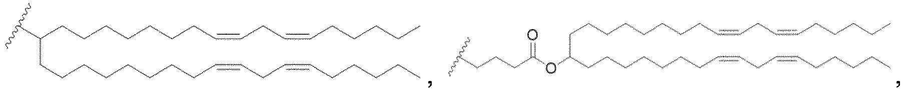

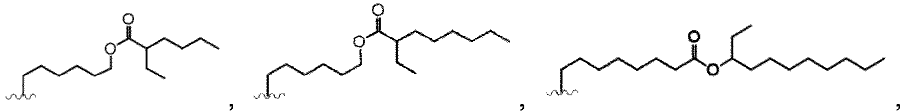

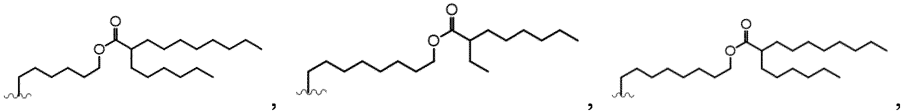

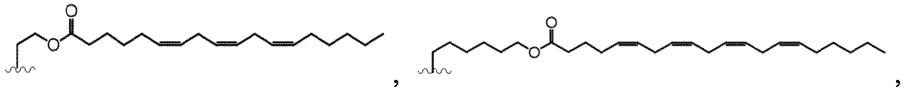

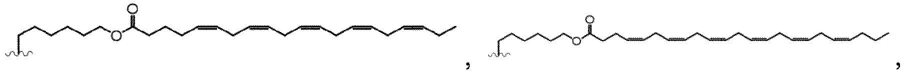

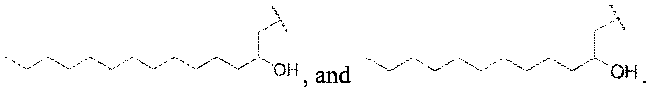

11. The phosphatidylamine compound of claim 1, wherein the phosphatidylamine compound is one of following compounds: 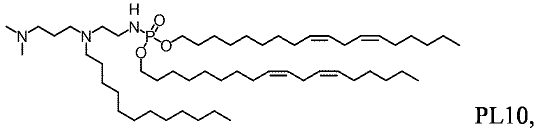 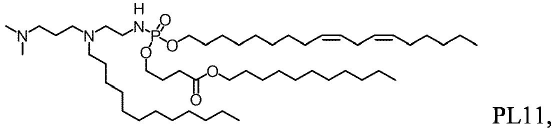 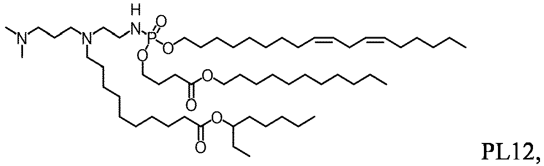 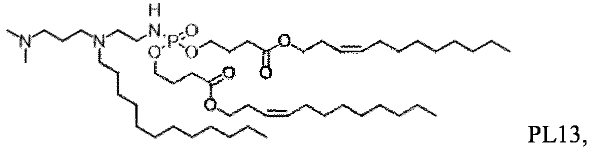 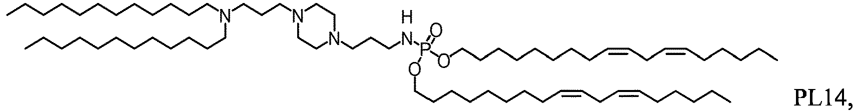 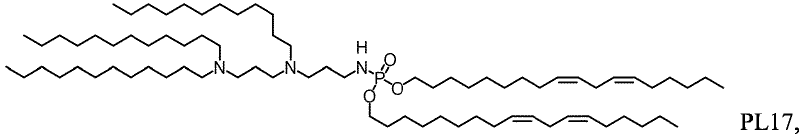 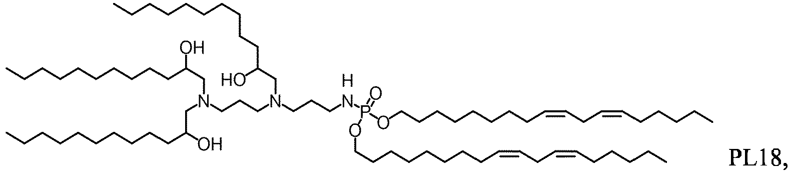 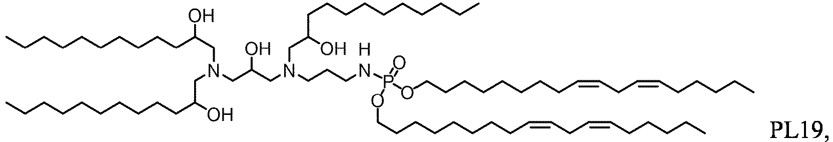 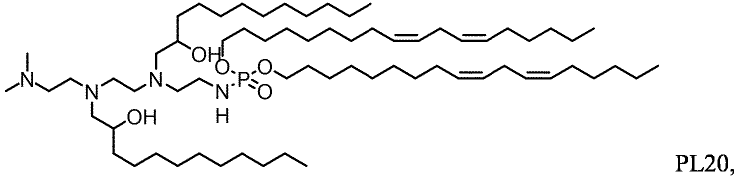 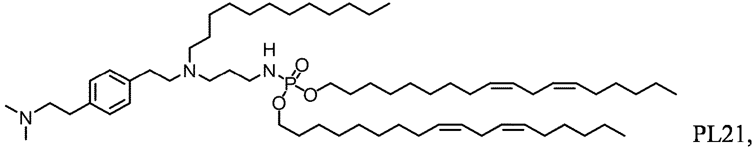 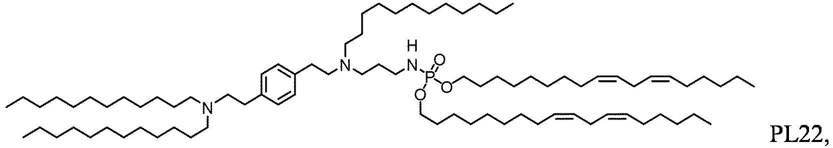 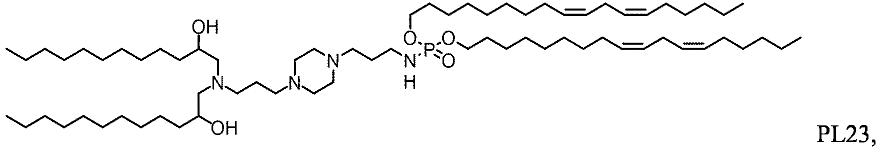 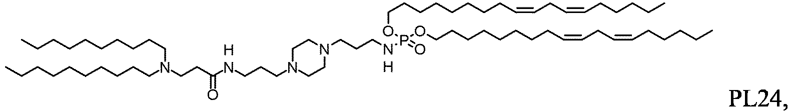 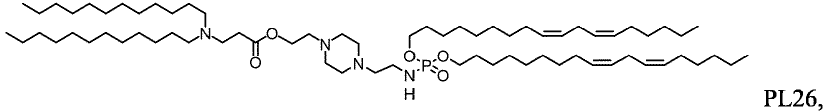 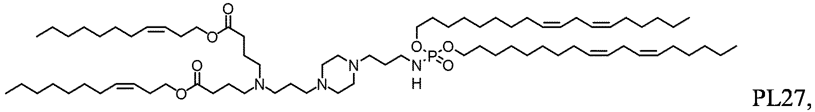 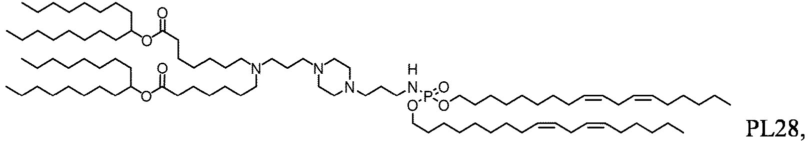 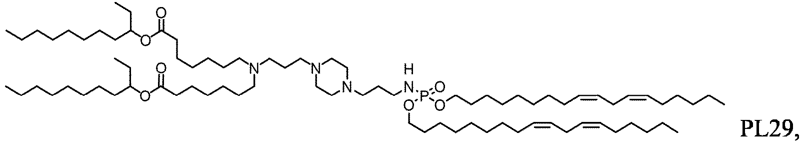 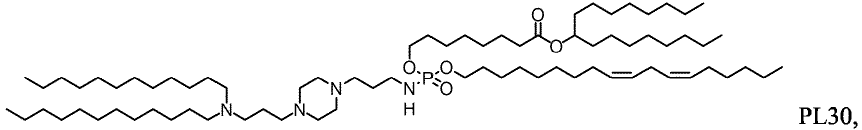 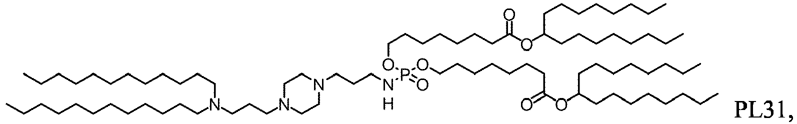 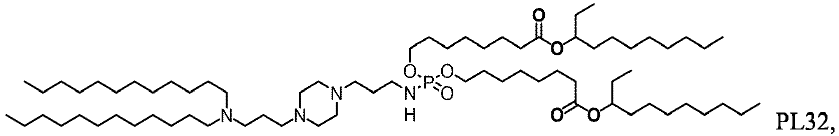 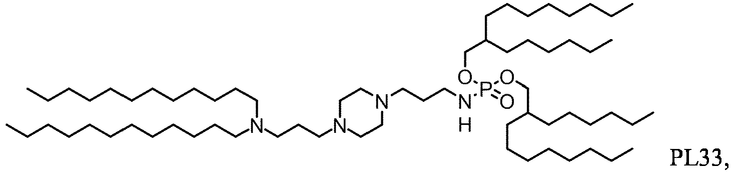 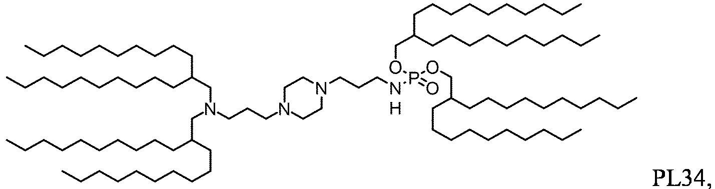 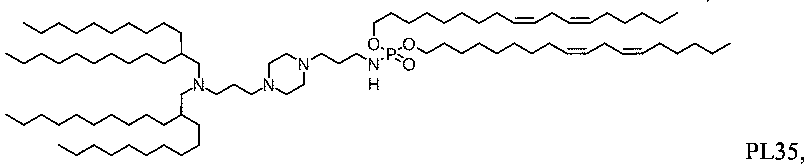 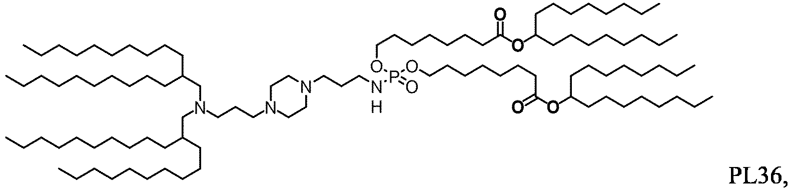 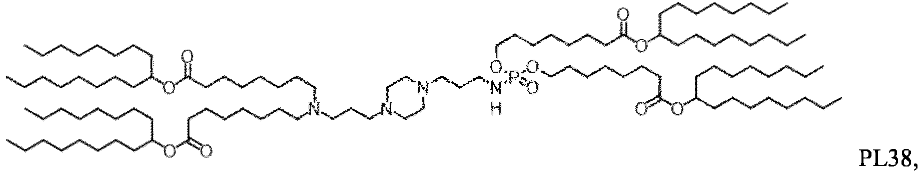 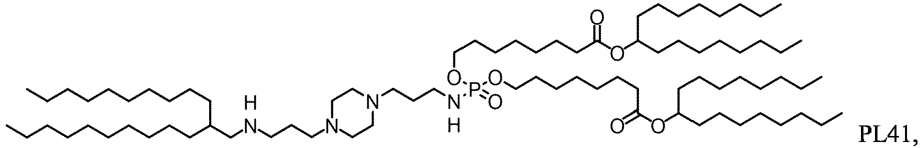 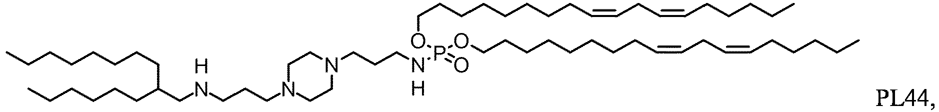 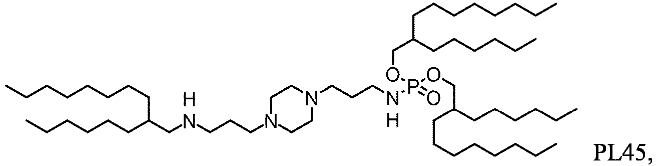 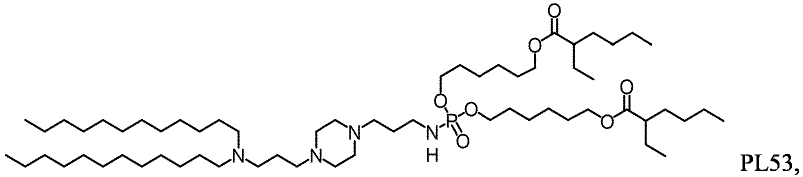 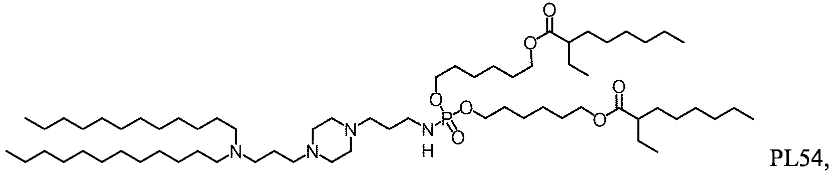 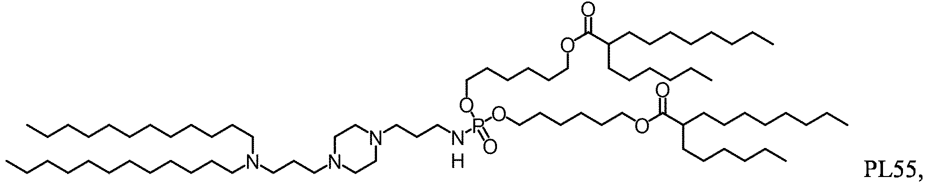 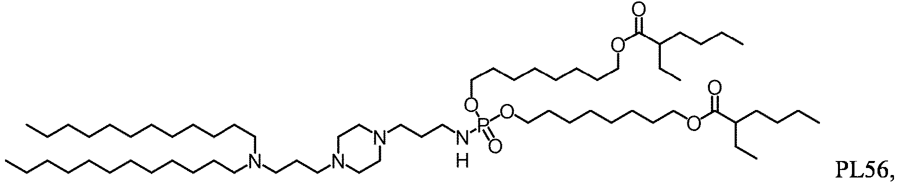 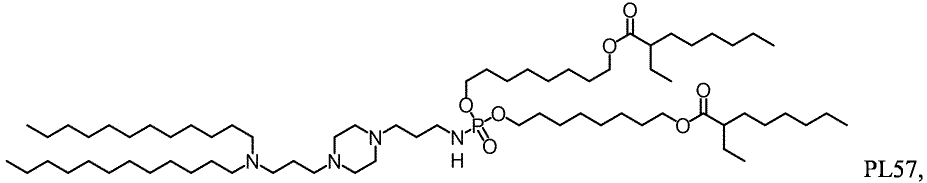 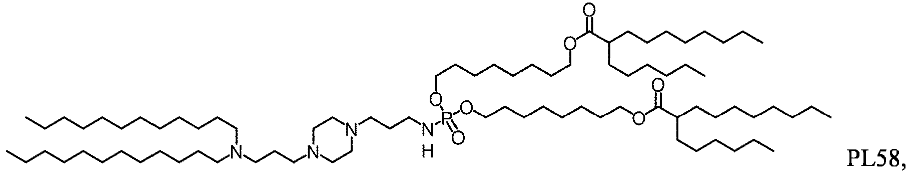 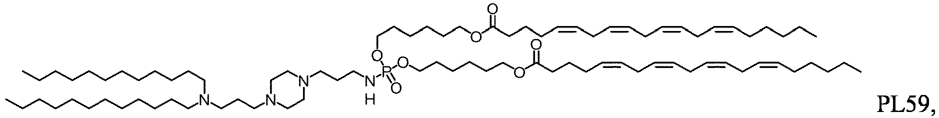 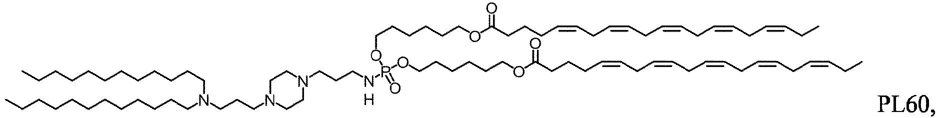 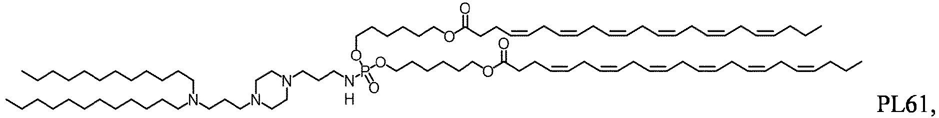 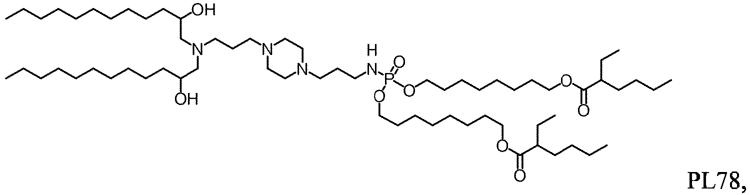 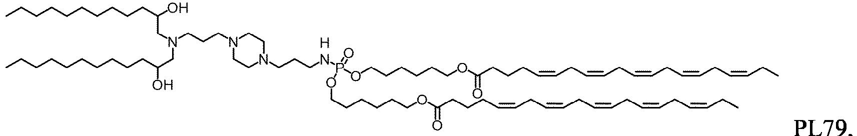 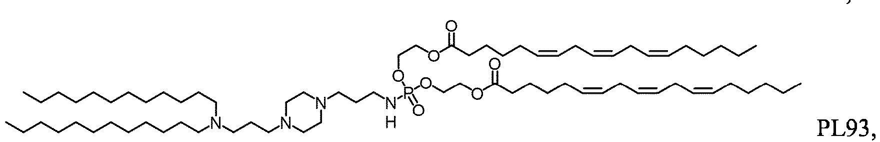 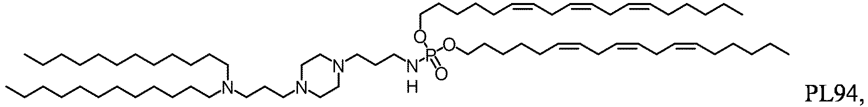 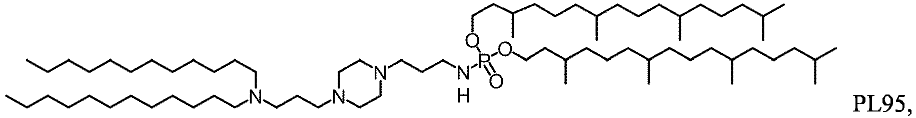 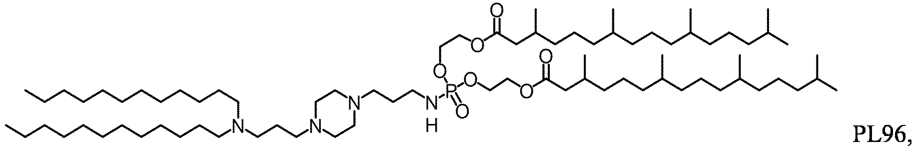 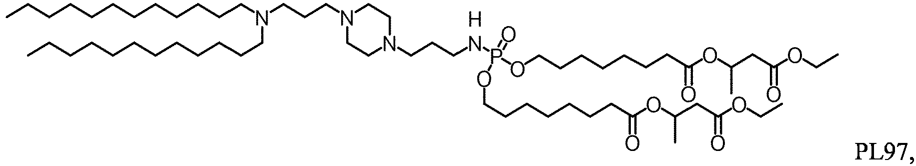 and 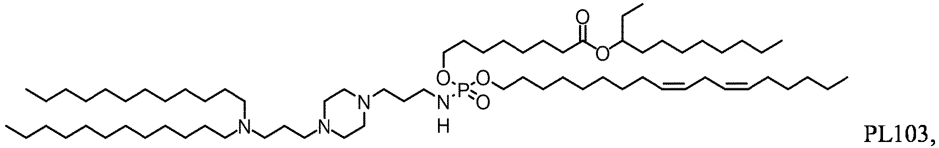 or stereoisomers, prodrugs, pharmaceutically acceptable salts thereof.

12. A lipid composition, comprising a phosphatidylamine compound of any of the claims 1-11, or a stereoisomer, a prodrug, and a pharmaceutically acceptable salt thereof and a therapeutic agent or a prophylactic agent.

13. The lipid composition of claim 12, the lipid composition further comprises an additional lipid, wherein the additional lipid is selected from one or more of a neutral lipid, a steroid, and a polymer-conjugated lipid, wherein
optionally, the neutral lipid is selected from one or more of DSPC, DPPC, DOPC, POPC, DOPE, DOPS, and SM, preferably, DOPE;
optionally, a molar ratio of the neutral lipid to the phosphatidylamine compound is from 2:1 to 8:1;
optionally, the steroid is cholesterol;
optionally, a molar ratio of the steroid to the phosphatidylamine compound is from 1:1 to 5:1, and
optionally, the polymer-conjugated lipid is a PEGylated lipid selected from PEG-DAG, PEG-PE, PEG-S-DAG, and PEG-cer.

14. The lipid composition of claim 12, wherein the therapeutic agent or the prophylactic agent is selected from one or more of a nucleic acid drug, a gene vaccine, a small molecule drug, a polypeptide, and a protein drug.

15. The lipid composition of any of the claims 12-14, wherein the lipid composition is composed of lipid nanoparticles.

16. A lipid nanoparticle, the lipid nanoparticle including a phosphatidylamine compound of any of the claims 1-11 or the lipid composition of any of the claims 12-14.

17. A pharmaceutical composition, a pharmaceutical composition comprising the phosphatidylamine compound of any of the claims 1-11, the lipid composition of any of the claims 12-15, or the lipid nanoparticle of claim 16, and pharmaceutically acceptable diluents or excipients.

18. The lipid composition of any of the claims 12-15, the lipid nanoparticle of claim 16, or the pharmaceutical composition of claim 17 for treating or preventing a disease in an individual in corresponding need; or a use of the lipid composition of any of the claims 12-15, the lipid nanoparticle of claim 16, or the pharmaceutical composition of claim 17 in the preparation of a drug for treating or preventing a disease in a corresponding need, optionally, the disease includes, but is not limited to, fatty liver, obesity, tumors, arthritis, viral infections, or the like.

19. A method for treating or preventing a disease in an individual in a corresponding need, the method including administering the lipid composition of any of the claims 12-15, the lipid nanoparticle of claim 16, or the pharmaceutical composition of claim 17 to the individual, optionally, the disease includes, but is not limited to, fatty liver, obesity, tumors, arthritis, viral infections, or the like.

20. A method for vaccinating an individual in a corresponding need against antiviral pathogens, the method including administering the lipid composition of any of the claims 12-15, the lipid nanoparticle of claim 16, or the pharmaceutical composition of claim 17 to the individual.
